# EUROPEAN PATENT APPLICATION

(11) **EP 4 501 853 A1**
(43) Date of publication of application: **05.02.2025**
(21) Application number: 23780481.0
(22) Date of filing: 28.03.2023
(51) Int. Cl.: C01B 39/48, C07D 401/08, C07D 295/135

(54) **CYCLOHEXYLENEDIAMINIUM SALT, USE THEREOF, AND MSE TYPE ZEOLITE OBTAINED USING SAME**

(30) Priority: 29.03.2022 JP 2022054677; 30.11.2022 JP 2022190915
(71) Applicant: Sagami Chemical Research Institute, Ayase-shi Kanagawa 252-1193 (JP); Tosoh Corporation, Yamaguchi 746-8501 (JP)
(72) Inventor: ARAKI Keisuke, Ayase-shi, Kanagawa 252-1193 (JP); YOSHIOKA Masato, Shunan-shi, Yamaguchi 746-8501 (JP); NAKANISHI Yusuke, Shunan-shi, Yamaguchi 746-8501 (JP); NAKAZAWA Naoto, Shunan-shi, Yamaguchi 746-8501 (JP); MITSUHASHI Ryo, Shunan-shi, Yamaguchi 746-8501 (JP)
(74) Representative: TBK
(86) International application number: PCT/JP2023/012422
(87) International publication number: WO 2023/190472

(57) **Abstract**

Provided is at least one of a novel cyclohexylenediaminium salt, a producing method therefor and use thereof, and an MSE-type zeolite obtained by using the same. An MSE-type zeolite having the following powder X-ray diffraction peaks.

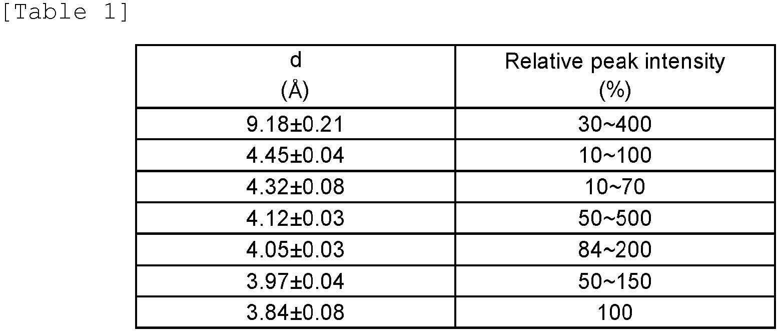

## Description

### Technical Field

The present invention relates to a novel cyclohexylenediaminium salt, use thereof, and an MSE-type zeolite obtained therefrom.

### Background Art

Zeolites are produced by crystallizing a composition comprising various organic structure-directing agents (hereinafter, also referred to as "SDA"). Known as the organic structure-directing agent for producing a zeolite in which pores formed with an oxygen-containing 12-membered ring is the largest pore (hereinafter, also referred to as "large pore zeolite") are salts such as N,N,N',N'-tetraethylbicyclo[2.2.2]oct-7-ene-2,3,5:6-dipyrrolidinium (hereinafter, also referred to as "TEBOP") and a tetraalkylammonium cation (Patent Literature 1 and Non Patent Literature 1). Specifically, an MSE-type zeolite having three-dimensional porous structure constituted by one-dimensional oxygen-containing 12-membered ring pores and two-dimensional oxygen-containing 10-membered ring pores has been reported as a light olefin synthesis catalyst (Non Patent Literature 2), a hydroquinone synthesis catalyst (Non Patent Literature 3), and a hydrocarbon adsorbent (Patent Literature 2).

### Citation List

### Patent Literature

Patent Literature 1: US2010/0081775 A1
Patent Literature 2: JP2022-068871 A

### Non Patent Literature

Non Patent Literature 1: J. Phys. Chem. B, 2006, Vol. 110, No. 5, pp. 2045-2050.
Non Patent Literature 2: Chem. Commun., 2010, No. 46, pp. 2662-2664.
Non Patent Literature 3: ACS Catal., 2014, No. 4, pp. 2653-2657.

### Summary of Invention

### Technical Problem

Tetraalkylammonium salts suitable for zeolite production have been conventionally known. Meanwhile, a cyclohexylenediaminium salt suitable as the organic structure-directing agent for the zeolite production has not been reported. In addition, there has been no report on an MSE-type zeolite obtained with the cyclohexylenediaminium salt as the SDA.

It is an object of the present disclosure to provide at least one of a novel cyclohexylenediaminium salt, a producing method thereof and use thereof, and an MSE-type zeolite obtained using the same. Specifically, it is an object of the present disclosure to provide at least one of a novel cyclohexylenediaminium salt usable as an organic structure-directing agent for zeolite production, a producing method thereof, and a novel zeolite obtained therefrom as a raw material and a producing method therefor.

### Solution to Problem

The present inventors have found a cyclohexylenediaminium salt that acts as the organic structure-directing agent for zeolite production, and also found that using this cyclohexylenediaminium salt as an SDA yields a novel MSE-type zeolite. As a result, the present inventors have completed the present invention.

Specifically, the present invention is as denoted in Claims, and the summery of the present disclosure is as follows.
[1] An MSE-type zeolite having the following powder X-ray diffraction peaks.

**[Table 1]**

| d (Å) | Relative peak intensity (%) |
|---|---|
| 9.18±0.21 | 30~400 |
| 4.45±0.04 | 10~100 |
| 4.32±0.08 | 10~70 |
| 4.12±0.03 | 50~500 |
| 4.05±0.03 | 84~200 |
| 3.97±0.04 | 50~150 |
| 3.84±0.08 | 100 |

[2] The MSE-type zeolite according to the [1], wherein a mole ratio of silica to alumina is 10 or more and 40 or less.
[3] The MSE-type zeolite according to the [1] or [2], wherein an average crystal grain diameter is 0.005 µm or more and 1.0 µm or less.
[4] An MSE-type zeolite, comprising a cyclohexylenediaminium cation represented by the following formula (1'), 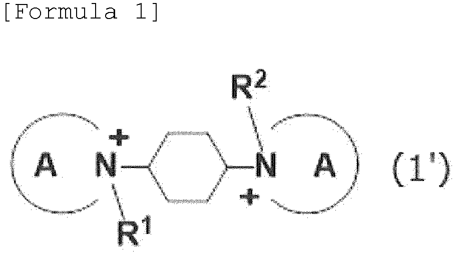
wherein R¹ and R² each independently represent an alkyl group having 1 to 4 carbon atoms, and the alkyl group is optionally substituted with one or more substituents selected from the group consisting of a halogen atom, a hydroxy group, an alkoxy group having 1 to 4 carbon atoms, and a dialkylamino group having 2 to 8 carbon atoms, and
wherein the ring A each independently represents a five to seven-membered nitrogen-containing heterocycle optionally having a crosslinking or a condensed ring, and the nitrogen-containing heterocycle is optionally substituted with one or more substituents selected from the group consisting of: a halogen atom; a hydroxy group; an oxo group; an alkyl group having 1 to 6 carbon atoms optionally substituted with a hydroxy group or an alkoxy group having 1 to 6 carbon atoms; a haloalkyl group having 1 to 6 carbon atoms; an alkoxy group having 1 to 6 carbon atoms; an aryl group having 6 to 12 carbon atoms; an aralkyl group having 7 to 13 carbon atoms; an aryloxy group having 6 to 12 carbon atoms; an alkyloxycarbonyl group having 1 to 6 carbon atoms; an acylamino group having 1 to 8 carbon atoms optionally substituted on a nitrogen atom with an alkyl group having 1 to 4 carbon atoms; an aminocarbonyl group having 1 to 9 carbon atoms optionally substituted on a nitrogen atom with one or more alkyl groups having 1 to 4 carbon atoms; an alkyloxycarbonyloxy group having 1 to 5 carbon atoms; an alkyloxycarbonylamino group having 1 to 5 carbon atoms optionally substituted on a nitrogen atom with an alkyl group having 1 to 4 carbon atoms; an ureide group optionally substituted on a nitrogen atom with one or more alkyl groups having 1 to 4 carbon atoms; a dialkylamino group having 2 to 12 carbon atoms; and a diarylamino group having 12 to 24 carbon atoms.

[5] A cyclohexylenediaminium salt represented by the following formula (1), 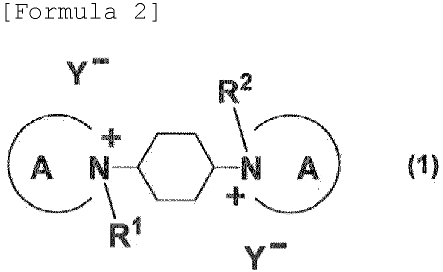
wherein R¹ and R² each independently represent an alkyl group having 1 to 4 carbon atoms, and the alkyl group is optionally substituted with one or more substituents selected from the group consisting of a halogen atom, a hydroxy group, an alkoxy group having 1 to 4 carbon atoms, and a dialkylamino group having 2 to 8 carbon atoms,
wherein the ring A each independently represents a five to seven-membered nitrogen-containing heterocycle optionally having a crosslinking or a condensed ring, and the nitrogen-containing heterocycle is optionally substituted with one or more substituents selected from the group consisting of: a halogen atom; a hydroxy group; an oxo group; an alkyl group having 1 to 6 carbon atoms optionally substituted with a hydroxy group or an alkoxy group having 1 to 6 carbon atoms; a haloalkyl group having 1 to 6 carbon atoms; an alkoxy group having 1 to 6 carbon atoms; an aryl group having 6 to 12 carbon atoms; an aralkyl group having 7 to 13 carbon atoms; an aryloxy group having 6 to 12 carbon atoms; an alkyloxycarbonyl group having 1 to 6 carbon atoms; an acylamino group having 1 to 8 carbon atoms optionally substituted on a nitrogen atom with an alkyl group having 1 to 4 carbon atoms; an aminocarbonyl group having 1 to 9 carbon atoms optionally substituted on a nitrogen atom with one or more alkyl groups having 1 to 4 carbon atoms; an alkyloxycarbonyloxy group having 1 to 5 carbon atoms; an alkyloxycarbonylamino group having 1 to 5 carbon atoms optionally substituted on a nitrogen atom with an alkyl group having 1 to 4 carbon atoms; an ureide group optionally substituted on a nitrogen atom with one or more alkyl groups having 1 to 4 carbon atoms; a dialkylamino group having 2 to 12 carbon atoms; and a diarylamino group having 12 to 24 carbon atoms, and wherein Y⁻ each independently represents an anion.

[6] The cyclohexylenediaminium salt according to the [5], wherein R¹ and R² in the formula (1) both represent the alkyl group having 1 to 4 carbon atoms.
[7] The cyclohexylenediaminium salt according to the [5] or [6], wherein A in the formula (1) all represent a five to six-membered nitrogen-containing heterocycle optionally having a crosslinking or a condensed ring, and the nitrogen-containing heterocycle is optionally substituted with one or more substituents selected from the group consisting of: a methyl group; an ethyl group; a methoxy group; an ethoxy group; a hydroxy group; and a halogen atom.
[8] The cyclohexylenediaminium salt according to any one of the [5] to [7], wherein R¹ and R² in the formula (1) both represent a methyl group or an ethyl group.
[9] The cyclohexylenediaminium salt according to any one of the [5] to [8], wherein Y⁻ in the formula (1) all represent Cl⁻ (a chloride ion), Br⁻ (a bromide ion), I⁻(an iodide ion), CH₃OSO₂O⁻ (a methyl sulfate ion), CH₃CH₂OSO₂O⁻ (an ethyl sulfate ion), CH₃OCOO⁻ (a methyl carbonate ion), CH₃CH₂OCOO⁻ (an ethyl carbonate ion), C₆H₅SO₂O⁻ (a benzenesulfonate ion), p-CH₃C₆H₄SO₂O⁻ (a p-toluenesulfonate ion), CH₃SO₂O⁻ (a methanesulfonate ion), CF₃SO₂O⁻ (a trifluoromethanesulfonate ion), or OH⁻ (a hydroxide ion).
[10] The cyclohexylenediaminium salt according to any one of the [5] to [9], wherein A in the formula (1) all represent a piperidine ring.
[11] The cyclohexylenediaminium salt according to any one of the [5] to [9], wherein A in the formula (1) all represent a pyrrolidine ring.
[12] A method for producing a zeolite, the method comprising a crystallizing step of crystallizing a composition comprising the cyclohexylenediaminium salt according to any one of the [5] to [11], a silica source, an alumina source, an alkali source, and water.
[13] The method for producing a zeolite according to [12], wherein the zeolite is an MSE-type zeolite.

### Advantageous Effect of Invention

The present disclosure can provide at least one of the novel cyclohexylenediaminium salt, the producing method therefor, and the use thereof. Further, the present disclosure can provide at least one of the cyclohexylenediaminium salt for zeolite production, specifically the cyclohexylenediaminium salt for MSE-type zeolite production, the producing method thereof, and the method for producing a zeolite using the same. Furthermore, the present disclosure can provide at least one of the MSE-type zeolite, the producing method thereof, and the adsorbent comprising the same.

### Brief Description of Drawings

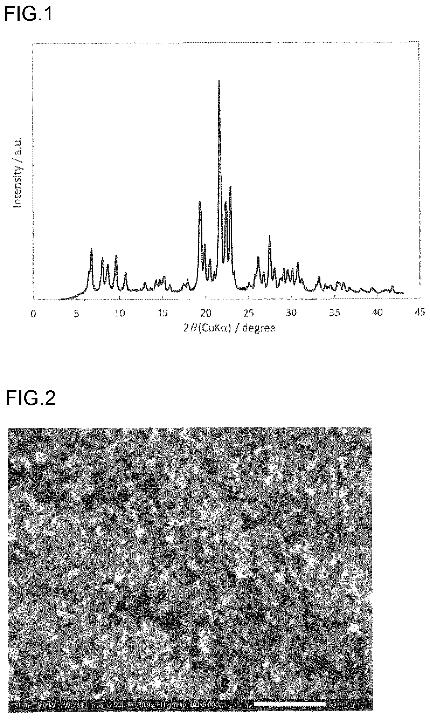

[Figure 1] Figure 1 is an XRD pattern of an MSE-type zeolite of Example 2-2.
[Figure 2] Figure 2 is an SEM observation view of the MSE-type zeolite of Example 2-2.

### Description of Embodiments

Hereinafter, the present disclosure will be described with showing an example of an embodiment. The terms in the present embodiment are as follows.

R¹ and R² each independently represent an alkyl group having 1 to 4 carbon atoms optionally substituted with one or more substituents selected from the group consisting of a halogen atom, a hydroxy group, an alkoxy group having 1 to 4 carbon atoms, and a dialkylamino group having 2 to 8 carbon atoms.

The ring A each independently represents a five to seven-membered nitrogen-containing heterocycle optionally having at least one of a crosslinking and a condensed ring, and the nitrogen-containing heterocycle is optionally substituted with one or more substituents selected from the group consisting of: a halogen atom; a hydroxy group; an oxo group; an alkyl group having 1 to 6 carbon atoms optionally substituted with a hydroxy group or an alkoxy group having 1 to 6 carbon atoms; a haloalkyl group having 1 to 6 carbon atoms; an alkoxy group having 1 to 6 carbon atoms; an aryl group having 6 to 12 carbon atoms; an aralkyl group having 7 to 13 carbon atoms; an aryloxy group having 6 to 12 carbon atoms; an alkyloxycarbonyl group having 1 to 6 carbon atoms; an acylamino group having 1 to 8 carbon atoms optionally substituted on a nitrogen atom with an alkyl group having 1 to 4 carbon atoms; an aminocarbonyl group having 1 to 9 carbon atoms optionally substituted on a nitrogen atom with one or more alkyl groups having 1 to 4 carbon atoms; an alkyloxycarbonyloxy group having 1 to 5 carbon atoms; an alkyloxycarbonylamino group having 1 to 5 carbon atoms optionally substituted on a nitrogen atom with an alkyl group having 1 to 4 carbon atoms; an ureide group optionally substituted on a nitrogen atom with one or more alkyl groups having 1 to 4 carbon atoms; a dialkylamino group having 2 to 12 carbon atoms; and a diarylamino group having 12 to 24 carbon atoms.

Y⁻ each independently represents an anion. Examples of the anion of Y⁻ include a halide ion, a sulfonate ion represented by R₃SO₂O⁻, a carboxylate ion represented by R₄COO⁻, or a phosphate ion optionally substituted with an alkoxide having 1 to 4 carbon atoms or an alkyl group having 1 to 4 carbon atoms. In the phosphate ion of Y⁻ optionally substituted with an alkyl group having 1 to 4 carbon atoms, specific examples of the alkyl group having 1 to 4 carbon atoms include a methyl group, an ethyl group, a propyl group, an isopropyl group, a butyl group, and a cyclopropyl group. In the phosphate ion of Y⁻ optionally substituted with an alkoxide having 1 to 4 carbon atoms, specific examples of the alkoxide having 1 to 4 carbon atoms include a methoxide ion, an ethoxide ion, a propoxide ion, an isopropoxide ion, or a butoxide ion. Among the anions of Y⁻, Y⁻ preferably represents a halide ion, a sulfonate ion represented by R³SO₂O⁻, or a carboxylate ion represented by R⁴COO⁻; and more preferably represents a chloride ion, a bromide ion, an iodide ion, a sulfonate ion represented by R³SO₂O⁻, or a carboxylate ion represented by R⁴COO⁻.

Y⁻ is not limited to a monovalent anion, and may be a divalent anion. When Y⁻ represents a divalent anion in the formula (1), the number of Y⁻ is one per the cation moiety. That is, when Y⁻ in the formula (1) is represented by Y'²⁻, which represents the divalent anion, the cyclohexylenediaminium salt of the present embodiment represented by the formula (1) can be represented by the following formula (1da). Note that the cyclohexylenediaminium salt represented by the following formula (1da) is also referred to as "cyclohexylenediaminium salt (1da)" hereinafter.

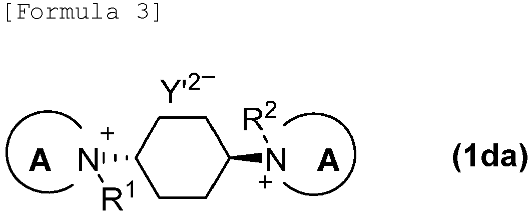

In the formula (1da), Y'²⁻ represents a divalent anion. In the formula (1da), R¹, R², and the ring A are as noted above (same as R¹, R², and the ring A in the formula (1) respectively).

Among divalent anions, Y'²⁻ in the formula (1da) preferably represents a sulfate ion or a carbonate ion; and more preferably represents a sulfate ion.

Y⁻ is not limited to a monovalent anion, and may be a trivalent anion. When Y⁻ represents a trivalent anion in the formula (1), the number of Y⁻ is two per three of the cation moiety. That is, when Y⁻ in the formula (1) is represented by Y"³⁻, which represents the trivalent anion, the cyclohexylenediaminium salt of the present embodiment represented by the formula (1) can be represented by the following formula (1ta). Note that the cyclohexylenediaminium salt represented by the following formula (1ta) is also referred to as "cyclohexylenediaminium salt (1ta)" hereinafter.

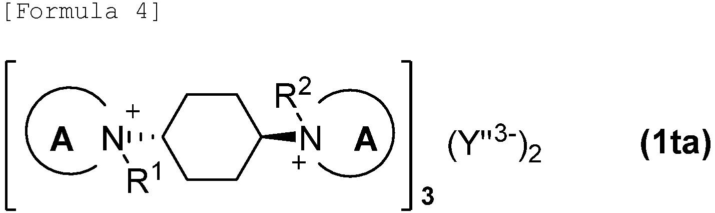

In the formula (1ta), Y"³⁻ represents a trivalent anion. In the formula (1ta), R¹, R², and the ring A are as noted above (same as R¹, R², and the ring A in the formula (1) respectively).

Among trivalent anions, Y"³⁻ preferably represents a phosphate ion.

Ya represents a chlorine atom, a bromine atom, an iodine atom, a sulfonyloxy group represented by R₃SO₂O⁻, a carboxy group represented by R₄COO⁻, or a phosphoric acid group represented by (O=)P(R⁵)₂O-. Ya⁻ represents an ionized product of Ya. Specifically, Ya⁻ each independently represents Cl⁻ (a chloride ion), Br⁻ (a bromide ion), I⁻ (an iodide ion), a sulfonate ion represented by R₃SO₂O⁻, a carboxylate ion represented by R₄COO⁻, or a phosphate ion represented by (O=)P(R⁵)₂O⁻.

Ya' represents a sulfonyloxy group represented by -OSO₂O-, a carboxy group represented by -OC(=O)O-, or a phosphate ion represented by (O=)P(R⁵)(O-)₂. Ya'²⁻represents an ionized product of Ya'. Specifically, Ya'²⁻represents a sulfonate ion represented by SO₄²⁻, a carbonate ion represented by CO₂²⁻, or a phosphate ion represented by P(OR⁵)(O)₂²⁻.

Ya'' represents a phosphoric acid group represented by (O=)P(O-)₃. Ya"³⁻ represents an ionized product of Ya''. Specifically, Ya"³⁻ represents a phosphate ion represented by PO₃³⁻.

Yb represents a halogen atom, a sulfonyloxy group represented by R₃SO₂O⁻, a carboxy group represented by R₄COO⁻, or a phosphoric acid group represented by (O=)P(R⁵)₂O-. Yb⁻ represents an ionized product of Yb. Specifically, Yb⁻ each independently represents a halogen ion, a sulfonate ion represented by R³SO₂O⁻, a carboxylate ion represented by R₄COO⁻, or a phosphate ion represented by (O=)P(R⁵)₂O⁻.

R³ represents a hydrogen atom, a hydroxy group (-OH), a metal oxide group (-OM) in which a hydrogen atom in a hydroxy group (-OH) is replaced with an alkali metal atom, an alkyl group having 1 to 4 carbon atoms, a fluoroalkyl group having 1 to 4 carbon atoms, an alkoxy group having 1 to 4 carbon atoms, a halogen atom, a phenyl group, or a 4-methylphenyl group. Examples of the alkali metal atom M in the metal oxide group (-OM) of R³ include a lithium atom, a sodium atom, a potassium atom, and a cesium atom.

R⁴ represents a hydrogen atom, an alkyl group having 1 to 4 carbon atoms, a fluoroalkyl group having 1 to 4 carbon atoms, an alkoxy group having 1 to 4 carbon atoms, or a phenyl group optionally substituted with an alkyl group having 1 to 4 carbon atoms.

R⁵ represents a hydrogen atom, a hydroxy group (-OH), a metal oxide group (-OM) in which a hydrogen atom in a hydroxy group (-OH) is replaced with an alkali metal atom, an alkyl group having 1 to 4 carbon atoms, an alkoxy group having 1 to 4 carbon atoms, a phenyl group, or a 4-methylphenyl group. Examples of the alkali metal atom M in the metal oxide group (-OM) of R⁵ include a lithium atom, a sodium atom, a potassium atom, and a cesium atom.

The expression "optionally substituted" with the predetermined substituent means that a substituent which can be structurally substituted with two or more substituents may be substituted with one or more substituents unless the number of the substituents is not specified. A substituent that is not described as "optionally substituted" means an unsubstituted substituent.

Examples of the halogen atom in R¹ to R³, the ring A, and Yb include a fluorine atom, a chlorine atom, a bromine atom, and an iodine atom.

The alkoxy group having 1 to 4 carbon atoms in R¹ to R⁵ is any one of linear, branched, or cyclic alkoxy groups, and examples thereof include a methoxy group, an ethoxy group, a propoxy group, an isopropoxy group, a butoxy group, a 1-methylpropoxy group, a 2-methylpropoxy group, a cyclopropoxy group, a tert-butoxy group, or a cyclobutoxy group.

Examples of the dialkylamino group having 2 to 8 carbon atoms in R¹ and R² include a dimethylamino group, a diethylamino group, a dipropylamino group, a diisopropylamino group, a dibutylamino group, a bis(2-methylpropyl)amino group, a bis(2,2-dimethylpropyl)amino group, a bis(3-cyclopropyl)amino group, a dicyclopropylamino group, a di(tert-butyl)amino group, a dicyclobutylamino group, a dipentylamino group, a bis(2-methylpentyl)amino group, a di(1-methylbutyl)amino group, a bis(1,2-dimethylbutyl)amino group, a di(1-ethylpropyl)amino group, a dicyclopentylamino group, a dihexylamino group, a dicyclohexylamino group, an N-ethyl-N-methylamino group, an N-methyl-N-propylamino group, an N-cyclopropyl-N-methylamino group, an N-butyl-N-methylamino group, an N-cyclobutyl-N-methylamino group, an N-methyl-N-pentylamino group, an N-cyclopentyl-N-methylamino group, an N-hexyl-N-methylamino group, an N-cyclohexyl-N-methylamino group, an N-cycloheptyl-N-methylamino group, an azetidil group, a pyrrolidinyl group, a piperidino group, an azepanyl group, a morpholino group, or a 2-morpholinyl group.

The fluoroalkyl group having 1 to 4 carbon atoms in R³ and R⁴ refers to a linear, branched, or cyclic fluoroalkyl group, and examples thereof include a trifluoromethyl group, a difluoromethyl group, a perfluoroethyl group, a 2,2,2-trifluoroethyl group, a 1,1-difluoroethyl group, a 2,2-difluoroethyl group, a perfluoropropyl group, a 2,2,3,3,3-pentafluoropropyl group, a 2,2,3,3-tetrafluoropropyl group, a 3,3,3-trifluoropropyl group, a 1,1-difluoropropyl group, a perfluoro(1-methylpropyl) group, a 2,2,2-trifluoro-1-(trifluoromethyl)ethyl group, a perfluorocyclopropyl group, a 2,2,3,3-tetrafluorocyclopropyl group, a perfluorobutyl group, a 2,2,3,3,4,4,4-heptafluorobutyl group, a 3,3,4,4,4-pentafluorobutyl group, a 4,4,4-trifluorobutyl group, a 1,2,2,3,3,3-hexafluoro-1-(trifluoromethyl)propyl group, a 1-(trifluoromethyl)propyl group, a 1-methyl-3,3,3-trifluoropropyl group, a perfluorocyclobutyl group, or a 2,2,3,3,4,4-hexafluorocyclobutyl group.

The alkyl group having 1 to 4 carbon atoms in R¹ and R² is a linear, branched, or cyclic alkyl group, and examples thereof include a methyl group, an ethyl group, a propyl group, an isopropyl group, a butyl group, a 1-methylpropyl group, a 2-methylpropyl group, a tert-butyl group, a cyclopropyl group, or a cyclobutyl group. Specific examples of R¹ and R² also include a trifluoromethyl group, a difluoromethyl group, a perfluoroethyl group, a 2,2,2-trifluoroethyl group, a 1,1-difluoroethyl group, a 2,2-difluoroethyl group, a perfluoropropyl group, a 2,2,3,3,3-pentafluoropropyl group, a 2,2,3,3-tetrafluoropropyl group, a 3,3,3-trifluoropropyl group, a 1,1-difluoropropyl group, a perfluoro(1-methylpropyl)group, a 2,2,2-trifluoro-1-(trifluoromethyl)ethyl group, a perfluorobutyl group, a 2,2,3,3,4,4,4-heptafluorobutyl group, a 3,3,4,4,4-pentafluorobutyl group, a 4,4,4-trifluorobutyl group, a 1,2,2,3,3,3-hexafluoro-1-(trifluoromethyl)propyl group, a 1-(trifluoromethyl)propyl group, a 1-methyl-3,3,3-trifluoropropyl group, a chloromethyl group, a bromomethyl group, an iodomethyl group, a 2-chloroethyl group, a 3-bromopropyl group, a hydroxymethyl group, a 2-hydroxyethyl group, a 2-hydroxypropyl group, a 3-hydroxypropyl group, a 4-hydroxybutyl group, a methoxymethyl group, a 2-methoxyethyl group, a 2-methoxypropyl group, a 3-methoxypropyl group, a 4-methoxybutyl group, an ethoxymethyl group, an ethoxyethyl group, a 2-ethoxypropyl group, a 3-ethoxypropyl group, a 4-ethoxybutyl group, a 2-(dimethylamino)ethyl group, a 2-(diethylamino)ethyl group, a 2-(dipropylamino)ethyl group, a 2-(dibutylamino)ethyl group, and a 2-(dimethylamino)propyl group.

The alkyl group having 1 to 4 carbon atoms in R³, R⁴, and R⁵ is a linear, branched, or cyclic alkyl group, and examples thereof include a methyl group, an ethyl group, a propyl group, an isopropyl group, a butyl group, a 1-methylpropyl group, a 2-methylpropyl group, a tert-butyl group, a cyclopropyl group, or a cyclobutyl group.

Examples of the five to seven-membered nitrogen-containing heterocycle optionally having at least one of a crosslinking or a condensed ring in the ring A include a pyrrolidine ring, an imidazolidine ring, an indoline ring, an isoindoline ring, a morpholine ring, a piperidine ring, a piperazine ring, a quinuclidine ring, a tetrahydrotriazine ring, or a decahydroquinoline ring. Examples of the five to seven-membered nitrogen-containing heterocycle having a crosslinking include a five to seven-membered nitrogen-containing heterocycle having a crosslinking and 1 to 2 carbon atoms, and examples of the five to seven-membered nitrogen-containing heterocycle having a condensed ring include a compound in which two of the nitrogen-containing heterocycle are condensed. Note that, in the nitrogen-containing heterocycle having a crosslinking, the number of atoms constituting the crosslinking is not included in calculation of the number of the ring member of the nitrogen-containing heterocycle. For example, a quinuclidine ring, which is the nitrogen-containing heterocycle having a crosslinking, is a six-membered nitrogen-containing heterocycle.

The alkyl group having 1 to 6 carbon atoms optionally substituted with an alkoxy group having 1 to 6 carbon atoms in the ring A may be any one of linear, branched, or cyclic alkyl groups, and not particularly limited. Examples thereof include a methyl group, a methoxymethyl group, an ethoxymethyl group, an ethyl group, a methoxyethyl group, an ethoxyethyl group, a propyl group, a 3-ethoxypropyl group, an isopropyl group, a butyl group, a 4-ethoxybutyl group, a 4-butoxybutyl group, a cyclohexylmethyl group, a 2-cyclopentylethyl group, a 2-methylpropyl group, a 2,2-dimethylpropyl group, a 3-cyclopropylpropyl group, a cyclopropyl group, a 2-methylbutyl group, a 3-methylbutyl group, a 3-methylbutan-2-yl group, a tert-butyl group, a cyclobutyl group, a pentyl group, a 2-methylpentyl group, a 1-methylbutyl group, a 2-methylpentan-2-yl group, a 2-ethylpropyl group, a cyclopentyl group, a hexyl group, a 1-methylpentyl group, a 2-ethylbutyl group, a cyclohexyl group, or a 4-methoxycyclohexyl group.

The haloalkyl group having 1 to 6 carbon atoms in the ring A include a trifluoromethyl group, a difluoromethyl group, a perfluoroethyl group, a 2,2,2-trifluoroethyl group, a 1,1-difluoroethyl group, a 2,2-difluoroethyl group, a perfluoropropyl group, a 2,2,3,3,3-pentafluoropropyl group, a 2,2,3,3-tetrafluoropropyl group, a 3,3,3-trifluoropropyl group, a 1,1-difluoropropyl group, a perfluoro(1-methylpropyl)group, a 2,2,2-trifluoro-1-(trifluoromethyl)ethyl group, a perfluorocyclopropyl group, a 2,2,3,3-tetrafluorocyclopropyl group, a perfluorobutyl group, a 2,2,3,3,4,4,4-heptafluorobutyl group, a 3,3,4,4,4-pentafluorobutyl group, a 4,4,4-trifluorobutyl group, a 1,2,2,3,3,3-hexafluoro-1-(trifluoromethyl)propyl group, a 1-(trifluoromethyl)propyl group, a 1-methyl-3,3,3-trifluoropropyl group, a perfluorocyclobutyl group, a 2,2,3,3,4,4-hexafluorocyclobutyl group, a perfluoropentyl group, a 2,2,3,3,4,4,5,5,5-nonafluoropentyl group, a 3,3,4,4,5,5,5-heptafluoropentyl group, a 4,4,5,5,5-pentafluoropentyl group, a 5,5,5-trifluoropentyl group, a 1,2,2,3,3,3-hexafluoro-1-(perfluoroethyl)propyl group, a 2,2,3,3,3-pentafluoro-1-(perfluoroethyl)propyl group, a perfluorocyclopentyl group, a perfluorohexyl group, a 2,2,3,3,4,4,5,5,6,6,6-undecafluorohexyl group, a 3,3,4,4,5,5,6,6,6-nonafluorohexyl group, a 4,4,5,5,6,6,6-heptafluorohexyl group, a 5,5,6,6,6-pentafluorohexyl group, a 6,6,6-trifluorohexyl group, a perfluorocyclohexyl group, a chloromethyl group, a bromomethyl group, an iodomethyl group, a 2-chloroethyl group, or a 3-bromopropyl group.

Example of the alkoxy group having 1 to 6 carbon atoms in the ring A include a methoxy group, an ethoxy group, a propoxy group, an isopropoxy group, a butoxy group, a 2-methylpropoxy group, a 2,2-dimethylpropoxy group, a 3-cyclopropylpropoxy group, a cyclopropoxy group, a 2-methylbutoxy group, a 3-methylbutoxy group, a tert-butoxy group, a cyclobutoxy group, a pentyloxy group, a 2-methylpentyloxy group, a 1-methylbutyloxy group, a 1,2-dimethylbutoxy group, a 1-ethylpropoxy group, a cyclopentyloxy group, a hexyloxy group, or a cyclohexyloxy group.

Examples of the aryl group having 6 to 12 carbon atoms in the ring A include a phenyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,4-trimethylphenyl group, a 2,3,5-trimethylphenyl group, a 2,3,6-trimethylphenyl group, a 2,4,5-trimethylphenyl group, a 2,4,6-trimethylphenyl group, a 3,4,5-trimethylphenyl group, a 2,3,4,5-tetramethylphenyl group, a 2,3,4,6-tetramethylphenyl group, a 2,3,5,6-tetramethylphenyl group, a 2-ethylphenyl group, a 3-ethylphenyl group, a 4-ethylphenyl group, a 2,3-diethylphenyl group, a 2,4-diethylphenyl group, a 2,5-diethylphenyl group, a 2,6-diethylphenyl group, a 3,4-diethylphenyl group, a 3,5-diethylphenyl group, a 2-propylphenyl group, a 3-propylphenyl group, a 4-propylphenyl group, a 2-isopropylphenyl group, a 3-isopropylphenyl group, a 4-isopropylphenyl group, a 2-cyclopropylphenyl group, a 3-cyclopropylphenyl group, a 4-cyclopropylphenyl group, a 2-butylphenyl group, a 3-butylphenyl group, a 4-butylphenyl group, a 2-(1-methylpropyl)phenyl group, a 3-(1-methylpropyl)phenyl group, a 4-(1-methylpropyl)phenyl group, a 2-(2-methylpropyl)phenyl group, a 3-(2-methylpropyl)phenyl group, a 4-(2-methylpropyl)phenyl group, a 2-cyclobutylphenyl group, a 3-cyclobutylphenyl group, a 4-cyclobutylphenyl group, a 1-naphthyl group, a 2-naphthyl group, a 2-biphenylyl group, a 3-biphenylyl group, or a 4-biphenylyl group.

Examples of the aralkyl group having 7 to 13 carbon atoms in the ring A include a benzyl group, an α-methylbenzyl group, a phenethyl group, an α-methylphenethyl group, an α,α-dimethylbenzyl group, an α,α-dimethylphenethyl group, a 4-methylphenethyl group, a 4-methylbenzyl group, a 4-isopropylbenzyl group, a 4-phenylbenzyl group, a diphenylmethyl group, a 1-naphthylmethyl group, or a 2-naphthylmethyl group.

Examples of the aryloxy group having 6 to 12 carbon atoms in the ring A include a phenyloxy group, a 2-methylphenyloxy group, a 3-methylphenyloxy group, a 4-methylphenyloxy group, a 2,3-dimethylphenyloxy group, a 2,4-dimethylphenyloxy group, a 2,5-dimethylphenyloxy group, a 2,6-dimethylphenyloxy group, a 3,4-dimethylphenyloxy group, a 3,5-dimethylphenyloxy group, a 2,3,4-trimethylphenyloxy group, a 2,3,5-trimethylphenyloxy group, a 2,3,6-trimethylphenyloxy group, a 2,4,5-trimethylphenyloxy group, a 2,4,6-trimethylphenyloxy group, a 3,4,5-trimethylphenyloxy group, a 2,3,4,5-tetramethylphenyloxy group, a 2,3,4,6-tetramethylphenyloxy group, a 2,3,5,6-tetramethylphenyloxy group, a 2-ethylphenyloxy group, a 3-ethylphenyloxy group, a 4-ethylphenyloxy group, a 2,3-diethylphenyloxy group, a 2,4-diethylphenyloxy group, a 2,5-diethylphenyloxy group, a 2,6-diethylphenyloxy group, a 3,4-diethylphenyloxy group, a 3,5-diethylphenyloxy group, a 2-propylphenyloxy group, a 3-propylphenyloxy group, a 4-propylphenyloxy group, a 2-isopropylphenyloxy group, a 3-isopropylphenyloxy group, a 4-isopropylphenyloxy group, a 2-cyclopropylphenyloxy group, a 3-cyclopropylphenyloxy group, a 4-cyclopropylphenyloxy group, a 2-butylphenyloxy group, a 3-butylphenyloxy group, a 4-butylphenyloxy group, a 2-(1-methylpropyl)phenyloxy group, a 3-(1-methylpropyl)phenyloxy group, a 4-(1-methylpropyl)phenyloxy group, a 2-(2-methylpropyl)phenyloxy group, a 3-(2-methylpropyl)phenyloxy group, a 4-(2-methylpropyl)phenyloxy group, a 2-cyclobutylphenyloxy group, a 3-cyclobutylphenyloxy group, a 4-cyclobutylphenyloxy group, a 1-naphthyloxy group, or a 2-naphthyloxy group.

Examples of the alkyloxycarbonyl group having 1 to 6 carbon atoms in the ring A include an oxycarbonyl group having a methyl group, an ethyl group, a propyl group, an isopropyl group, a butyl group, a 2-methylpropyl group, a 2,2-dimethylpropyl group, a cyclopropyl group, a 2-methylbutyl group, a 3-methylbutyl group, a 3-methylbutan-2-yl group, a tert-butyl group, or a cyclobutyl group.

The acylamino group having 1 to 8 carbon atoms, the aminocarbonyl group having 1 to 9 carbon atoms, the alkyloxycarbonylamino group having 1 to 5 carbon atoms, and the ureide group in the ring A are each optionally substituted on the nitrogen atom with an alkyl group having 1 to 4 carbon atoms. The alkyl group may be any one of a linear, branched, or cyclic alkyl group, and examples thereof include a methyl group, an ethyl group, a propyl group, an isopropyl group, a butyl group, a 1-methylpropyl group, a 2-methylpropyl group, a tert-butyl group, a cyclopropyl group, or a cyclobutyl group.

Examples of the acylamino group having 1 to 8 carbon atoms optionally substituted on the nitrogen atom with the alkyl group having 1 to 4 carbon atoms in the ring A include an acylamino group having 1 to 8 carbon atoms optionally substituted with a methyl group, an ethyl group, a propyl group, an isopropyl group, a butyl group, a cyclopropylmethyl group, a 2-methylpropyl group, a 2,2-dimethylpropyl group, a cyclopropyl group, a tert-butyl group, or a cyclobutyl group, and the examples further include a formylamino group, an acetylamino group, or a propanoylamino group.

Examples of the aminocarbonyl group having 1 to 9 carbon atoms optionally substituted on the nitrogen atom with one or more alkyl groups having 1 to 4 carbon atoms in the ring A include an aminocarbonyl group having 1 to 9 carbon atoms optionally substituted with a methyl group, an ethyl group, a propyl group, an isopropyl group, a butyl group, a cyclopropylmethyl group, a 2-methylpropyl group, a 2,2-dimethylpropyl group, a cyclopropyl group, a tert-butyl group, or a cyclobutyl group.

Examples of the alkyloxycarbonyloxy group having 1 to 5 carbon atoms in the ring A include an oxycarbonyloxy group having 1 to 5 carbon atoms with a methyl group, an ethyl group, a propyl group, an isopropyl group, a butyl group, a cyclopropylmethyl group, a 2-cyclopentylethyl group, a 2-methylpropyl group, a 2,2-dimethylpropyl group, a 3-cyclopropylpropyl group, a cyclopropyl group, a 2-methylbutyl group, a 3-methylbutyl group, a 3-methylbutan-2-yl group, a tert-butyl group, or a cyclobutyl group.

Examples of the alkyloxycarbonylamino group having 1 to 5 carbon atom optionally substituted on the nitrogen atom with an alkyl group having 1 to 4 carbon atoms in the ring A include an alkyloxycarbonylamino group having 1 to 5 carbon atoms optionally substituted with, as the alkyl group having 1 to 4 carbon atoms, a methyl group, an ethyl group, a propyl group, an isopropyl group, a butyl group, a cyclopropylmethyl group, a 2-methylpropyl group, a 2,2-dimethylpropyl group, a cyclopropyl group, a tert-butyl group, or a cyclobutyl group. Further, in the alkyloxycarbonylamino group having 1 to 5 carbon atoms optionally substituted on the nitrogen atom with the alkyl group having 1 to 4 carbon atoms in the ring A, examples of the alkyl group having 1 to 5 carbon atoms in the alkyloxycarbonylamino group having 1 to 5 carbon atoms include a methyl group, an ethyl group, a propyl group, an isopropyl group, a butyl group, a cyclobutylmethyl group, a 2-cyclopropylethyl group, a **2-**methylpropyl group, a 2,2-dimethylpropyl group, a 3-cyclopropyl group, a cyclopropyl group, a 2-methylbutyl group, a 3-methylbutyl group, a 3-methylbutan-2-yl group, a tert-butyl group, or a cyclobutyl group that is optionally substituted with a methyl group, an ethyl group, a propyl group, an isopropyl group, a butyl group, a cyclopropylmethyl group, a 2-methylpropyl group, a 2,2-dimethylpropyl group, a cyclopropyl group, a tert-butyl group, or a cyclobutyl group.

Examples of the dialkylamino group having 2 to 12 carbon atoms in the ring A include a dimethylamino group, a diethylamino group, a dipropylamino group, a diisopropylamino group, a bis(3-cyclopropyl)amino group, a dicyclopropylamino group, an N-ethyl-N-methylamino group, an N-methyl-N-propylamino group, an N-cyclopropyl-N-methylamino group, an N-butyl-N-methylamino group, an N-cyclobutyl-N-methylamino group, an N-methyl-N-pentylamino group, an N-cyclopentyl-N-methylamino group, an azetidil group, a pyrrolidinyl group, a piperidino group, an azepanyl group, a morpholino group, or a 2-morpholinyl group.

Examples of the diarylamino group having 12 to 24 carbon atoms in the ring A include, but not particularly limited to, a diphenylamino group, a bis(2-methylphenyl)amino group, a bis(3-methylphenyl)amino group, a bis(4-methylphenyl)amino group, a bis(2,3-dimethylphenyl)amino group, a bis(2,4-dimethylphenyl)amino group, a bis(2,5-dimethylphenyl)amino group, a bis(2,6-dimethylphenyl)amino group, a bis(3,4-dimethylphenyl)amino group, a bis(3,5-dimethylphenyl)amino group, a bis(2,3,4-trimethylphenyl)amino group, a bis(2,3,5-trimethylphenyl)amino group, a bis(2,3,6-trimethylphenyl)amino group, a bis(2,4,5-trimethylphenyl)amino group, a bis(2,4,6-trimethylphenyl)amino group, a bis(3,4,5-trimethylphenyl)amino group, a bis(2,3,4,5-tetramethylphenyl)amino group, a bis(2,3,4,6-tetramethylphenyl)amino group, a bis(2,3,5,6-tetramethylphenyl)amino group, a bis(2-ethylphenyl)amino group, a bis(3-ethylphenyl)amino group, a bis(4-ethylphenyl)amino group, a bis(2,3-diethylphenyl)amino group, a bis(2,4-diethylphenyl)amino group, a bis(2,5-diethylphenyl)amino group, a bis(2,6-diethylphenyl)amino group, a bis(3,4-diethylphenyl)amino group, a bis(3,5-diethylphenyl)amino group, a bis(2-propylphenyl)amino group, a bis(3-propylphenyl)amino group, a bis(4-propylphenyl)amino group, a bis(2-isopropylphenyl)amino group, a bis(3-isopropylphenyl)amino group, a bis(4-isopropylphenyl)amino group, a bis(2-cyclopropylphenyl)amino group, a bis(3-cyclopropylphenyl)amino group, a bis(4-cyclopropylphenyl)amino group, a bis(2-butylphenyl)amino group, a bis(3-butylphenyl)amino group, a bis(4-butylphenyl)amino group, a bis[2-(1-methylpropyl)phenyl]amino group, a bis[3-(1-methylpropyl)phenyl]amino group, a bis[4-(1-methylpropyl)phenyl]amino group, a bis[2-(2-methylpropyl)phenyl]amino group, a bis[3-(2-methylpropyl)phenyl]amino group, a bis[4-(2-methylpropyl)phenyl]amino group, a bis(2-cyclobutylphenyl)amino group, a bis(3-cyclobutylphenyl)amino group, a bis(4-cyclobutylphenyl)amino group, a di(1-naphthyl)amino group, a di(2-naphthyl)amino group, an N-(2-methylphenyl)-N-phenylamino group, an N-(3-methylphenyl)-N-phenylamino group, an N-(4-methylphenyl)-N-phenylamino group, an N-(2,3-dimethylphenyl)-N-phenylamino group, an N-(2,4-dimethylphenyl)-N-phenylamino group, an N-(2,5-dimethylphenyl)-N-phenylamino group, an N-(2,6-dimethylphenyl)-N-phenylamino group, an N-(3,4-dimethylphenyl)-N-phenylamino group, an N-(3,5-dimethylphenyl)-N-phenylamino group, an N-(2,3,4-trimethylphenyl)-N-phenylamino group, an N-(2,3,5-trimethylphenyl)-N-phenylamino group, an N-(2,3,6-trimethylphenyl)-N-phenylamino group, an N-(2,4,5-trimethylphenyl)-N-phenylamino group, an N-(2,4,6-trimethylphenyl)-N-phenylamino group, an N-(3,4,5-trimethylphenyl)-N-phenylamino group, an N-(1-naphthyl)-N-phenylamino group, or an N-(2-naphthyl)-N-phenylamino group.

The present embodiment is the cyclohexylenediaminium salt represented by the formula (1) (hereinafter, also referred to as "the cyclohexylenediaminium salt of the present embodiment").

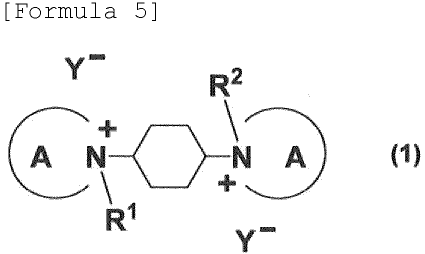

In the formula, R¹, R², the ring A, and Y⁻ as noted above.

In the cyclohexylenediaminium salt of the present embodiment, both R¹ and R² in the formula (1) preferably represent an alkyl group having 1 to 4 carbon atoms optionally substituted with one or more selected from the group consisting of a halogen atom, a hydroxy group, an alkoxy group having 1 to 4 carbon atoms, and a dialkylamino group having 2 to 4 carbon atoms. In the formula (1), both R¹ and R² preferably represent an alkyl group having 1 to 4 carbon atoms; the both more preferably represent a methyl group or an ethyl group; and the both further preferably represent a methyl group.

In the cyclohexylenediaminium salt of the present embodiment, all the ring A in the formula (1) preferably represent a five-membered or six-membered nitrogen-containing heterocycle optionally having a crosslinking or a condensed ring and optionally substituted with one or more substituents selected from the group consisting of a halogen atom, an alkyl group having 1 to 4 carbon atoms, a hydroxy group, an oxo group, a hydroxymethyl group, a methoxymethyl group, a trifluoromethyl group, a benzyl group, an ethoxycarbonyl group, a methoxycarbonyl group, an acylamino group having 1 to 8 carbon atoms optionally substituted on a nitrogen atom with an alkyl group having 1 to 4 carbon atoms, an alkyloxycarbonylamino group having 1 to 5 carbon atoms, an aminocarbonyl group having 1 to 9 carbon atoms, and a dialkylamino group having 2 to 6 carbon atoms; the all more preferably represent a five-membered or six-membered nitrogen-containing heterocycle optionally having a crosslinking or a condensed ring and optionally substituted with one or more substituents selected from the group consisting of a halogen atom and an alkyl group having 1 to 4 carbon atoms; the all more preferably represent a five-membered or six-membered nitrogen-containing heterocycle optionally substituted with one or more substituents selected from the group consisting of a methyl group, an ethyl group, a methoxy group, an ethoxy group, a hydroxy group, and a halogen atom; the all further preferably represent an unsubstituted five-membered or six-membered nitrogen-containing heterocycle optionally having a crosslinking or a condensed ring; the all further preferably represent a pyrrolidine ring or a piperidine ring optionally substituted with one or more substituents selected from the group consisting of a halogen atom, an alkyl group having 1 to 4 carbon atoms, a hydroxy group, an oxo group, a hydroxymethyl group, a methoxymethyl group, a trifluoromethyl group, a benzyl group, an ethoxycarbonyl group, a methoxycarbonyl group, an acylamino group having 1 to 8 carbon atoms optionally substituted on a nitrogen atom with an alkyl group having 1 to 4 carbon atoms, an alkyloxycarbonylamino group having 1 to 5 carbon atoms, an aminocarbonyl group having 1 to 9 carbon atoms, and a dialkylamino group having 2 to 6 carbon atoms; the all furthermore preferably represent a pyrrolidine ring or a piperidine ring optionally substituted with one or more substituents selected from the group consisting of a halogen atom and an alkyl group having 1 to 4 carbon atoms; and the all particularly preferably represent an unsubstituted pyrrolidine ring or an unsubstituted piperidine ring.

The cyclohexylenediaminium salt of the present embodiment represented by the formula (1) encompasses the cyclohexylenediaminium salt (1da) in which Y⁻ in the formula (1) is represented as Y'²⁻ being a divalent anion and the cyclohexylenediaminium salt (1ta) in which Y⁻ in the formula (1) is represented as Y"³⁻ being a trivalent anion, and the aforementioned preferable form of R¹, R², and the ring A in the formula (1) can be similarly applied for the formula (1da) and the formula (1ta).

In the cyclohexylenediaminium salt of the present embodiment, all Y⁻ in the formula (1) preferably represent a chloride ion (Cl⁻), a bromide ion (Br⁻), an iodide ion (I⁻), a sulfonate ion, a methyl sulfate ion (CH₃OSO₂O⁻), an ethyl sulfate ion (CH₃CH₂OSO₂O⁻), a trifluoromethanesulfonate ion, a methanesulfonate ion (CH₃SO₂O⁻), a benzenesulfonate ion (C₆H₅SO₂O⁻), a p-toluenesulfonate ion (p-CH₃C₆H₄SO₂O⁻), a fluorosulfonate ion, a trifluoromethanesulfonate ion (CF₃SO₂O⁻), a phenyl sulfate ion, a formate ion, an acetate ion, a propionate ion, a trifluoroacetate ion, a benzoate ion, a 4-methylbenzoate ion, a methyl carbonate ion (CH₃OCOO⁻), an ethyl carbonate ion (CH₃CH₂OCOO⁻), a sulfate ion, a carbonate ion, or a hydroxide ion (OH⁻); the all more preferably represent a chloride ion, a bromide ion, an iodide ion, a methyl sulfate ion, an ethyl sulfate ion, a methyl carbonate ion, an ethyl carbonate ion, a benzenesulfonate ion, a p-toluenesulfonate ion, a methanesulfonate ion, a trifluoromethanesulfonate ion, or a hydroxide ion; and the all further preferably a chloride ion, a bromide ion, a methyl sulfate ion, a sulfate ion, or a hydroxide ion.

Examples of a preferable form of the cyclohexylenediaminium salt of the present embodiment include cyclohexylenediaminium salts in which, in the formula (1), both R¹ and R² represent a methyl group, an ethyl group, a propyl group, an isopropyl group, or a butyl group, in which all the rings A represent a pyrrolidine ring, a 2-methylpyrrolidine ring, a 3,4-dimethylpyrrolidine ring, a 2,5-dimethylpyrrolidine ring, a 3-hydroxypyrrolidine ring, a piperidine ring, a 4-methylpiperidine ring, a 4-methylpiperazine ring, or a morpholine ring, and in which all Y⁻ represent a chloride ion, a bromide ion, an iodide ion, a methanesulfonate ion, a trifluoromethanesulfonate ion, an acetate ion, a benzoate ion, a benzenesulfonate ion, a methyl sulfate ion, an ethyl sulfate ion, a sulfate ion, a carbonate ion, a methyl carbonate ion, an ethyl carbonate ion, or a hydroxide ion. Examples of a more preferable form of the cyclohexylenediaminium salt of the present embodiment include cyclohexylenediaminium salts in which, in the formula (1), both R¹ and R² represent a methyl group or an ethyl group, in which all the rings A represent a pyrrolidine ring, a piperidine ring, a 4-methylpiperidine ring, or a morpholine ring, and in which all Y⁻ represent a chloride ion, an iodide ion, a methyl sulfate ion, a sulfate ion, or a hydroxide ion.

The cyclohexylenediaminium salt of the present embodiment is preferably a cyclohexylenediaminium salt represented by at least any one of the formula (1-1'), the formula (1-2'), the formula (1-3'), and the formula (1-4'). In the present specification, Me represents a methyl group, Et represents an ethyl group, Ph represents a phenyl group, TsO⁻ represents a p-toluenesulfonate ion, TsO⁻ represents a p-toluenesulfonate ion, TfO⁻ represents a trifluoromethanesulfonate ion, PhSO₂O⁻ represents a benzenesulfonate ion, MeSO₂O⁻ represents a methanesulfonate ion, MeCOO⁻ represents an acetate ion, CF₃COO⁻ represents a trifluoroacetate ion, PhCOO⁻represents a benzoate ion, MeOSO₂O⁻ represents a methyl sulfate ion, and EtOSO₂O⁻ represents an ethyl sulfate ion.

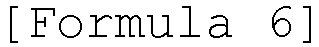

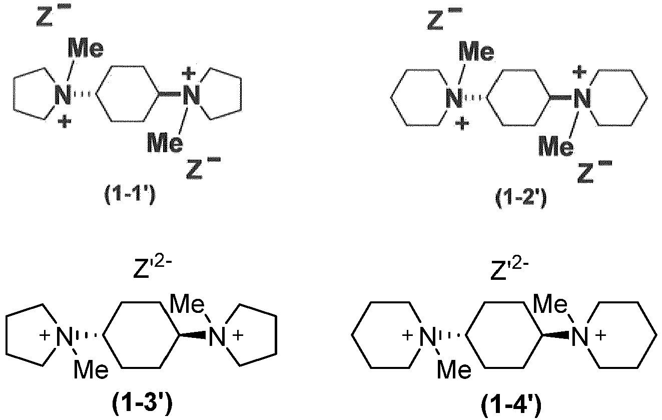

In the formula (1-1') and the formula (1-2'), Z⁻each independently represents a chloride ion, a bromide ion, an iodide ion, a methyl sulfate ion, or a hydroxide ion. In the formula (1-3') and the formula (1-4'), Z'²⁻represents a sulfate ion.

All Z⁻ in the formula (1-1') and the formula (1-2') preferably represent a chloride ion, a bromide ion, an iodide ion, a methyl sulfate ion, or a hydroxide ion.

Further, the cyclohexylenediaminium salt of the present embodiment is preferably the cyclohexylenediaminium salt represented by the formula (1-2') or the formula (1-4') to the cyclohexylenediaminium salt represented by the formula (1-1') or the formula (1-3'); and more preferably the cyclohexylenediaminium salt represented by the formula (1-2').

The cyclohexylenediaminium salt of the present embodiment is preferably a cyclohexylenediaminium salt represented by one or more selected from the group consisting of the following formulae (1-1) to (1-56).

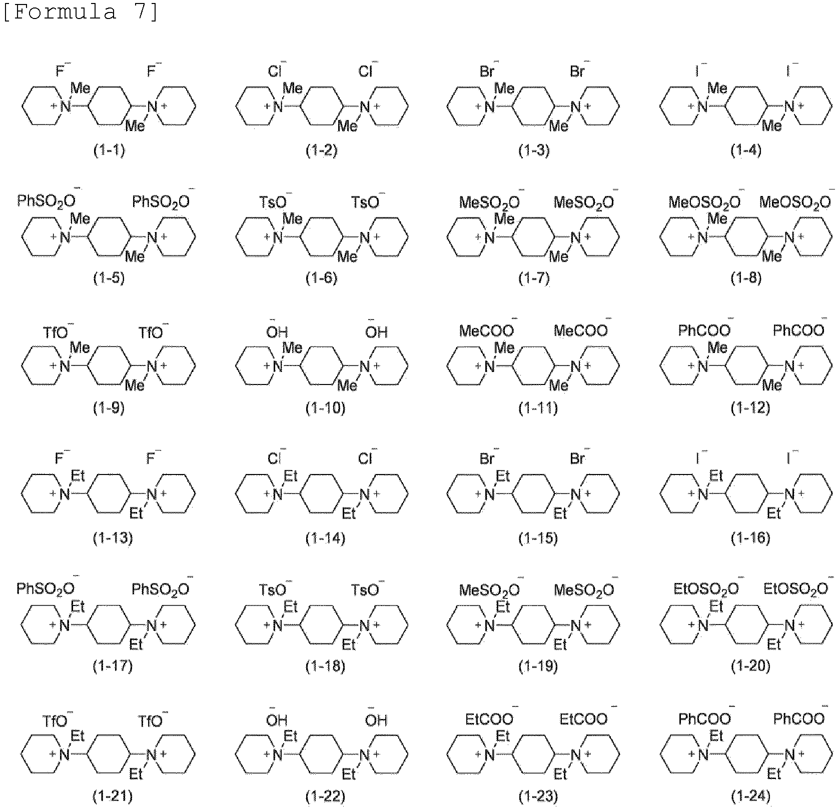

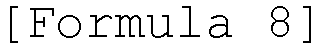

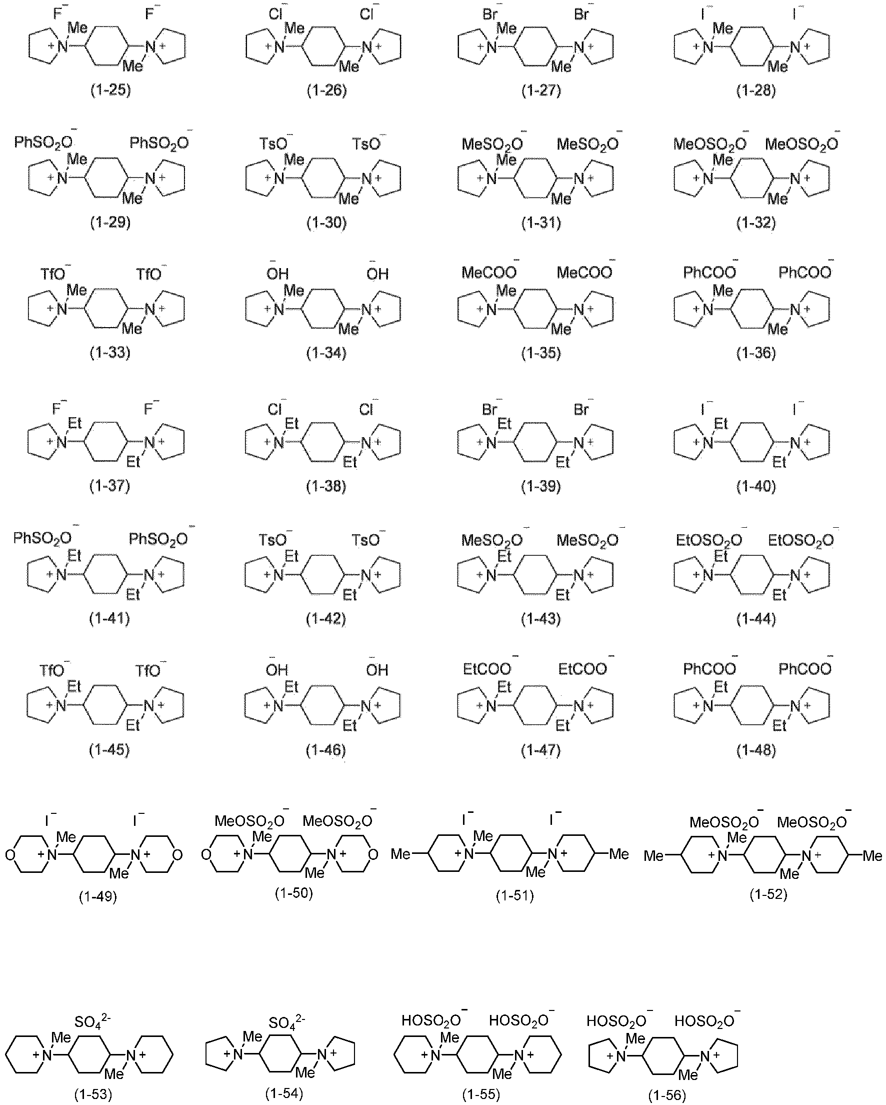

Among the cyclohexylenediaminium salts represented by the formulae (1-1) and (1-56), a more preferable cyclohexylenediaminium salt of the present embodiment is a cyclohexylenediaminium salt represented by the formula (1-2), (1-3), (1-4), (1-7), (1-8), (1-9), (1-10), (1-14), (1-15), (1-16), (1-19), (1-20), (1-22), (1-26), (1-27), (1-28), (1-31), (1-32), (1-33), (1-34), (1-38), (1-39), (1-40), (1-43), (1-44), (1-46), (1-49), (1-50), (1-51), (1-52), (1-53), or (1-54). A further preferable cyclohexylenediaminium salt of the present embodiment is a cyclohexylenediaminium salt represented by the formula (1-2), (1-3), (1-4), (1-7), (1-8), (1-10), (1-14), (1-15), (1-16), (1-19), (1-20), (1-22), (1-26), (1-27), (1-28), (1-31), (1-32), (1-34), (1-38), (1-39), (1-40), (1-43), (1-44), (1-46), (1-49), (1-51), (1-53), or (1-54), and a particularly preferable cyclohexylenediaminium salt of the present embodiment is a cyclohexylenediaminium salt represented by the formula (1-2), (1-4), (1-8), (1-10), (1-16), (1-26), (1-28), (1-32), (1-34), (1-40), (1-49), (1-51), or (1-54).

The cyclohexylenediaminium salt of the present embodiment is further preferably a cyclohexylenediaminium salt represented by one or more selected from the group consisting of (1-1t) to (1-56t).

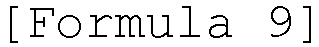

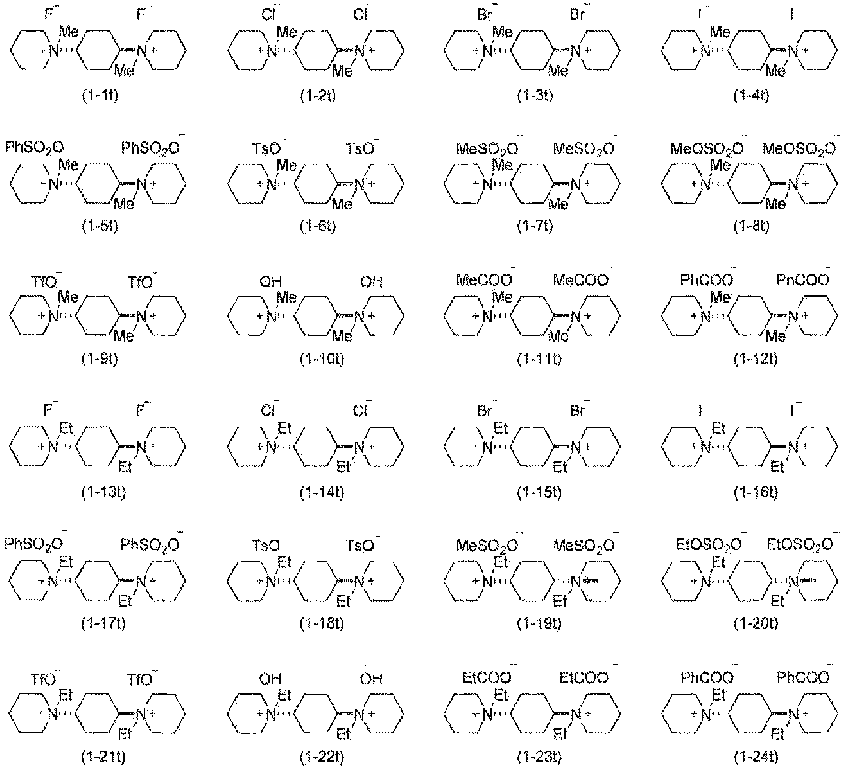

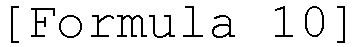

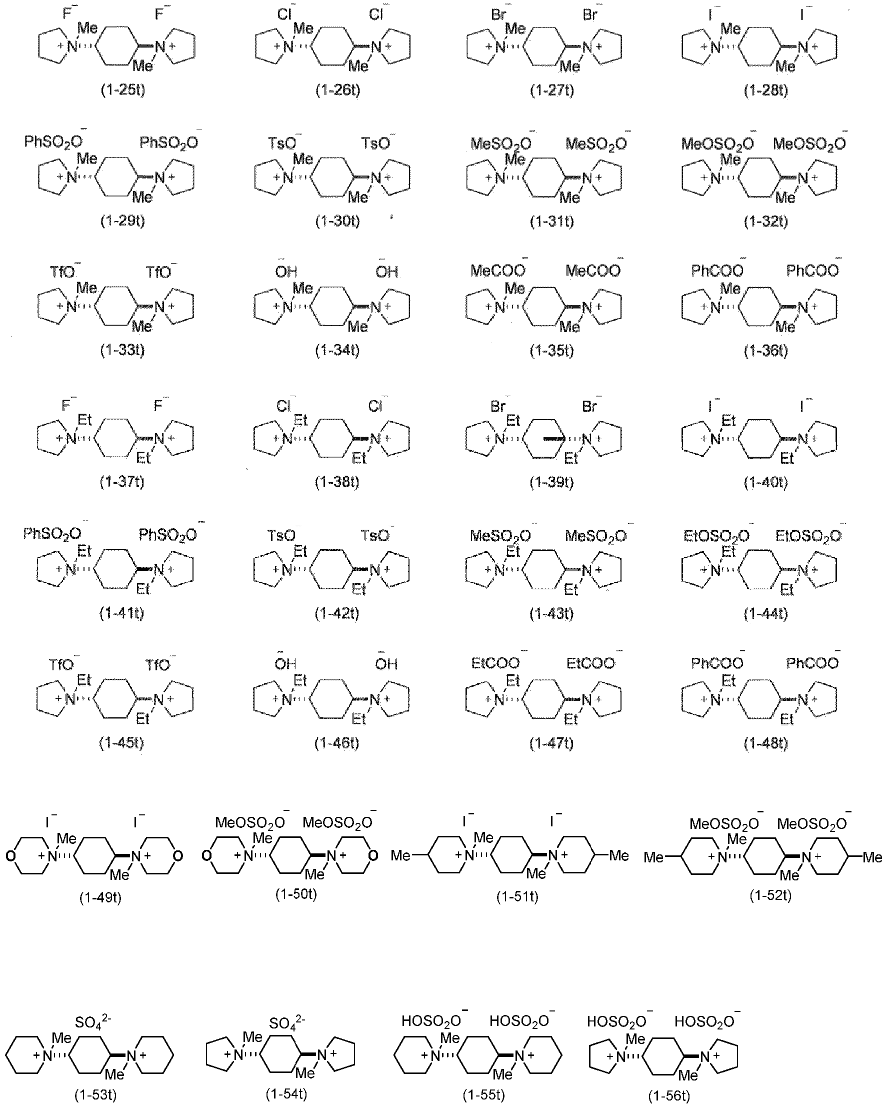

Among the cyclohexylenediaminium salts represented by the formulae (1-1t) to (1-56t), the cyclohexylenediaminium salt of the present embodiment is furthermore preferably a cyclohexylenediaminium salt represented by the formula (1-2t), (1-3t), (1-4t), (1-7t), (1-8t), (1-9t), (1-10t), (1-14t), (1-15t), (1-16t), (1-19t), (1-20t), (1-22t), (1-26t), (1-27t), (1-28t), (1-31t), (1-32t), (1-33t), (1-34t), (1-38t), (1-39t), (1-40t), (1-43t), (1-44t), (1-46t), (1-49t), (1-50t), (1-51t), (1-52t), (1-53t), or (1-54t). A furthermore preferable cyclohexylenediaminium salt of the present embodiment is a cyclohexylenediaminium salt represented by the formula (1-2t), (1-3t), (1-4t), (1-7t), (1-8t), (1-10t), (1-14t), (1-15t), (1-16t), (1-19t), (1-20t), (1-22t), (1-26t), (1-27t), (1-28t), (1-31t), (1-32t), (1-34t), (1-38t), (1-39t), (1-40t), (1-43t), (1-44t), (1-46t), (1-49t), (1-51t), (1-53t), or (1-54t).

The cyclohexylenediaminium salt of the present embodiment is particularly preferably a cyclohexylenediaminium salt represented by the formula (1-2t), (1-4t), (1-8t), (1-10t), (1-16t), (1-26t), (1-28t), (1-32t), (1-34t), (1-40t), (1-49t), (1-51t), or (1-54t).

### <Method for producing Cyclohexylenediaminium Salt (1) >

Next, a method for producing the cyclohexylenediaminium salt of the present embodiment (hereinafter, also referred to as "the producing method of the present embodiment") will be described.

### <Embodiment 1 of Producing Method>

The cyclohexylenediaminium salt of the present embodiment can be produced by a producing method comprising a step of reacting a diamine compound represented by the formula (2) (hereinafter, also referred to as "diamine (2)"), an alkylating agent represented by the formula (3a) (hereinafter, also referred to as "alkylating agent (3a)"), and an alkylating agent represented by the formula (3b) (hereinafter, also referred to as "alkylating agent (3b)")**.**

The producing method of the present embodiment comprises the step of reacting the diamine (2), the alkylating agent (3a), and the alkylating agent (3b) (hereinafter, also referred to as "reacting step 1"). This step yields a reaction product comprising a cyclohexylenediaminium salt represented by the formula (1a) (hereinafter, also referred to as "cyclohexylenediaminium salt (1a)"). The reaction in the reacting step 1 can be represented by the following reaction formula.

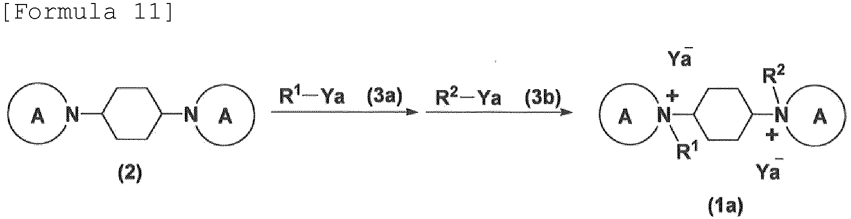

In the formula (2), the ring A is as noted above. In the formula (3a), R¹ and Ya are as noted above. In the formula (3b), R² and Ya are as noted above. In the formula (1a), R¹, R², the ring A, and Ya⁻ are as noted above.

The diamine (2) may be any diamine compound represented by the formula (2), and all the rings A in the formula (2) preferably represent a five-membered or six-membered nitrogen-containing heterocycle optionally having a crosslinking or a condensed ring and optionally substituted with one or more substituents selected from the group consisting of a halogen atom, an alkyl group having 1 to 4 carbon atoms, a hydroxy group, an oxo group, a hydroxymethyl group, a methoxymethyl group, a trifluoromethyl group, a benzyl group, an ethoxycarbonyl group, a methoxycarbonyl group, an acylamino group having 1 to 8 carbon atoms optionally substituted on a nitrogen atom with an alkyl group having 1 to 4 carbon atoms, an alkyloxycarbonylamino group having 1 to 5 carbon atoms, an aminocarbonyl group having 1 to 9 carbon atoms, and a dialkylamino group having 2 to 6 carbon atoms; the all more preferably represent a five-membered or six-membered nitrogen-containing heterocycle optionally having a crosslinking or a condensed ring and optionally substituted with one or more substituents selected from the group consisting of a halogen atom and an alkyl group having 1 to 4 carbon atoms; the all more preferably represent a five-membered or six-membered nitrogen-containing heterocycle optionally substituted with one or more substituents selected from the group consisting of a methyl group, an ethyl group, a methoxy group, an ethoxy group, a hydroxy group, and a halogen atom; the all further preferably represent an unsubstituted five-membered or six-membered nitrogen-containing heterocycle optionally having a crosslinking or a condensed ring; the all further preferably represent a pyrrolidine ring or a piperidine ring optionally substituted with one or more substituents selected from the group consisting of a halogen atom, an alkyl group having 1 to 4 carbon atoms, a hydroxy group, an oxo group, a hydroxymethyl group, a methoxymethyl group, a trifluoromethyl group, a benzyl group, an ethoxycarbonyl group, a methoxycarbonyl group, an acylamino group having 1 to 8 carbon atoms optionally substituted on a nitrogen atom with an alkyl group having 1 to 4 carbon atoms, an alkyloxycarbonylamino group having 1 to 5 carbon atoms, an aminocarbonyl group having 1 to 9 carbon atoms, and a dialkylamino group having 2 to 6 carbon atoms; the all furthermore preferably represent a pyrrolidine ring or a piperidine ring optionally substituted with one or more substituents selected from the group consisting of a halogen atom and an alkyl group having 1 to 4 carbon atoms; and the all furthermore preferably represent an unsubstituted pyrrolidine ring or an unsubstituted piperidine ring. The diamine (2) is preferably a diamine compound obtained by a method for producing a diamine compound represented by the formula (2) described later.

The alkylating agent (3a) and the alkylating agent (3b) are respectively compounds represented by the formula (3a) and the formula (3b), and both R¹ and R² in the formula (3a) and the formula (3b) preferably represent an alkyl group having 1 to 4 carbon atoms optionally substituted with one or more selected from the group consisting of a halogen atom, a hydroxy group, an alkoxy group having 1 to 4 carbon atoms, and a dialkylamino group having 2 to 4 carbon atoms. In the formula (3a) and the formula (3b), both R¹ and R² preferably represent an alkyl group having 1 to 4 carbon atoms; the both more preferably represent a methyl group or an ethyl group; and the both further preferably represent a methyl group. The alkylating agent (3a) and the alkylating agent (3b) may be different from each other, but preferably the same alkylating agent.

A preferable embodiment of the aforementioned reacting step 1 is a step of reacting: a diamine (2) in which all the rings A in the formula (2) represent a pyrrolidine ring, a 2-methylpyrrolidine ring, a 3,4-dimethylpyrrolidine ring, a 2,5-dimethylpyrrolidine ring, a 3-hydroxypyrrolidine ring, a piperidine ring, a 4-methylpiperidine ring, a 4-methylpiperazine ring, or a morpholine ring; an alkylating agent (3a) in which, in the formula (3a), R¹ represents a methyl group, an ethyl group, a propyl group, an isopropyl group, or a butyl group, and in which Ya represents a chlorine atom, a bromine atom, an iodine atom, a sulfonyloxy group represented by MeSO₂O-, a sulfonyloxy group represented by CF₃SO₂O-, a sulfonyloxy group represented by PhSO₂O-, a sulfonyloxy group represented by MeOSO₂O-, a sulfonyloxy group represented by EtOSO₂O-, a carboxy group represented by MeCOO-, or a carboxy group represented by EtCOO-; and an alkylating agent (3b) that is the same substance as the alkylating agent (3a). A more preferable embodiment of the aforementioned reacting step 1 is a step of reacting: a diamine (2) in which all the rings A in the formula (2) represent a pyrrolidine ring, a piperidine ring, a 4-methylpiperidine ring, or a morpholine ring; an alkylating agent (3a) in which, in the formula (3a), R¹ represents a methyl group or an ethyl group and Ya represents an iodine atom or a sulfonyloxy group represented by MeOSO₂O-; and an alkylating agent (3b) that is the same substance as the alkylating agent (3a).

Amounts of the diamine (2), the alkylating agent (3a), and the alkylating agent (3b) subjected to the reacting step 1 may be set so that a total amount of substance [mol] of the alkylating agent (3a) and the alkylating agent (3b) is 1.0 or more relative to an amount of substance [mol] of the diamine (2) in any cases of the aforementioned reacting step 1. Since a yield of the cyclohexylenediaminium salt (1a) tends to be high, a range of the ratio of the total amounts of substance [mol] of the alkylating agent (3a) and the alkylating agent (3b) relative to the amount of substance of the diamine (2) is preferably 1.0 or more and 200 or less, more preferably 1.0 or more and 40 or less, and furthermore preferably 2.0 or more and 10 or less in any of the aforementioned reacting step 1.

The reaction of the diamine (2), the alkylating agent (3a), and the alkylating agent (3b) is preferably performed in a solvent in any cases of the aforementioned reacting step 1. The solvent usable for the reacting step 1 may be any solvent that does not inhibit the reaction, and examples thereof include one or more solvents selected from the group consisting of aromatic hydrocarbon solvents, ether solvents, ester solvents, halogen solvents, amide solvents, urea solvents, ketone solvents, nitrile solvents, sulfur-containing compound solvents, alcohols, and water. Examples of specific solvents include: toluene or xylene as the aromatic hydrocarbon solvents; diethyl ether, diisopropyl ether, tetrahydrofuran, cyclopentyl methyl ether, tert-butyl methyl ether, 1,2-dimethoxyethane, or 1,4-dioxane as the ether solvents; ethyl acetate or butyl acetate as the ester solvents; chloroform, carbon tetrachloride, or chlorobenzene as the halogen solvents; N,N-dimethylformamide or N,N-dimethylacetamide as the amide solvents; 1,3-dimethyl-2-imidazolidinone or 1,3-dimethyl-3,4,5,6-tetrahydropyrimidin-2(1H)-one as the urea solvents; acetone or methyl ethyl ketone as the ketone solvents; acetonitrile, propionitrile, or benzonitrile as the nitrile solvents; dimethyl sulfoxide or sulfolane as the sulfur-containing compound solvents; and methanol, ethanol, propanol, butanol, or isopropanol as the alcohols. These solvents may be used singly, or two or more types thereof may be mixed at any ratio for use.

Since the yield of the cyclohexylenediaminium salt (1a) tends to be high, the solvent usable in the reacting step 1 is preferably the halogen solvents, the nitrile solvents, or the alcohols, and more preferably dichloromethane, chloroform, acetonitrile, ethanol, or methanol.

The reaction temperature for the diamine (2), the alkylating agent (3a), and the alkylating agent (3b) may be any temperature to proceed the aforementioned reaction in any cases of the aforementioned reacting step 1, and examples thereof include -80°C or higher, -20°C or higher, 0°C or higher, or 20°C or higher, and 200°C or lower or 80°C or lower. Examples of a range of the reaction temperature for the diamine (2), the alkylating agent (3a), and the alkylating agent (3b) include -80°C or higher and 200°C or lower, -20°C or higher and 80°C or lower, or 0°C or higher and 80°C or lower in any cases of the aforementioned reacting step 1.

The reaction time for the diamine (2), the alkylating agent (3a), and the alkylating agent (3b) may be appropriately regulated depending on the amounts of the diamine (2), the alkylating agent (3a), and the alkylating agent (3b) that are subjected to the reaction in any cases of the aforementioned reacting step 1, and examples thereof include 1 hour or longer and 100 hours or shorter.

The producing method of the present embodiment may further include, in addition to the aforementioned reacting step 1, at least one of: a step of isolating the cyclohexylenediaminium salt (1a) (hereinafter, also referred to as "isolating step 1"); and a step of ion-exchanging the cyclohexylenediaminium salt (1a) (hereinafter, also referred to as "ion-exchanging step 1").

In the isolating step 1, the cyclohexylenediaminium salt (1a) is isolated from the reaction product obtained in the reacting step 1. The isolating method may be any method by which the cyclohexylenediaminium salt (1a) can be isolated from the reaction product, and the isolation may be performed by a known isolating method. Examples of the isolating method include commonly used methods by which a person skilled in the art purifies an organic ammonium salt, such as filtration, decantation, centrifugal separation, solvent extraction, column chromatography, preparative thin layer chromatography, preparative liquid chromatography, recrystallization, and reprecipitation.

In the ion-exchanging step 1, the cyclohexylenediaminium salt (1a) obtained in the reacting step 1 or the cyclohexylenediaminium salt (1a) obtained in the isolating step 1 is subjected to ion-exchange to yield a cyclohexylenediaminium salt represented by the formula (1b) (hereinafter, also referred to as "cyclohexylenediaminium salt (1b)").

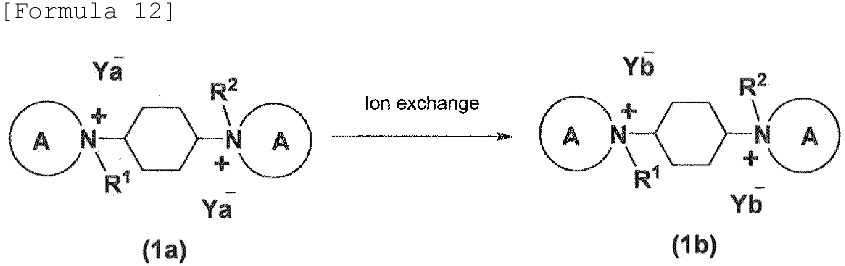

In the formula (1a), R¹, R², the ring A, and Ya⁻ are as noted above. In the formula (1b), R¹, R², the ring A, and Yb⁻ are as noted above.

The method for ion-exchanging the cyclohexylenediaminium salt (1a) may be any method by which the cyclohexylenediaminium salt (1a) is converted into a desired ion-exchanged product, and may be any known ion-exchanging method. Examples of the method for ion-exchanging the cyclohexylenediaminium salt (1a) include a method of contacting an ion exchange resin or at least one of a solution of hydrochloric acid and hydrobromic acid (hereinafter, also referred to as "ion-exchanging solution") with the cyclohexylenediaminium salt (1a).

The ion exchange resin may be any ion exchange resin having a desired exchanging group Yb⁻. Examples thereof include Amberlite IRA-400, Amberlite IRA-402, Amberlite IRA-404, Amberlite IRA-900, Amberlite IRA-904, Amberlite IRA-458, Amberlite IRA-958, Amberlite IRA-420, Amberlite IRN-78, Amberlite IRA-411, Amberlite IRA-910, and Ambersep 900, and Amberlite IRA-900 or Ambersep 900 is preferable.

The ion-exchanging solution used for ion-exchanging the cyclohexylenediaminium salt (1a) may be any solution having a desired exchanging group Yb⁻. Examples thereof include hydrochloric acid, a hydrochloric acid - ethyl acetate solution, a hydrochloric acid - 1,4-dioxane solution, a hydrochloric acid - cyclopentyl methyl ether solution, a hydrochloric acid - 2-propanol solution, a hydrochloric acid - methanol solution, a hydrochloric acid - acetic acid solution, hydrobromic acid, or a hydrobromic acid - acetic acid solution, and hydrochloric acid or hydrochloric acid - ethyl acetate is preferable.

The ion-exchange of the cyclohexylenediaminium salt (1a) is preferably performed in a solvent. The solvent used for ion-exchanging the cyclohexylenediaminium salt (1a) may be any solvent that does not inhibit the ion-exchange, and examples thereof include the ether solvents, the ester solvents, the ketone solvents, the nitrile solvents, the alcohol solvents, or water that are exemplified in the description of the aforementioned reacting step 1.

### <Embodiment 2 of Producing Method>

The cyclohexylenediaminium salt of the present embodiment may also be produced by another producing method, not limited to the aforementioned producing method of embodiment 1. For example, the cyclohexylenediaminium salt of the present embodiment can also be produced by a producing method comprising a step of reacting a diamine compound represented by the formula (2) (hereinafter, also referred to as "diamine (2)") and an alkylating agent represented by the formula (3c) (hereinafter, also referred to as "alkylating agent (3c)") .

The producing method of the present embodiment comprises the step of reacting the diamine (2) and the alkylating agent (3c) (hereinafter, also referred to as "reacting step 2"). This step yields a reaction product comprising a cyclohexylenediaminium salt represented by the following formula (la') (hereinafter, also referred to as "cyclohexylenediaminium salt (1a')"). The reaction in the reacting step 2 can be represented by the following reaction formula.

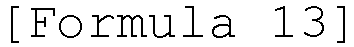

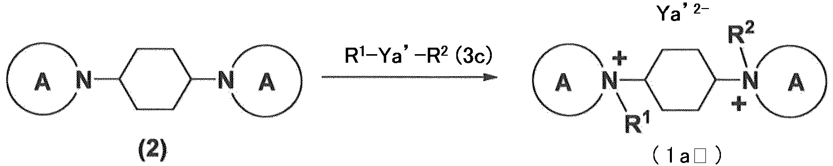

In the formula (2), the ring A is as noted above. In the formula (3c), R¹, R², and Ya' are as noted above. In the formula (1a'), R¹, R², the ring A, and Ya'²⁻ are as noted above.

The diamine (2) may be any diamine compound represented by the formula (2), and a preferable diamine (2) is same as the preferable diamine (2) in the aforementioned reacting step 1 of the embodiment 1.

The alkylating agent (3c) is a compound represented by the formula (3c), and in the formula (3c), both R¹ and R² preferably represent an alkyl group having 1 to 4 carbon atoms optionally substituted with one or more selected from the group consisting of a halogen atom, a hydroxy group, an alkoxy group having 1 to 4 carbon atoms, and a dialkylamino group having 2 to 4 carbon atoms. In the formula (3c), both R¹ and R² preferably represent an alkyl group having 1 to 4 carbon atoms; the both more preferably represent a methyl group or an ethyl group; and the both further preferably represent a methyl group.

A preferable embodiment of the aforementioned reacting step 2 is a step of reacting: a diamine (2) in which all the rings A in the formula (2) represent a pyrrolidine ring, a 2-methylpyrrolidine ring, a 3,4-dimethylpyrrolidine ring, a 2,5-dimethylpyrrolidine ring, a 3-hydroxypyrrolidine ring, a piperidine ring, a 4-methylpiperidine ring, a 4-methylpiperazine ring, or a morpholine ring; and an alkylating agent (3c) in which, in the formula (3c), R¹ and R² both represent a methyl group, an ethyl group, a propyl group, an isopropyl group, or a butyl group, and in which Ya' represents a sulfonyloxy group represented by -OSO₂O- or a carboxy group represented by -COO-. A more preferable embodiment of the aforementioned reacting step 2 is a step of reacting: a diamine (2) in which all the rings A in the formula (2) represent a pyrrolidine ring or a piperidine ring; and an alkylating agent (3c) in which, in the formula (3c), R¹ and R² both represent a methyl group and Ya' represents a sulfonyloxy group represented by - OSO₂O-.

Amounts of the diamine (2) and the alkylating agent (3c) subjected to the reacting step 2 may be set so that a ratio of an amount of substance [mol] of the alkylating agent (3c) relative to an amount of substance [mol] of the diamine (2) is 0.2 or more in any cases of the aforementioned reacting step 2. Since a yield of the cyclohexylenediaminium salt (la') tends to be high, a range of the ratio of the amount of substance [mol] of the alkylating agent (3c) relative to the amount of substance of the diamine (2) is preferably 0.2 or more and 10 or less, more preferably 0.3 or more and 5.0 or less, and furthermore preferably 0.5 or more and 2.0 or less in any of the aforementioned reacting step 2.

The reaction of the diamine (2) and the alkylating agent (3c) is preferably performed in a solvent in any cases of the aforementioned reacting step 2. The solvent usable in the reacting step 2 is same as the solvent usable in the aforementioned reacting step 1 of the embodiment 1. The reaction temperature and the reaction time for the diamine (2) and the alkylating agent (3c) in the reacting step 2 are respectively same as the reaction temperature and the reaction time in the aforementioned reacting step 1 of the embodiment 1.

The producing method of the present embodiment may further comprise, in addition to the aforementioned reacting step 2, at least one of: a step of isolating the cyclohexylenediaminium salt (la') (hereinafter, also referred to as "isolating step 2"); and a step of ion-exchanging the cyclohexylenediaminium salt (la') (hereinafter, also referred to as "ion-exchanging step 2"). The isolating step 2 and the ion-exchanging step 2 are respectively same as the aforementioned isolating step 1 and ion-exchanging step 1 of the embodiment 1.

### <Embodiment 3 of Producing Method>

The cyclohexylenediaminium salt of the present embodiment may also be produced by another producing method, not limited to the aforementioned producing method of embodiment 1. For example, the cyclohexylenediaminium salt of the present embodiment can also be produced by a producing method comprising a step of reacting a diamine compound represented by the formula (2) (hereinafter, also referred to as "diamine (2)") and an alkylating agent represented by the formula (3d) (hereinafter, also referred to as "alkylating agent (3d)").

The producing method of the present embodiment comprises the step of reacting the diamine (2) and the alkylating agent (3d) (hereinafter, also referred to as "reacting step 3"). This step yields a reaction product comprising a cyclohexylenediaminium salt represented by the following formula (1a'') (hereinafter, also referred to as "cyclohexylenediaminium salt (1a'')". The reaction in the reacting step 3 can be represented by the following reaction formula.

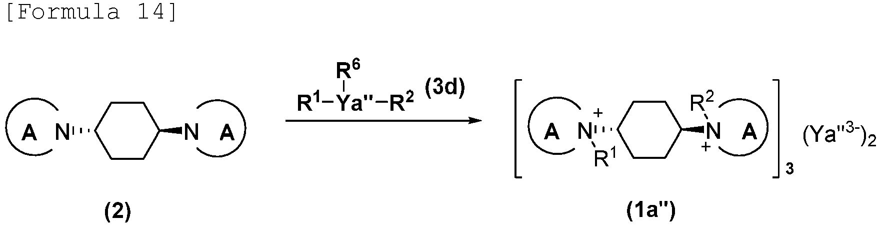

In the formula (2), the ring A is as noted above. In the formula (3d), R¹, R², and Ya'' are as noted above. In the formula (1a"), R¹, R², the ring A, and Ya"³⁻ are as noted above. R⁶ represents an alkyl group having 1 to 4 carbon atoms optionally substituted with one or more substituents selected form the group consisting of a halogen atom, a hydroxy group, an alkoxy group having 1 to 4 carbon atoms, and a dialkylamino group having 2 to 8 carbon atoms.

R⁶ in the formula (1a'') is same as R¹ and R² in the formula (1), and specific substituents and preferable substituents are similar to the specific substituents and the preferable substituents of R¹ and R² in the aforementioned formula (1).

The diamine (2) may be any diamine compound represented by the formula (2), and a preferable diamine (2) is same as the preferable diamine (2) in the aforementioned reacting step 1 of the embodiment 1.

The alkylating agent (3d) is a compound represented by the formula (3d), and in the formula (3d), all of R¹, R², and R⁶ preferably represent an alkyl group having 1 to 4 carbon atoms optionally substituted with one or more selected from the group consisting of a halogen atom, a hydroxy group, an alkoxy group having 1 to 4 carbon atoms, and a dialkylamino group having 2 to 4 carbon atoms. In the formula (3d), all of R¹, R², and R⁶ preferably represent an alkyl group having 1 to 4 carbon atoms; the all more preferably represent a methyl group or an ethyl group; and the all further preferably represent a methyl group.

A preferable embodiment of the aforementioned reacting step 3 is a step of reacting: a diamine (2) in which all the rings A in the formula (2) represent a pyrrolidine ring, a 2-methylpyrrolidine ring, a 3,4-dimethylpyrrolidine ring, a 2,5-dimethylpyrrolidine ring, a 3-hydroxypyrrolidine ring, a piperidine ring, a 4-methylpiperidine ring, a 4-methylpiperazine ring, or a morpholine ring; and an alkylating agent (3d) in which, in the formula (3d), all of R¹, R², and R⁶ represent a methyl group, an ethyl group, a propyl group, an isopropyl group, or a butyl group, and in which Ya'' represents a phosphoric acid group represented by O=P(O₃-). A more preferable embodiment of the aforementioned reacting step 3 is a step of reacting: a diamine (2) in which all the rings A in the formula (2) represent a pyrrolidine ring or a piperidine ring; and an alkylating agent (3d) in which, in the formula (3d), all of R¹, R², and R⁶ represent a methyl group and in which Ya'' represents a phosphoric acid group represented by O=P(O₃-).

Amounts of the diamine (2) and the alkylating agent (3d) subjected to the reacting step 3 may be set so that a ratio of an amount of substance [mol] of the alkylating agent (3d) is 0.1 or more relative to an amount of substance [mol] of the diamine (2) in any cases of the aforementioned reacting step 3. Since a yield of the cyclohexylenediaminium salt (1a") tends to be high, a range of the ratio of the amount of substance [mol] of the alkylating agent (3d) relative to the amount of substance of the diamine (2) is preferably 0.2 or more and 10 or less, more preferably 0.25 or more and 5.0 or less, and furthermore preferably 0.34 or more and 2.0 or less in any cases of the aforementioned reacting step 3.

The reaction of the diamine (2) and the alkylating agent (3d) is preferably performed in a solvent in any cases of the aforementioned reacting step 3. The solvent usable in the reacting step 3 is same as the solvent usable in the aforementioned reacting step 1 of the embodiment 1. The reaction temperature and the reaction time for the diamine (2) and the alkylating agent (3d) in the reacting step 3 are respectively same as the reaction temperature and the reaction time in the aforementioned reacting step 1 of the embodiment 1.

The producing method of the present embodiment may further comprise, in addition to the aforementioned reacting step 3, at least one of: a step of isolating the cyclohexylenediaminium salt (1a") (hereinafter, also referred to as "isolating step 3"); and a step of ion-exchanging the cyclohexylenediaminium salt (1a") (hereinafter, also referred to as "ion-exchanging step 3"). The isolating step 3 and the ion-exchanging step 3 are respectively same as the aforementioned isolating step 1 and ion-exchanging step 1 of the embodiment 1.

### <Method for producing Zeolite>

The cyclohexylenediaminium salt of the present embodiment can be applied for use of known quaternary ammonium salts. Examples of such use include at least one of a ligand for a transition metal catalyst and an organic structure-directing agent (hereinafter, also referred to as "SDA") for zeolite production. The cyclohexylenediaminium salt of the present embodiment can be used as a cyclohexylenediaminium salt for zeolite production, preferably used as an SDA for large pore zeolite production, and more preferably used as an SDA for MSE-type zeolite production.

Examples of the method for producing a zeolite using the cyclohexylenediaminium salt of the present embodiment (hereinafter, also referred to as "zeolite producing method of the present embodiment") include a method for producing a zeolite comprising a crystallizing step of crystallizing a composition comprising an organic structure-directing agent source containing the cyclohexylenediaminium salt represented by the formula (1), a silica source, an alumina source, an alkali source, and water. This method yields a zeolite comprising a cyclohexylenediaminium cation represented by the following formula (1'), specifically an MSE-type zeolite comprising the cyclohexylenediaminium cation represented by the following formula (1'). The zeolite producing method of the present embodiment yields a zeolite suitable for use of an adsorbent, specifically a zeolite suitable for use of a hydrocarbon adsorbent.

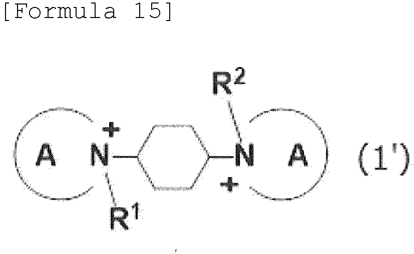

In the formula, R¹, R², and the ring A are as noted above.

"Aluminosilicate" is a composite oxide having a structure constituted by repetition of networks with aluminum (Al) and silicon (Si) via oxygen (O). Among the aluminosilicate, in a powder X-ray diffraction (hereinafter, also referred to as "XRD") pattern thereof, one having a crystalline XRD peak is "crystalline aluminosilicate", and one having no crystalline XRD peak is "amorphous aluminosilicate".

The XRD pattern in the present embodiment is measured with CuKα radiation as a radiation source, and the measurement condition can be as follows.
Acceleration current and voltage: 50 mA and 40 kV
Radiation source: CuKα radiation (λ=1.5405 Å)
Measurement mode: Continuous scan
Scanning condition: 50°/min
Measurement range: 2θ = 5° to 45°
Divergence vertical-limiting slit: 10 mm
Divergence / incidence slit: 1°
Photodetecting slit: open
Photodetecting solar slit: 5°
Detector: Semiconductor detector (D/teX Ultra)
Filter: Ni filter

The crystalline XRD peak is a peak with which 2θ of a peak top is specified and detected in analysis of the XRD pattern using a common analysis software (for example, SmartLab StudioII, manufactured by Rigaku Corporation). Examples of a half width (full width at half maximum) of the crystalline XRD peak is 2θ = 0.50° or less. The analysis condition of the XRD pattern can be as follows.
Fitting condition: automatic, refined background
   Variance pseudo-Voigt function (peak shape)
Method for removing background: Fitting method
Method for removing Kα2: Kα1/Kα2 ratio = 0.497
Smoothing method: B-Spline curve
Smoothing condition: secondary differential method, σ-cut value = 3, χ-threshold = 1.5

The composition of the present embodiment, such as the mole ratio of silica to alumina, can be measured by ICP analysis with a common inductively coupled plasma atomic emission spectrometer (for example, OPTIMA7300DV, manufactured by PERKIN ELMER).

The "zeolite" refers to a compound having a regular structure with a skeleton atom (hereinafter, also referred to as "T atom") via oxygen (O), wherein the T atom is a metal atom. The zeolite may comprise two or more metal atoms as the T atom. The metal atom in the present embodiment is at least one atom of metal elements and semimetal elements. Examples of the metal atom include one or more selected from the group consisting of iron (Fe), titanium (Ti), manganese (Mn), and aluminum (Al), and aluminum is preferable. Examples of the semimetal element include at least one selected from the group consisting of boron (B), silicon (Si), germanium (Ge), arsenic (As), antimony (Sb), and tellurium (Te), and silicon is preferable. Note that the zeolite in which the T atom is constituted by aluminum (Al) and silicon (Si) corresponds to the crystalline aluminosilicate. The crystalline aluminosilicate includes: an unsubstituted crystalline aluminosilicate in which the T atom is composed of only aluminum and silicon; and substituted crystalline aluminosilicate in which a part of aluminum and silicon constituting the T atom is substituted with another metal atom.

"Zeolite analogous substance" refers to a compound having a regular structure with the T atom via oxygen, and the compound comprises at least an atom other than a metal atom (hereinafter, "non-metal atom") as the T atom. As an example, the zeolite analogous substance comprises the metal atom and the non-metal atom as the T atom. Specific examples of the zeolite analogous substance include a composite phosphorus compound comprising phosphorus (P) as the T atom, such as aluminophosphate (AlPO) and silicoaluminophosphate (SAPO). The zeolite of the present embodiment preferably comprises no phosphorus as the T atom, and more preferably comprises no phosphorus.

The term "regular structure (hereinafter, also referred to as "zeolite structure") in the zeolite or the zeolite analogous substance refers to a skeleton structure specified by a structural code regulated by Structure Commission of International Zeolite Association (hereinafter, also simply referred to as "structural code"). For example, the MSE structure is a skeleton structure specified as the structural code "MSE". The zeolite structure can be identified by comparison with the XRD pattern of each structure described in Collection of simulated XRD powder patterns for zeolites, Fifth revised edition (2007) (hereinafter, also referred to as "referential pattern"). For example, the structure of a sample can be identified as MSE by extraction of peaks with "d" (Å) of 2 or more and 14 or less and a relative intensity (Iᵣₑₗ) of 20 or more ("d" (Å) = 13.559, 12.930, 10.891, 10.104, 9.143, and 4.089)) from the referential pattern of MSE described in the above literature (p. 290, MCM-68) and by presence of a measured peak of the sample within a range of a relative error of 4% or less of each "d" (Å) in 80% or more of the extracted peaks (hereinafter, also referred to as "referential peaks"). When a plurality of the measured peaks is present within the range of the "d" (Å) relative error of 4% or less of a certain referential peak, a measured peak having a smaller relative error is corresponded. As for the zeolite structure, the skeleton structure, the crystal structure, and the crystal phase is used as the same mean.

In the present embodiment, "-type zeolite" such as "MSE-type zeolite" means a zeolite having the zeolite structure of the structural code, preferably means a zeolite having only the zeolite structure of the structural code, and more preferably means crystalline aluminosilicate having the zeolite structure of the structural code.

Hereinafter, a method for producing an MSE-type zeolite will be exemplified and described as a method for producing a zeolite using the cyclohexylenediaminium salt of the present embodiment as the SDA.

The method for producing an MSE-type zeolite using the cyclohexylenediaminium salt of the present embodiment as the SDA (hereinafter, also referred to as "MSE-type zeolite producing method of the present embodiment") is a producing method comprising a crystallizing step of crystallizing a composition (hereinafter, also referred to as "raw material composition") comprising an organic structure-directing agent source containing the cyclohexylenediaminium salt represented by the formula (1), a silica source, an alumina source, an alkali source, and water (hereinafter, also simply referred to as "crystallizing step").

The silica source comprised in the raw material composition is a silicon compound to be silica (SiO₂) or a precursor thereof. Examples thereof include one or more selected from the group consisting of colloidal silica, amorphous silica, sodium silicate, tetraethyl orthosilicate, precipitation silica, fumed silica, crystalline aluminosilicate, and amorphous aluminosilicate. The silica source is preferably one or more selected from the group consisting of colloidal silica, crystalline aluminosilicate, amorphous aluminosilicate, and sodium silicate, more preferably one or more selected from the group consisting of colloidal silica, amorphous aluminosilicate, and sodium silicate, further preferably at least one of colloidal silica and amorphous aluminosilicate, and furthermore preferably colloidal silica.

The alumina source comprised in the raw material composition is an aluminum compound to be alumina (Al₂O₃) or a precursor thereof. Examples thereof include one or more selected from the group consisting of aluminum sulfate, sodium aluminate, aluminum hydroxide, aluminum hydroxide gel, aluminum chloride, amorphous aluminosilicate, crystalline aluminosilicate, and metal aluminum. The alumina silicate is preferably one or more selected from the group consisting of aluminum hydroxide, aluminum sulfate, crystalline aluminosilicate, and amorphous aluminosilicate, more preferably one or more selected from the group consisting of aluminum hydroxide, aluminum hydroxide gel, aluminum sulfate, and amorphous aluminosilicate, further preferably at least one of aluminum hydroxide gel and amorphous aluminosilicate, and furthermore preferably aluminum hydroxide gel.

The organic structure-directing agent source comprised in the raw material composition comprises the cyclohexylenediaminium salt of the present embodiment. The cyclohexylenediaminium salt of the present embodiment comprised in the organic structure-directing agent source may be any cyclohexylenediaminium salt as long as it is the aforementioned cyclohexylenediaminium salt of the present embodiment.

The organic structure-directing agent source comprised in the raw material composition preferably comprises one or more cyclohexylenediaminium salts represented by the formula (1-2), (1-3), (1-4), (1-7), (1-8), (1-9), (1-10), (1-14), (1-15), (1-16), (1-19), (1-20), (1-22), (1-26), (1-27), (1-28), (1-31), (1-32), (1-33), (1-34), (1-38), (1-39), (1-40), (1-43), (1-44), or (1-46), further preferably comprises one or more cyclohexylenediaminium salts represented by the formula (1-2), (1-3), (1-4), (1-7), (1-8), (1-10), (1-14), (1-15), (1-16), (1-19), (1-20), (1-22), (1-26), (1-27), (1-28), (1-31), (1-32), (1-34), (1-38), (1-39), (1-40), (1-43), (1-44), (1-46), (1-49), (1-50), (1-51), (1-52), (1-53), or (1-54), and particularly preferably comprises one or more cyclohexylenediaminium salts represented by the formula (1-2), (1-4), (1-8), (1-26), (1-28), (1-32), (1-34), (1-53), or (1-54).

The organic structure-directing agent source comprised in the raw material composition preferably comprises one or more cyclohexylenediaminium salts represented by the formula (1-2t), (1-3t), (1-4t), (1-7t), (1-8t), (1-9t), (1-10t), (1-14t), (1-15t), (1-16t), (1-19t), (1-20t), (1-22t), (1-26t), (1-27t), (1-28t), (1-31t), (1-32t), (1-33t), (1-34t), (1-38t), (1-39t), (1-40t), (1-43t), (1-44t), (1-46t), (1-49t), (1-50t), (1-51t), (1-52t), (1-53t), or (1-54t), more preferably comrises a cyclohexylenediaminium salt represented by the formula (1-2t), (1-3t), (1-4t), (1-7t), (1-8t), (1-10t), (1-14t), (1-15t), (1-16t), (1-19t), (1-20t), (1-22t), (1-26t), (1-27t), (1-28t), (1-31t), (1-32t), (1-34t), (1-38t), (1-39t), (1-40t), (1-43t), (1-44t), (1-46t), (1-49t), (1-51t), (1-53t), or (1-54t), and further preferably comprises a cyclohexylenediaminium salt represented by the formula (1-2t), (1-4t), (1-8t), (1-26t), (1-28t), (1-32t), (1-34t), (1-53t), or (1-54t).

The organic structure-directing agent source comprised in the raw material composition may be any as long as it comprises the cyclohexylenediaminium salt of the present embodiment, and may comprise only the cyclohexylenediaminium salt of the present embodiment as the SDA. Meanwhile, the raw material composition may further comprise a known SDA or another ammonium salt to direct the MSE-type zeolite in addition to the cyclohexylenediaminium salt of the present embodiment. The organic structure-directing agent source comprised in the raw material composition may further comprise, for example, at least any one of a TEBOP cation and a dimethyldipropylammonium cation as the known SDA to direct the MSE-type zeolite in addition to the cyclohexylenediaminium salt of the present embodiment.

The alkali source comprised in the raw material composition may be any compounds containing an alkali metal element, and examples thereof include at least any one of a hydroxide of an alkali metal and a halide of an alkali metal. The preferable alkali metal is: one or more selected from the group consisting of sodium, potassium, rubidium, and cesium; at least one of sodium and potassium; sodium and potassium; or potassium. Specific examples of the alkali source comprised in the raw material composition include one or more selected from the group consisting of sodium hydroxide, sodium chloride, potassium hydroxide, and potassium chloride, further include at least any one of potassium hydroxide and potassium chloride, and further include potassium hydroxide. The alkali source comprised in the raw material composition may be comprised in another starting material such as the silica source, and this may also be used as the alkali source comprised in the raw material composition. Examples of the starting material comprising the alkali source include colloidal silica, sodium silicate, and potassium silicate.

Examples of the water include pure water (ion exchanged water), ultrapure water, industrial water, tap water, or water as a solvent or structural water of the other starting materials such as the silica source.

In the MSE-type zeolite producing method of the present embodiment, SiO₂/Al₂O₃ in the raw material composition is preferably 8 or more or 10 or more, and preferably 100 or less, 50 or less, 40 or less, or 30 or less. The range of SiO₂/Al₂O₃ in the raw material composition is preferably 8 or more and 100 or less, 8 or more and 50 or less, 8 or more and 40 or less, 8 or more and 30 or less, or 10 or more and 30 or less, and particularly preferably 15 or more and 30 or less.

In the MSE-type zeolite producing method of the present embodiment, SDA/SiO₂ in the raw material composition is preferably 0.01 or more or 0.03 or more, and preferably 1.0 or less, 0.25 or less, or 0.15 or less. The range of SDA/SiO₂ in the raw material composition is preferably 0.01 or more and 1.0 or less, 0.01 or more and 0.25 or less, 0.01 or more and 0.15 or less, or 0.03 or more and 0.15 or less. The SDA in SDA/SiO₂ is the cyclohexylenediaminium salt of the present embodiment comprised in the raw material composition.

In the MSE-type zeolite producing method of the present embodiment, M/SiO₂ in the raw material composition is preferably 0.10 or more, 0.15 or more, 0.20 or more, or 0.25 or more, and preferably 1.0 or less, 0.60 or less, or 0.50 or less. The range of M/SiO₂ in the raw material composition is preferably 0.10 or more and 1.0 or less, 0.10 or more and 0.60 or less, 0.15 or more and 0.50 or less, 0.20 or more and 0.50 or less, or 0.25 or more and 0.50 or less. M in M/SiO₂ is the alkali metal comprised in the raw material composition.

In the MSE-type zeolite producing method of the present embodiment, OH/SiO₂ in the raw material composition is preferably 0.10 or more, 0.20 or more, or 0.30 or more, and preferably 1.0 or less, 0.80 or less, or 0.60 or less. The range of OH/SiO₂ in the raw material composition is preferably 0.10 or more and 1.0 or less, 0.10 or more and 0.80 or less, 0.10 or more and 0.60 or less, 0.20 or more and 0.60 or less, or 0.30 or more and 0.60 or less.

In the MSE-type zeolite producing method of the present embodiment, H₂O/SiO₂ in the raw material composition is preferably 5 or more, 8 or more, or 10 or more, and preferably 1000 or less, 200 or less, 80 or less, 60 or less, or 40 or less. The range of H₂O/SiO₂ in the raw material composition is preferably 5 or more and 1000 or less, 5 or more and 200 or less, 5 or more and 80 or less, 8 or more and 60 or less, or 10 or more and 40 or less.

In the MSE-type zeolite producing method of the present embodiment, the raw material composition preferably has any one combination of the following mole compositions.
SiO₂/Al₂O₃: 8 or more, or 10 or more, and
   100 or less, 50 or less, 40 or less, or 30 or less
SDA/SiO₂: 0.01 or more, or 0.03 or more, and
   1.0 or less, 0.25 or less, or 0.15 or less
M/SiO₂: 0.10 or more, 0.20 or more, or 0.25 or more, and
   1.0 or less, 0.60 or less, or 0.50 or less,
OH/SiO₂: 0.10 or more, 0.15 or more, 0.20 or more, or 0.30 or more, and
   1.0 or less, 0.80 or less, or 0.60 or less
H₂O/SiO₂: 5 or more, 8 or more, or 10 or more, and
   1000 or less, 200 or less, 80 or less, 60 or less, or 40 or less

In the above composition, the SDA is the cyclohexylenediaminium salt of the present embodiment comprised in the raw material composition, and M is the alkali metal comprised in the raw material composition. The alkali metal comprised in the raw material composition is preferably at least any one of sodium and potassium, and more preferably potassium.

Examples of a particularly preferable composition of the raw material composition is the following mole composition.
SiO₂/Al₂O₃: 15 or more and 30 or less
SDA/SiO₂: 0.01 or more and 0.15 or less
M/SiO₂: 0.10 or more and 0.50 or less
OH/SiO₂: 0.10 or more and 0.50 or less
H₂O/SiO₂: 10 or more and 40 or less

In the above composition, the SDA is the cyclohexylenediaminium salt of the present embodiment comprised in the raw material composition, and M is the alkali metal comprised in the raw material composition. The alkali metal comprised in the raw material composition is preferably at least any one of sodium and potassium, and more preferably potassium.

In the present description, SiO₂/Al₂O₃ represents a mole ratio of silica to alumina in the raw material composition, and OH/SiO₂, M/SiO₂, SDA/SiO₂, and H₂O/SiO₂ respectively represent mole ratios of hydroxide ions, the alkali metal, the SDA (the cyclohexylenediaminium salt of the present embodiment), or water relative to silica in the raw material composition. The M represents the alkali metal, and when the alkali metal is potassium or when the alkali metal is sodium and potassium, M/SiO₂ is respectively K/SiO₂ or (Na+K)/SiO₂. The SDA is preferably the cyclohexylenediaminium salt represented by any one of the formula (1-1') or (1-2'), more preferably the cyclohexylenediaminium salt represented by the formula (1-2t), (1-3t), (1-4t), (1-7t), (1-8t), (1-9t), (1-10t), (1-14t), (1-15t), (1-16t), (1-19t), (1-20t), (1-22t), (1-26t), (1-27t), (1-28t), (1-31t), (1-32t), (1-33t), (1-34t), (1-38t), (1-39t), (1-40t), (1-43t), (1-44t), (1-46t), (1-49t), (1-50t), (1-51t), (1-52t), (1-53t), or (1-54t), and further preferably the cyclohexylenediaminium salt represented by the formula (1-2t), (1-4t), (1-8t), (1-26t), (1-28t), (1-32t), (1-34t), (1-53t), or (1-54t).

To enhance the crystallization, the raw material composition may comprise a seed crystal. When the raw material composition comprises the seed crystal, a content of the seed crystal in the raw material composition, which is a mass proportion (hereinafter, also referred to as "seed crystal content") of silicon in the seed crystal in term of silica (SiO₂) relative to a mass of silicon in the raw material composition (excluding the seed crystal) in terms of silica, is more than 0 mass%, or 0.5 mass% or more, and 10 mass% or less, 5 mass% or less, or 3.5 mass% or less. Examples of the range of the seed crystal content in the raw material composition include more than 0 mass% and 10 mass% or less, more than 0 mass% and 5 mass% or less, 0.5 mass% or more and 5 mass% or less, and 0.5 mass% or more and 3.5 mass% or less. Meanwhile, in the present embodiment, it is acceptable that the raw material composition comprises no seed crystal (that is, the seed crystal content is 0 mass%) or that the raw material composition comprises the seed crystal at an extent not to affect enhancement of the crystallization (for example, more than 0 mass% and 0.05 mass% or less as the seed crystal content).

The seed crystal that can be comprised in the raw material composition may be any zeolite having CBU common with the MSE-type zeolite in the crystal structure. For example, the seed crystal is preferably a zeolite having any one zeolite structure selected from the group consisting of MSE, MFI, MOR, *BEA, FER, MEL, MTW, and CON, more preferably a zeolite having any one zeolite structure selected from the group consisting of MSE, MFI, MOR, *BEA, and FER, and further preferably an MSE-type zeolite.

In the present embodiment, the raw material composition is crystallized in the crystallizing step. The crystallization is performed by subjecting the raw material composition to a hydrothermal treatment. As the hydrothermal treatment, the raw material composition is put in a sealed pressure-resistant container, and heated.

Examples of the treatment temperature for the hydrothermal treatment of the raw material composition include 80°C or higher, or 140°C or higher, and 190°C or lower or 180°C or lower. Examples of the range of the treatment temperature for the hydrothermal treatment of the raw material composition include 80°C or higher and 190°C or lower, 80°C or higher and 180°C or lower, 120°C or higher and 180°C or lower, 140°C or higher and 180°C or lower, or 140°C or higher and 170°C or lower.

Examples of the treatment time for the hydrothermal treatment of the raw material composition include 2 hours or longer and 500 hours or shorter, 10 hours or longer and 300 hours or shorter, 20 hours or longer and 200 hours or shorter, or 50 hours or longer and 100 hours or shorter.

Examples of the treatment pressure for the hydrothermal treatment of the raw material composition include a self-generative pressure.

The hydrothermal condition for the hydrothermal treatment of the raw material composition can be the following combination.
Treatment temperature: 80°C or higher or 140°C or higher, and 190°C or lower or 180°C or lower
Treatment time: 2 hours or longer and 500 hours or shorter
Treatment pressure: Self-generative pressure

The hydrothermal condition for the hydrothermal treatment of the raw material composition is preferably the following combination.
Treatment temperature: 120°C or higher and 180°C or lower
Treatment time: 10 hours or longer and 300 hours or shorter
Treatment pressure: Self-generative pressure

The hydrothermal condition for the hydrothermal treatment of the raw material composition is more preferably the following combination.
Treatment temperature: 140°C or higher and 170°C or lower
Treatment time: 50 hours or longer and 100 hours or shorter
Treatment pressure: Self-generative pressure

The state of the raw material composition during the crystallization may be any of under leaving to strand or under stirring, but preferably under stirring.

The MSE-type zeolite producing method of the present embodiment may further comprise post-treating steps such as a washing step, a drying step, an SDA-removing step, or an ammonium-treating step in addition to the aforementioned crystallizing step.

In the washing step, the MSE-type zeolite obtained in the crystallizing step is washed. The washing method in the washing step may be any, and example thereof include contacting the MSE-type zeolite with a sufficient amount of pure water.

In the drying step, moisture is removed from the MSE-type zeolite obtained in the crystallizing step or the MSE-type zeolite washed in the washing step. The drying method in the drying step may be any, and examples thereof include a method in which the MSE-type zeolite is treated in the atmosphere at 100°C or higher and 150°C or lower for 2 hours or longer.

In the SDA-removing step, the SDA remaining in the MSE-type zeolite obtained in the crystallizing step, the MSE-type zeolite washed in the washing step, or the MSE-type zeolite dried in the drying step is removed. For the SDA removal in the SDA-removing step, the condition may be appropriately set with an amount of the MSE-type zeolite subjected to the treatment and the removing method and operation to be provided. Examples of the SDA-removing method include a treatment in the atmosphere at 400°C or higher and 700°C or lower for 1 to 2 hours.

In the ammonium-treating step, the alkali metal is removed from the MSE-type zeolite obtained in the crystallizing step, the MSE-type zeolite washed in the washing step, the MSE-type zeolite dried in the drying step, or the MSE-type zeolite in which the SDA is removed in the SDA-removing step, and the cation type is changed to an ammonium type (hereinafter, referred to as "NH₄⁺ type"). Examples of the ammonium-treating method in the ammonium-treating step include contacting an aqueous solution comprising an ammonium ion and the zeolite. The NH₄⁺-type zeolite obtained in the ammonium-treating step may be further subjected to a thermal treatment to yield an MSE-type zeolite having a cation type of a proton type (hereinafter, referred to as "H⁺ type"). Specific examples of the thermal treatment condition for the NH₄⁺-type zeolite obtained in the ammonium-treating step to have the proton type include heating the NH₄⁺-type zeolite obtained in the ammonium-treating step under a condition in the atmosphere at 500 to 700°C for 1 to 2 hours.

As above, the method for producing a zeolite using the cyclohexylenediaminium salt (1) of the present embodiment has been described with exemplifying the MSE-type zeolite as the large pore zeolite to be produced. However, the cyclohexylenediaminium salt (1) of the present embodiment may be used as the SDA in producing other large pore zeolites, namely zeolites other than the MSE-type zeolite and having a zeolite structure in which the largest pore is an oxygen-containing 12-membered ring. In this case, the aforementioned composition of the raw material composition and crystallization condition are appropriately set.

### <Zeolite>

Next, the zeolite produced by the MSE-type zeolite producing method of the present embodiment will be described. The zeolite obtained by the MSE-type zeolite producing method of the present embodiment may be any, and examples thereof include the MSE-type zeolite.

First, an MSE-type zeolite of the first embodiment produced by the MSE-type zeolite producing method of the present embodiment will be described. The MSE-type zeolite of the first embodiment has XRD peaks shown in the following Table 2 in an XRD pattern measured with CuKα radiation as a radiation source. The MSE-type zeolite having the following XRD peaks has been named STZ-1 (Sagami-Tosoh-Zeolite-1).

**[Table 2]**

| d (Å) | Relative peak intensity (%) |
|---|---|
| 9.18±0.21 | 30~400 |
| 4.45±0.04 | 10~100 |
| 4.32±0.08 | 10~70 |
| 4.12±0.03 | 50~500 |
| 4.05±0.03 | 84~200 |
| 3.97±0.04 | 50~150 |
| 3.84±0.08 | 100 |

| | |
|---|---|
| The relative peak intensity in Table is an intensity relative to a peak intensity at d = 3.84±0.08. | |

The expression "having an XRD peak shown in Table" means that a peak having a relative peak intensity shown in Table can be observed in an XRD pattern within a range of a lattice spacing "d" (Å) shown in Table. Expression "the peak can be observed within a range of a lattice spacing "d" (Å) shown in Table" means that the peak having a peak top within the range of a lattice spacing "d" (Å) can be observed.

The MSE-type zeolite of the first embodiment preferably has XRD peaks in the following Table 3 in the XRD pattern.

**[Table 3]**

| d (Å) | Relative peak intensity (%) |
|---|---|
| 9.18±0.14 | 30~350 |
| 4.45±0.03 | 25~90 |
| 4.32±0.05 | 10~50 |
| 4.11±0.02 | 60~450 |
| 4.05±0.03 | 84~160 |
| 3.97±0.04 | 60~120 |
| 3.86±0.06 | 100 |

| | |
|---|---|
| The relative peak intensity in Table is an intensity relative to a peak intensity at d = 3.86±0.06. | |

The MSE-type zeolite of the first embodiment more preferably has XRD peaks in the following Table 4 in the XRD pattern.

**[Table 4]**

| d (Å) | Relative peak intensity (%) |
|---|---|
| 9.18±0.08 | 30~250 |
| 4.45±0.02 | 30~60 |
| 4.32±0.03 | 10~40 |
| 4.11±0.02 | 110~360 |
| 4.05±0.03 | 84~160 |
| 3.97±0.04 | 72~120 |
| 3.87±0.05 | 100 |

| | |
|---|---|
| The relative peak intensity in Table is an intensity relative to a peak intensity at d = 3.87±0.05. | |

The MSE-type zeolite of the first embodiment furthermore preferably has XRD peaks in the following Table 5 in the XRD pattern.

**[Table 5]**

| d (Å) | Relative peak intensity (%) |
|---|---|
| 12.98±0.18 | 10~350 |
| 10.92±0.14 | 10~200 |
| 10.14±0.21 | 10~150 |
| 9.18±0.08 | 30~250 |
| 4.45±0.02 | 30~60 |
| 4.32±0.03 | 10~40 |
| 4.11±0.02 | 110~360 |
| 4.05±0.03 | 84~160 |
| 3.97±0.04 | 72~120 |
| 3.87±0.04 | 100 |
| 3.41±0.03 | 10~50 |
| 3.24±0.02 | 20~80 |

| | |
|---|---|
| The relative peak intensity in Table is an intensity relative to a peak intensity at d = 3.87±0.04. | |

The MSE-type zeolite of the first embodiment may comprise the cyclohexylenediaminium cation (1') or may not comprise the cyclohexylenediaminium cation (1') as long as it has the XRD peaks shown in Table 2 in the XRD pattern. The cyclohexylenediaminium cation (1') that can be comprised in the MSE-type zeolite of the first embodiment may be any cyclohexylenediaminium cation as long as it is the cyclohexylenediaminium cation represented by the aforementioned formula (1').

In the cyclohexylenediaminium cation (1') that can be comprised in the MSE-type zeolite of the first embodiment, both R¹ and R² in the formula (1') preferably represent an alkyl group having 1 to 4 carbon atoms optionally substituted with one or more selected from the group of a halogen atom, a hydroxy group, an alkoxy group having 1 to 4 carbon atoms, and a dialkylamino group having 2 to 4 carbon atoms. In the cyclohexylenediaminium cation (1') that can be comprised in the MSE-type zeolite of the first embodiment, both R¹ and R² in the formula (1') preferably represent an alkyl group having 1 to 4; the both more preferably each independently represent a methyl group or an ethyl group; and the both further preferably represent a methyl group.

In the cyclohexylenediaminium cation (1') that can be comprised in the MSE-type zeolite of the first embodiment, all the rings A in the formula (1') preferably represent a five-membered or six-membered nitrogen-containing heterocycle optionally having a crosslinking or a condensed ring and optionally substituted with one or more substituents selected from the group consisting of a halogen atom, an alkyl group having 1 to 4 carbon atoms, a hydroxy group, an oxo group, a hydroxymethyl group, a methoxymethyl group, a trifluoromethyl group, a benzyl group, an ethoxycarbonyl group, a methoxycarbonyl group, an acylamino group having 1 to 8 carbon atoms optionally substituted on a nitrogen atom with an alkyl group having 1 to 4 carbon atoms, an alkyloxycarbonylamino group having 1 to 5 carbon atoms, an aminocarbonyl group having 1 to 9 carbon atoms, and a dialkylamino group having 2 to 6 carbon atoms; the all more preferably represent a five-membered or six-membered nitrogen-containing heterocycle optionally having a crosslinking or a condensed ring and optionally substituted with one or more substituents selected from the group consisting of a halogen atom and an alkyl group having 1 to 4 carbon atoms; the all more preferably represent a five-membered or six-membered nitrogen-containing heterocycle optionally substituted with one or more substituents selected from the group consisting of a methyl group, an ethyl group, a methoxy group, an ethoxy group, a hydroxy group, and a halogen atom; the all further preferably represent an unsubstituted five-membered or six-membered nitrogen-containing heterocycle optionally having a crosslinking or a condensed ring; the both further preferably represent a pyrrolidine ring or a piperidine ring optionally substituted with one or more substituents selected from the group consisting of a halogen atom, an alkyl group having 1 to 4 carbon atoms, a hydroxy group, an oxo group, a hydroxymethyl group, a methoxymethyl group, a trifluoromethyl group, a benzyl group, an ethoxycarbonyl group, a methoxycarbonyl group, an acylamino group having 1 to 8 carbon atoms optionally substituted on a nitrogen atom with an alkyl group having 1 to 4 carbon atoms, an alkyloxycarbonylamino group having 1 to 5 carbon atoms, an aminocarbonyl group having 1 to 9 carbon atoms, and a dialkylamino group having 2 to 6 carbon atoms; the all furthermore preferably represent a pyrrolidine ring or a piperidine ring optionally substituted with one or more substituents selected from the group consisting of a halogen atom and an alkyl group having 1 to 4 carbon atoms; and the all particularly preferably represent an unsubstituted pyrrolidine ring or an unsubstituted piperidine ring.

Examples of a preferable embodiment of the cyclohexylenediaminium cation (1') that can be comprised in the MSE-type zeolite of the first embodiment include cyclohexylenediaminium cations (1') in which, in the formula (1'), both R¹ and R² represent a methyl group, an ethyl group, a propyl group, an isopropyl group, or a butyl group, in which all the rings A represent a pyrrolidine ring, a 2-methylpyrrolidine ring, a 3,4-dimethylpyrrolidine ring, a 2,5-dimethylpyrrolidine ring, a 3-hydroxypyrrolidine ring, a piperidine ring, a 4-methylpiperidine ring, a 4-methylpiperazine ring, or a morpholine ring. Examples of a more preferable enbodiment of the cyclohexylenediaminium cation (1') that can be comprised in the MSE-type zeolite of the first embodiment include cyclohexylenediaminium cations (1') in which, in the formula (1), both R¹ and R² represent a methyl group and in which all the rings A represent a pyrrolidine ring or a piperidine ring.

Examples of a particularly preferable embodiment of the cyclohexylenediaminium cation (1') that can be comprised in the MSE-type zeolite of the first embodiment include cyclohexylenediaminium cations (1') represented by at least any one of the following formulae (1'-1') and (1¹-2¹).

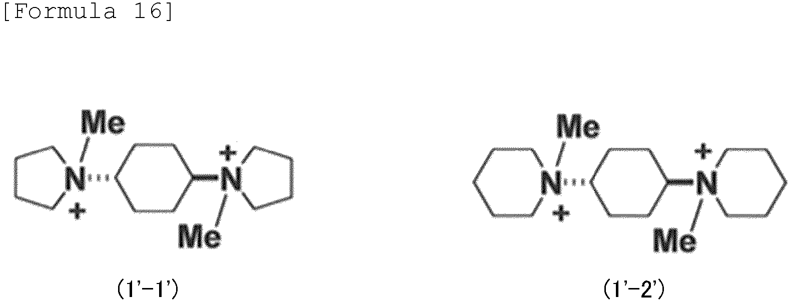

In the MSE-type zeolite of the first embodiment, examples of a mole ratio of the cyclohexylenediaminium cation (1') to silica include 0 or more and 0.2 or less, or 0.001 or more and 0.2 or less, and preferably 0.02 or more and 0.1 or less. In the present specification, the mole ratio of the cyclohexylenediaminium cation (1') to silica being 0 means that no cyclohexylenediaminium cation is comprised.

The MSE-type zeolite of the first embodiment may have an XRD peak having a relative peak intensity of less than 10% in the XRD pattern, and such an XRD peak may not be considered for identifying the zeolite structure.

The MSE-type zeolite of the first embodiment that does not comprise the cyclohexylenediaminium cation (1') preferably has XRD peaks shown in the following Table 6 in the XRD pattern.

**[Table 6]**

| d (Å) | Relative peak intensity (%) |
|---|---|
| 9.18±0.14 | 50~400 |
| 4.45±0.03 | 10~100 |
| 4.32±0.05 | 10~25 |
| 4.11±0.02 | 50~300 |
| 4.05±0.03 | 84~120 |
| 3.97±0.04 | 50~100 |
| 3.86±0.06 | 100 |

| | |
|---|---|
| The relative peak intensity in Table is an intensity relative to a peak intensity at d = 3.86±0.06. | |

The MSE-type zeolite of the first embodiment that does not comprise the cyclohexylenediaminium cation (1') more preferably has XRD peaks shown in the following Table 7 in the XRD pattern.

**[Table 7]**

| d (Å) | Relative peak intensity (%) |
|---|---|
| 9.18±0.08 | 70~350 |
| 4.45±0.02 | 10~50 |
| 4.32±0.03 | 10~20 |
| 4.11±0.02 | 50~200 |
| 4.05±0.03 | 84~110 |
| 3.97±0.04 | 60~90 |
| 3.87±0.04 | 100 |

| | |
|---|---|
| The relative peak intensity in Table is an intensity relative to a peak intensity at d = 3.87±0.04. | |

The MSE-type zeolite of the first embodiment that does not comprise the cyclohexylenediaminium cation (1') furthermore preferably has XRD peaks shown in the following Table 8 in the XRD pattern.

**[Table 8]**

| d (Å) | Relative peak intensity (%) |
|---|---|
| 12.98±0.18 | 20~200 |
| 10.92±0.14 | 30~100 |
| 10.14±0.21 | 30~80 |
| 9.18±0.08 | 70~350 |
| 4.45±0.02 | 10~50 |
| 4.32±0.03 | 10~20 |
| 4.11±0.02 | 50~200 |
| 4.05±0.03 | 84~110 |
| 3.97±0.04 | 60~90 |
| 3.87±0.04 | 100 |
| 3.41±0.03 | 20~40 |
| 3.24±0.02 | 40~70 |

| | |
|---|---|
| The relative peak intensity in Table is an intensity relative to a peak intensity at d = 3.87±0.04. | |

In the present embodiment, the expression "does not comprise the cyclohexylenediaminium cation (1')" refers to the cyclohexylenediaminium cation (1') substantially not comprised. Specifically, in the MSE-type zeolite of the first embodiment that does not comprise the cyclohexylenediaminium cation (1'), examples of the mole ratio of the cyclohexylenediaminium cation (1') to silica include 0 or more and 0.001 or less, and preferably more than 0 and 0.001 or less.

In addition, the MSE-type zeolite of the first embodiment that does not comprise the cyclohexylenediaminium cation (1') more preferably does not comprise another SDA in addition to the cyclohexylenediaminium cation (1'). Specifically, in the MSE-type zeolite of the first embodiment that does not comprise the cyclohexylenediaminium cation (1'), examples of the mole ratio of the SDA (all SDAs including the cyclohexylenediaminium cation (1')) to silica include 0 or more and 0.001 or less, and preferably more than 0 and 0.001 or less. The mole ratio of the SDA to silica being 0 means that all SDAs including the cyclohexylenediaminium cation (1') are not comprised.

In the MSE-type zeolite of the first embodiment, examples of a half width of an XRD peak having a peak top within a range of a lattice spacing d = 3.84±0.08 (Å) (hereinafter, referred to as "standard peak") with CuKα radiation as a radiation source include 0.18° or more, or 0.19° or more, and 0.36° or less, or 0.32° or less even in a case where the zeolite has any XRD peaks shown in the aforementioned Tables 2 to 8. Examples of the range of the half width of the standard peak of the MSE-type zeolite of the first embodiment include 0.18° or more and 0.36° or less, 0.18° or more and 0.32° or less, or 0.19° or more and 0.32° or less.

Examples of the SiO₂/Al₂O₃ ratio of the MSE-type zeolite of the first embodiment include 10 or more, or 12 or more, and 40 or less, or 35 or less, and examples of a range thereof include 10 or more and 40 or less, 12 or more and 30 or less, 15 or more and 30 or less, 16 or more and 28 or less, or 17 or more and 25 or less even in a case where the zeolite has any XRD peaks shown in the aforementioned Tables 2 to 8.

Examples of the average crystal grain diameter of the MSE-type zeolite of the first embodiment include 0.005 µm or more, 0.008 µm or more, 0.01 µm or more, 0.05 µm or more, 0.07 µm or more, 0.09 µm or more, or more than 0.10 µm, 1.0 µm or less, 0.8 µm or less, 0.7 µm or less, 0.6 µm or less, 0.5 µm or less, or 0.3 µm or less, and examples of a range thereof include 0.01 µm or more and 1.0 µm or less, 0.05 µm or more and 0.8 µm or less, 0.07 µm or more and 0.7 µm or less, 0.09 µm or more and 0.5 µm or less, or more than 0.10 µm and 0.3 µm or less even in a case where the zeolite has any XRD peaks shown in the aforementioned Tables 2 to 8.

As for the above value of the average crystal grain diameter of the MSE-type zeolite of the first embodiment, it can be expected that a smaller value facilitates dispersion of ions or molecules in pores to yield an excellent adsorbent. Meanwhile, it can be expected that a larger average crystal grain diameter strengthens the crystal structure to yield an adsorbent with high heat resistance. From the above reasons, the average crystal grain diameter of the MSE-type zeolite of the first embodiment is preferably 0.07 µm or more and 0.7 µm or less, more preferably 0.09 µm or more and 0.5 µm or less, and further preferably more than 0.10 µm and 0.3 µm or less even in a case where the zeolite has any XRD peaks shown in the aforementioned Tables 2 to 8.

In the present specification, "average crystal diameter" refers to an average value of grain diameters of primary particles, and the primary particles are particles with the smallest unit independently observed with a scanning electron microscope (hereinafter, also referred to as "SEM") under the following condition. The SEM observation is performed by using a common scanning electron microscope (for example, device name: JSM-IT200, manufactured by JEOL, Ltd.).
Acceleration voltage: 6 mV
Magnification: 20,000±5,000 times

As for the average crystal diameter, 100±5 primary particles observed with continuous outline in the SEM observation view are extracted, a longest diameter and a shortest diameter of the extracted primary particles are measured, and an average value thereof (= (the longest diameter [µm] + the shortest diameter [µm]) / 2) is determined to be specified as a crystal diameter of each primary particle. Then, an average value of the crystal diameters of the primary particles may be determined to be specified as the average crystal diameter. A number of the SEM observation views may be any number with which the number of the primary particles described above can be observed, and one or a plurality of the SEM observation views may be used. When the plurality of the SEM observation views are used, the observation views with different observation position may be used.

The MSE-type zeolite of the first embodiment may comprise a given alkali metal even in a case where the zeolite has any XRD peaks shown in the aforementioned Tables 2 to 8. Examples of a mole ratio of the alkali metal element to aluminum (hereinafter, also referred to as "M/Al", also referred to as "Na/Al" in a case where the alkali metal element is sodium, and also referred to as "K/Al" in a case where the alkali metal element is potassium) in the MSE-type zeolite of the first embodiment include 0.1 or more, 0.2 or more, or 0.3 or more, and 0.7 or less, or 0.6 or less, and examples of a range thereof include 0.1 or more and 0.7 or less, 0.2 or more and 0.5 or less, 0.2 or more and 0.4 or less, or 0.25 or more and 0.3 or less.

The MSE-type zeolite of the first embodiment preferably comprises cesium (Cs) even in a case where the zeolite has any XRD peaks shown in the aforementioned Tables 2 to 8. This tends to exhibit a high adsorption property when subjected to use of a hydrocarbon adsorbent etc. Examples of a mole ratio of cesium to aluminum (hereinafter, also referred to as "Cs/Al") in the MSE-type zeolite of the first embodiment include 0.2 or more, or 0.3 or more, and 0.7 or less, or 0.6 or less, and examples of a range thereof include 0.2 or more and 0.7 or less, or 0.3 or more and 0.6 or less. Meanwhile, when the MSE-type zeolite of the first embodiment is used as a catalyst, the cation type is preferably the H⁺ type even in a case where the zeolite has any XRD peaks shown in the aforementioned Tables 2 to 8, and preferably comprises substantially no cesium from the viewpoint of leaving H⁺ in the pores. The expression "comprise substantially no cesium" refers to a mole ratio of cesium to aluminum (hereinafter, also referred to as "Cs/Al") in the MSE-type zeolite of the first embodiment being 0.1 or less. Note that Cs/Al being 0 means that cesium is not comprised.

The MSE-type zeolite of the first embodiment can be applied for known use of zeolites even in a case where the zeolite has any XRD peaks shown in the aforementioned Tables 2 to 8. For example, the MSE-type zeolite of the first embodiment can be used for known use of zeolites such as at least one of an adsorbent and a catalyst, and a support therefor. Also, the MSE-type zeolite of the first embodiment can be used as at least any one of the hydrocarbon adsorbent and a support therefor even in a case where the zeolite has any XRD peaks shown in the aforementioned Tables 2 to 8.

The MSE-type zeolite of the first embodiment can adsorb a hydrocarbon by contacting a fluid comprising the hydrocarbon and the MSE-type zeolite of the first embodiment. Examples of the contacting temperature for the fluid comprising the hydrocarbon and the MSE-type zeolite of the first embodiment include 0°C or higher and 500°C or lower. Examples of the contacting time for the fluid comprising the hydrocarbon and the MSE-type zeolite of the first embodiment include 1 hour to 100 hours. The fluid to be contacted with the MSE-type zeolite of the first embodiment may comprise another component such as water and nitrogen in addition to the hydrocarbon.

Next, an MSE-type zeolite of the second embodiment produced by the MSE-type zeolite producing method of the present embodiment will be described. The MSE-type zeolite of the second embodiment comprises the cyclohexylenediaminium cation (1').

The cyclohexylenediaminium cation (1') comprised in the MSE-type zeolite of the second embodiment may be any cyclohexylenediaminium cation as long as it is the cyclohexylenediaminium cation represented by the formula (1'). The preferable cyclohexylenediaminium cation (1') comprised in the MSE-type zeolite of the second embodiment is same as the preferable cyclohexylenediaminium cation (1') that can be comprised in the MSE-type zeolite of the first embodiment.

In the MSE-type zeolite of the second embodiment comprising the cyclohexylenediaminium cation (1'), examples of a mole ratio of the cyclohexylenediaminium cation (1') to silica include more than 0.001 and 0.2 or less, and preferably 0.02 to 0.1.

The MSE-type zeolite of the second embodiment comprising the cyclohexylenediaminium cation (1') preferably has the XRD peaks shown in the Table 2 in the XRD pattern, more preferably has the XRD peaks shown in the Table 3 in the XRD pattern, and furthermore preferably has the XRD peaks shown in the Table 4 in the XRD pattern.

In the MSE-type zeolite of the second embodiment comprising the cyclohexylenediaminium cation (1'), examples of a half width of an XRD peak having a peak top within a range of a lattice spacing d = 3.87±0.04 (Å) (hereinafter, referred to as "standard peak") with CuKα radiation as a radiation source include 0.18° or more, or 0.19° or more, and include 0.36° or less or 0.32° or less. Examples of a range of the half width of the standard peak of the MSE-type zeolite of the second embodiment include 0.18° or more and 0.36° or less, 0.18° or more and 0.32° or less, or 0.19° or more and 0.32° or less.

Examples of the SiO₂/Al₂O₃ ratio of the MSE-type zeolite of the second embodiment comprising the cyclohexylenediaminium cation (1') include 10 or more, or 12 or more, and 40 or less, or 35 or less, and examples of a range thereof include 10 or more and 40 or less, 12 or more and 30 or less, 15 or more and 30 or less, 16 or more and 28 or less, or 17 or more and 25 or less.

Examples of the average crystal grain diameter of the MSE-type zeolite of the second embodiment comprising the cyclohexylenediaminium cation (1') include 0.005 µm or more, 0.008 µm or more, 0.01 µm or more, 0.05 µm or more, 0.07 µm or more, 0.09 µm or more, or more than 0.10 µm, 1.0 µm or less, 0.8 µm or less, 0.7 µm or less, 0.6 µm or less, 0.5 µm or less, or 0.3 µm or less, and examples of a range thereof include 0.01 µm or more and 1.0 µm or less, 0.05 µm or more and 0.8 µm or less, 0.07 µm or more and 0.7 µm or less, 0.09 µm or more and 0.5 µm or less, or more than 0.10 µm and 0.3 µm or less.

As for the above value of the average crystal grain diameter of the MSE-type zeolite of the second embodiment comprising the cyclohexylenediaminium cation (1'), it can be expected that a smaller value facilitates dispersion of ions or molecules in pores to yield an excellent adsorbent. Meanwhile, it can be expected that a larger average crystal grain diameter strengthens the crystal structure to yield an adsorbent with high heat resistance. From the above reasons, the average crystal grain diameter of the MSE-type zeolite of the second embodiment is preferably 0.07 µm or more and 0.7 µm or less, more preferably 0.09 µm or more and 0.5 µm or less, and further preferably more than 0.10 µm and 0.3 µm or less.

The MSE-type zeolite of the second embodiment comprising the cyclohexylenediaminium cation (1') may comprise a given alkali metal. Examples of a mole ratio of the alkali metal element to aluminum (hereinafter, also referred to as "M/Al", also referred to as "Na/Al" in a case where the alkali metal element is sodium, and also referred to as "K/Al" in a case where the alkali metal element is potassium) in the MSE-type zeolite of the second embodiment include 0.1 or more, 0.2 or more, or 0.3 or more, and 0.7 or less or 0.6 or less, and examples of a range thereof include 0.1 or more and 0.7 or less, 0.2 or more and 0.5 or less, 0.2 or more and 0.4 or less, or 0.25 or more and 0.3 or less.

The MSE-type zeolite of the second embodiment comprising the cyclohexylenediaminium cation (1') preferably comprises cesium (Cs). This tends to exhibit a high adsorption property when subjected to use of a hydrocarbon adsorbent etc. Examples of a mole ratio of cesium to aluminum (hereinafter, also referred to as "Cs/Al") in the MSE-type zeolite of the second embodiment include 0.2 or more, or 0.3 or more, and 0.7 or less, or 0.6 or less, and examples of a range thereof include 0.2 or more and 0.7 or less, or 0.3 or more and 0.6 or less. Meanwhile, when the MSE-type zeolite of the second embodiment is used as a catalyst, the cation type is preferably the H⁺ type, and preferably comprises substantially no cesium from the viewpoint of leaving H⁺ in the pores.

The MSE-type zeolite of the second embodiment comprising the cyclohexylenediaminium cation (1') can be applied for known use of zeolites. For example, the MSE-type zeolite of the second embodiment can be used for known use of zeolites such as at least one of an adsorbent and a catalyst, and a support therefor. Also, the MSE-type zeolite of the second embodiment can be used as at least any one of the hydrocarbon adsorbent and a support therefor.

The MSE-type zeolite of the second embodiment can adsorb a hydrocarbon by contacting a fluid comprising the hydrocarbon and the MSE-type zeolite of the second embodiment. Examples of the contacting temperature for the fluid comprising the hydrocarbon and the MSE-type zeolite of the second embodiment include 0°C or higher and 500°C or lower. Examples of the contacting time for which the fluid comprising the hydrocarbon and the MSE-type zeolite of the second embodiment include 1 hour to 100 hours. The fluid to be contacted with the MSE-type zeolite of the second embodiment may comprise another component such as water and nitrogen in addition to the hydrocarbon.

### Examples

Next, Examples of the present embodiments will be described. However, the present embodiments are not limited thereto.

### (1H-NMR)

By using Ascend 400 (400 MHz, manufactured by Bruker) or Ultra Shield Plus 400 (400 MHz, manufactured by Bruker), a ¹H-NMR spectrum of a sample was measured. As a measurement solvent, deuterated chloroform (CDCl₃), deuterated methyl sulfoxide (DMSO-d₆), or deuterium oxide (D₂O) was used, and tetramethylsilane (TMS) was used as an internal standard substance to measure the ¹H-NMR of the sample. As the measurement data, a chemical shift, a multiplicity, a coupling constant (Hz), and an integrated value are described in this order.

### (Ion Chromatography)

By using an ion chromatograph (device name: IC-2010, manufactured by Tosoh Corporation), a chloride ion concentration of the sample was measured under the following condition.
Moving phase: 7.5 mmol/L NaHCO₃ aqueous solution + 1.1 mmol/L Na₂CO₃ aqueous solution
Column: TSKgel Super IC AZ
   (4.6 mm I.D. × 15 cm)
   TSKgel guard column Super IC AZ
   (4.6 mm I.D. × 1 cm)
Column temperature: 40°C
Flow rate: 0.8 ml/min
Injection amount: 30 µL

From the obtained peak, the chloride ion concentration was quantified to determine an ion-exchanging rate.

### (Powder X-Ray Diffraction)

By using a common X-ray diffraction device (device name: UltimaIV Protectus, manufactured by Rigaku Corporation), XRD of the sample was measured. The measurement condition was as follows.
Acceleration current and voltage: 50 mA and 40 kV
Radiation source: CuKα radiation (λ=1.5405 Å)
Measurement mode: Continuous scan
Scanning condition: 50°/min
Measurement range: 2θ = 5° to 45°
Divergence vertical-limiting slit: 10 mm
Divergence / incidence slit: 1°
Photodetecting slit: open
Photodetecting solar slit: 5°
Detector: Semiconductor detector (D/teX Ultra)
Filter: Ni filter

The obtained XRD pattern as a result of the sample measurement was analyzed under the following condition by using a common analysis software (for example, SmartLab StudioII, manufactured by Rigaku Corporation), and compared with the referential pattern to identify a zeolite structure of the sample.
Fitting condition: automatic, refined background
   Variance pseudo-Voigt function (peak shape)
Method for removing background: Fitting method
Method for removing Kα2: Kα1/Kα2 ratio = 0.497
Smoothing method: B-Spline curve
Smoothing condition: secondary differential method, σ-cut value = 3, χ-threshold = 1.5

### (Composition Analysis and Quantification of Silicon and Aluminum)

Into 10 mL of an aqueous solution in which hydrofluoric acid (HF: 48 mass%) and nitric acid (HNO₃: 60 mass%) were mixed and diluted with pure water to be prepared (HF: 0.96 mass%, HNO₃: 1.2 mass%), 2 mg of the sample (for calculating SiO₂/Al₂O₃, Na/Al, and K/Al) or 10 mg of the sample (for calculating Cs/Al) was dissolved to prepare a transparent sample solution. By using a common inductively coupled plasma atomic emission spectrometer (device name: OPTIMA3300DV, manufactured by PERKIN ELMER), this sample solution was measured by the inductively coupled plasma atomic emission spectrometry (ICP-AES). The obtained molar concentrations of silicon (Si), aluminum (Al), sodium (Na), potassium (K), and cesium (Cs), SiO₂/Al₂O₃, Na/Al, K/Al, and Cs/Al of the sample were calculated.

### (Average Crystal Diameter)

In the SEM observation view obtained under the following condition, 100±5 primary particles observed with continuous outline were extracted, a longest diameter and a shortest diameter of the extracted primary particles were measured, and an average value thereof (= (the longest diameter [µm] + the shortest diameter [µm]) / 2) was determined to be specified as a crystal diameter of each primary particle. Then, an average value of the crystal diameters of the primary particles was specified as the average crystal diameter. When 100±5 primary particles failed to be extracted in one SEM observation view, a plurality of the SEM observation views with different observation position were used.

### (Thermal Analysis Measurement)

The measurement was performed under the following condition by using a common thermal analyzer (device name: EXSTRA 6000, TG/DTA 6200, manufactured by Hitachi High-Tech Science Corporation).
Measurement temperature, heating time: from 30°C to 700°C, 10 °C/min
Measurement atmosphere, flow rate: under air flow, 50 mL/min
Measured sample mass: 10 mg

On the obtained weight-reduction curve, a weight reduction corresponding to a region of 200°C or higher and 700°C or lower was attributed to SDA, and a mole ratio SDA/Si of the sample was calculated.

### Synthesis Example 1

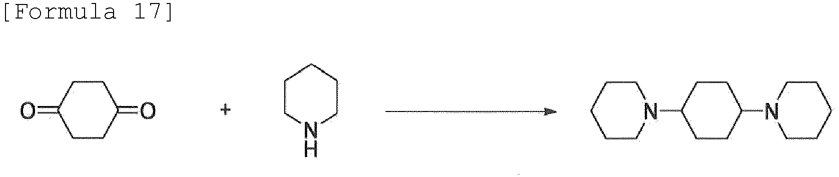

An argon atmosphere was prepared in a side-arm flask, then 1,4-cyclohexadione (8.08 g, 72.0 mmol) and 1,2-dichloroethane (145 mL) were added thereinto. Thereafter, the side-arm flask was cooled with an ice bath, piperidine (16.0 mL, 162 mmol) and acetic acid (0.824 mL, 14.4 mmol) were added in this order, and the mixture was stirred for 1 hour. Thereafter, sodium triacetoxy borohydride (45.8 g, 220 mmol) was added, and the mixture was stirred at room temperature for 16 hours. Thereafter, 1.0-M sodium hydroxide aqueous solution (420 mL) was added, a pH of the aqueous layer was confirmed to be 10 or higher, and the aqueous layer was extracted with ethyl acetate (120 mL × 3). The extract was dried with anhydrous sodium sulfate, and then a volatile component was distilled out with a rotary evaporator to obtain 1,1'-(1,4-cyclohexandiyl)dipiperidine (yield quantity: 18.5 g, yield rate: quant., cis:trans ratio = 61:39) as a reddish brown solid. ¹H-NMR (400 MHz, CDCl3) δ (ppm): 2.52-2.46 (m, 3.1H, trans isomer), 2.46-2.37 (m, 4.9H, cis isomer), 2.24-2.10 (m, 2H), 1.97-1.78 (m, 4H), 1.63-1.50 (m, 8H), 1.47-1.22 (m, 8H).

### Synthesis Example 2

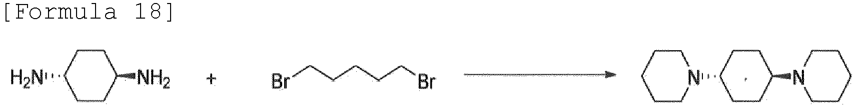

An argon atmosphere was prepared in a side-arm flask, then trans-1,4-diaminocyclohexane (16.2 g, 0.142 mol), potassium carbonate (124 g, 0.897 mol), acetonitrile (500 mL), and 1,5-dibromopentane (40.5 mL, 0.299 mol) were added thereinto, and the mixture was heated with reflux for 65 hours. After the heating with reflux, the mixture was left to be cooled to room temperature, chloroform (700 mL) was added thereinto, and a white insoluble product was separated by filtration. Acetonitrile and chloroform were removed with a rotary evaporator, the product was extracted by liquid separation with chloroform and water, and then chloroform was removed again with a rotary evaporator to obtain a pale orange solid of trans-1,1'-(1,4-cyclohexandiyl)dipiperidine (yield quantity: 34.2 g, yield rate: 96%). ¹H-NMR (400 MHz, CDCl3) δ (ppm): 2.49 (t, J=5.0Hz, 8H), 2.26-2.13 (m, 2H), 1.98-1.87 (m, 4H), 1.61-1.52 (m, 8H), 1.46-1.37 (m, 4H), 1.34-1.20 (m, 4H).

### Synthesis Example 3

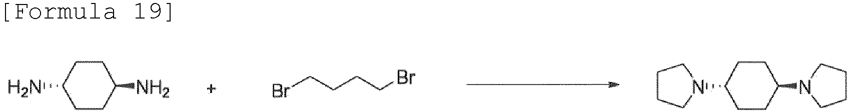

An argon atmosphere was prepared in a side-arm flask, then trans-1,4-diaminocyclohexane (22.8 g, 0.200 mol), potassium carbonate (170 g, 1.23 mol), acetonitrile (800 mL), and 1,4-dibromobutane (50.0 mL, 0.424 mol) were added thereinto, and the mixture was heated with reflux for 18 hours. After the heating with reflux, the mixture was left to be cooled to room temperature, chloroform (1.00 L) was added thereinto, and a white insoluble product was separated by filtration. Acetonitrile and chloroform were removed with a rotary evaporator, the product was extracted by liquid separation with chloroform and water, and then chloroform was removed again with a rotary evaporator to obtain a white solid of trans-1,1'-(1,4-cyclohexandiyl)dipyrrolidine (yield quantity: 17.4 g, yield rate: 39%). ¹H-NMR (400 MHz, CDCl3) δ (ppm): 2.65-2.52 (m, 8H), 2.10-1.90 (m, 6H), 1.84-1.72 (m, 8H), 1.36-1.22 (m, 4H).

### Synthesis Example 4

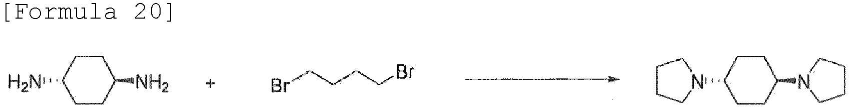

An argon atmosphere was prepared in a side-arm flask, then trans-1,4-diaminocyclohexane (75.0 g, 657 mmol), acetonitrile (300 mL), a sodium hydroxide aqueous solution (approximately 9.0 M, 447 g, 2.96 mol), and 1,4-dibromobutane (157 mL, 1.31 mol) were added, and the mixture was heated with reflux for 16 hours. After the mixture was left to be cooled to room temperature, the precipitated white crystal was separated by filtration and washed with pure water (600 mL) to obtain a white solid of trans-1,1'-(1,4-cyclohexandiyl)dipyrrolidine (yield quantity: 84.0 g, yield rate: 58%).

### Synthesis Example 5

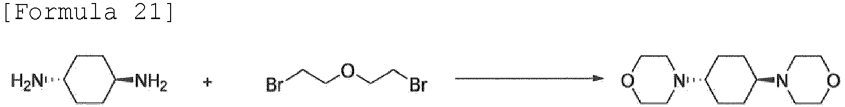

An argon atmosphere was prepared in a 200-mL side-arm flask, then trans-1,4-diaminocyclohexane (2.05 g, 18.0 mmol), potassium carbonate (15.0 g, 109 mmol), acetonitrile (75.0 mL), and bis(2-bromoethyl) ether (5.00 mL, 40.1 mmol) were added, and the mixture was heated with reflux for 17 hours. After the mixture was left to be cooled to room temperature, chloroform (100 mL) was added, and a white insoluble product was separated by filtration. Acetonitrile and chloroform were removed under a reduced pressure, the obtained crude product was extracted with liquid separation with chloroform and water, then the organic layer was concentrated under a reduced pressure, and washed with hexane to obtain a pale brown crystal of trans-1,4-dimorpholinocyclohexane (yield quantity: 4.09 g, yield rate: 90%). ¹H-NMR (400 MHz, CDCl3) δ (ppm): 3.74-3.68 (m, 8H), 2.59-2.51 (m, 8H), 2.20-2.09 (m, 2H), 2.07-1.94 (m, 4H), 1.31-1.16 (m, 4H).

### Synthesis Example 6

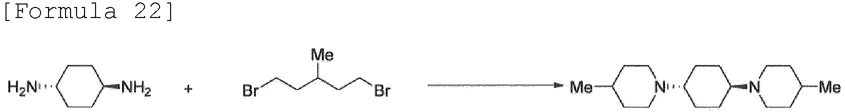

An argon atmosphere was prepared in a 200-mL side-arm flask, then trans-1,4-diaminocyclohexane (1.56 g, 13.7 mmol), potassium carbonate (11.4 g, 82.2 mmol), acetonitrile (44.0 mL), and 1,5-dibromopentane (3.50 mL, 28.9 mmol) were added, and the mixture was heated with reflux for 17 hours. After the mixture was left to be cooled to room temperature, chloroform (100 mL) was added, and a white insoluble product was separated by filtration. Acetonitrile and chloroform were removed under a reduced pressure, the obtained crude product was extracted with liquid separation with chloroform and water, and then the organic layer was concentrated under a reduced pressure to obtain a white solid of 1,4-bis(4-methylpiperidin-1-yl)cyclohexane (diastereomer mixture, ratio of 0.06:1, yield quantity: 3.95 g, yield rate: quant.). ¹H-NMR (400 MHz, CDCl3) δ (ppm): 2.92-2.81 (m, 4H), 2.26-1.87 (m, 10H), 1.67-1.56 (m, 4H), 1.40-1.14 (m, 10H), 0.93 (d, J=6.6Hz, 0.3H), 0.90 (d, J=6.2Hz, 5.7H).

### Synthesis Example 7

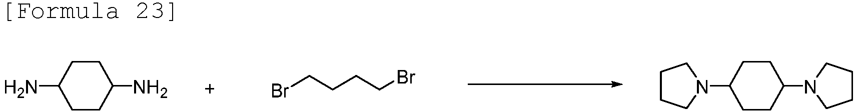

An argon atmosphere was prepared in a side-arm flask, then 1,4-diaminocyclohexane (trans/cis = 66:34) (9.13 g, 80.0 mmol), acetonitrile (40 mL), a sodium hydroxide aqueous solution (approximately 9.0 M, 56.0 g, 400 mmol), and 1,4-dibromobutane (18.9 mL, 160 mmol) were added, and the mixture was heated with reflux for 2 hours. After the heating with reflux, the mixture was left to be cooled to room temperature, pure water (100 mL) was added thereinto, and a precipitated white insoluble product was collected by filtration. The product was washed with pure water (approximately 100 mL), and then dried in vacuo with heating to obtain a white solid of 1,1'-(1,4-cyclohexandiyl)dipyrrolidine (yield quantity: 8.17 g, yield rate: 46%, trans/cis = 72:28). ¹H-NMR (400 MHz, CDCl₃) δ (ppm): 2.63-2.52 (m, 5.8H, trans isomer), 2.52-2.46 (m, 2.2H, cis isomer), 2.10-1.86 (m, 6H), 1.84-1.67 (m, 8H), 1.58-1.42 (m, 1.1H, cis isomer), 1.36-1.22 (m, 2.90H, trans isomer).

### (Synthesis of Cyclohexylenediaminium Salt) Example 1-1

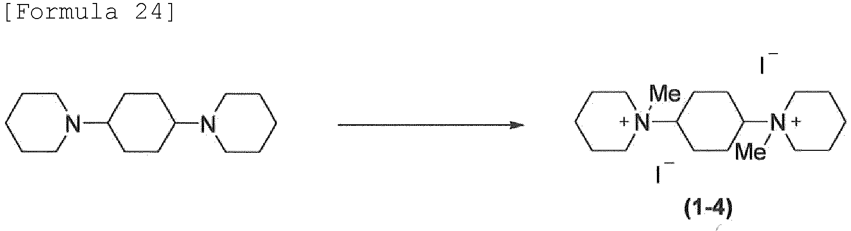

Into a round-bottom flask, 1,1'-(1,4-cyclohexandiyl)dipiperidine (18.4 g, 72.0 mmol), acetonitrile (120 mL), isopropyl alcohol (120 mL), and iodomethane (18.3 mL, 290 mmol) were added, and the mixture was stirred at 50°C for 16 hours. The mixture was left to be cooled to room temperature, then filtered, and washed with hot ethanol (20.0 mL) three times to obtain a white solid of 1,1'-(1,4-cyclohexandiyl)bis(1-methylpiperidinium) diiodide (formula (1-4); yield quantity: 9.00 g, yield rate: 25%, cis:trans ratio = 15:85). ¹H-NMR (400 MHz, D2O) δ (ppm): 3.67 (m, 1.7H, trans isomer), 3.50 (m, 0.3H, cis isomer), 3.43-3.22 (m, 8H), 2.87 (s, 0.9H, cis isomer), 2.85 (s, 5.1H, trans isomer), 2.43-2.29 (m, 3.4H, trans isomer), 2.06-2.01 (m, 0.6H, cis isomer), 1.85-1.46 (m, 16H).

### Example 1-2

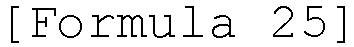

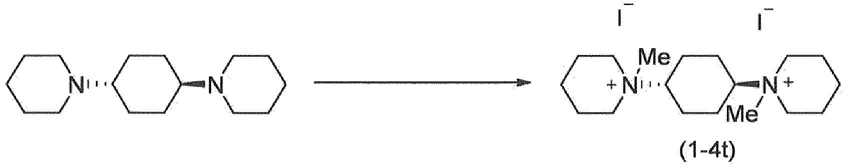

Into a round-bottom flask, trans-1,1'-(1,4-cyclohexandiyl)dipiperidine (1.31 g, 5.21 mmol), chloroform (15.0 mL), and iodomethane (1.30 mL, 20.8 mmol) were added, and the mixture was stirred at 50°C for 16 hours. The mixture was left to be cooled to room temperature, then filtered, and washed with ethanol (15.0 mL) twice to obtain a pale orange solid of trans-1,1'-(1,4-cyclohexandiyl)bis(1-methylpiperidinium) diiodide (formula (1-4t); yield quantity: 1.74 g, yield rate: 62%). ¹H-NMR (400 MHz, D2O) δ (ppm): 3.57-3.46 (m, 2H), 3.43-3.25 (m, 8H), 2.86 (s, 6H), 2.40-2.30 (m, 4H), 1.87-1.50 (m, 16H).

### Example 1-3

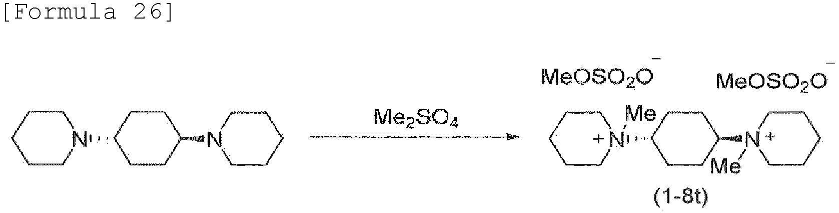

Into a round-bottom flask, trans-1,1'-(1,4-cyclohexandiyl)dipiperidine (30.3 g, 0.121 mmol), acetonitrile (250 mL), and dimethyl sulfate (24.1 mL, 0.254 mol) were added, and the mixture was stirred at room temperature for 24 hours. After filtration, the product was washed with ethyl acetate (100.0 mL × 2) to obtain a pale orange solid of trans-1,1'-(1,4-cyclohexandiyl)bis(1-methylpiperidinium) bis(methyl sulfate) (formula (1-8t); yield quantity: 51.8 g, yield rate: 85%). ¹H-NMR (400 MHz, D2O) δ (ppm): 3.65 (s, 6H), 3.53-3.43 (m, 2H), 3.41-3.22 (m, 8H), 2.85 (s, 6H), 2.40-2.29 (m, 4H), 1.86-1.49 (m, 16H).

### Example 1-4

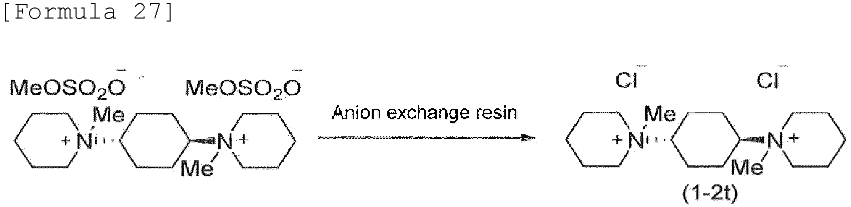

Into an aqueous solution (100 mL) of trans-1,1'-(1,4-cyclohexandiyl)dipiperidinium bis(methyl sulfate) (3.11 g, 6.19 mmol), an anion exchange resin (manufactured by ORGANO CORPORATION, Amberlite(R) IRA-400J CL, 50 mL) was added, and the mixture was left to stand for 12 hours. The anion exchange resin was separated by filtration, then the aqueous solution was condensed with a rotary evaporator, and dried in vacuo at 60°C to obtain a pale yellow solid of trans-1,1'-(1,4-cyclohexandiyl)bis(1-methylpiperidinium) dichloride (formula (1-2t); yield quantity: 2.35 g, yield rate: quant.). ¹H-NMR (400 MHz, D2O) δ (ppm): 3.55-3.42 (m, 2H), 3.42-3.22 (m, 8H), 2.84 (s, 6H), 2.39-2.27 (m, 4H), 1.86-1.47 (m, 16H).

### Example 1-5

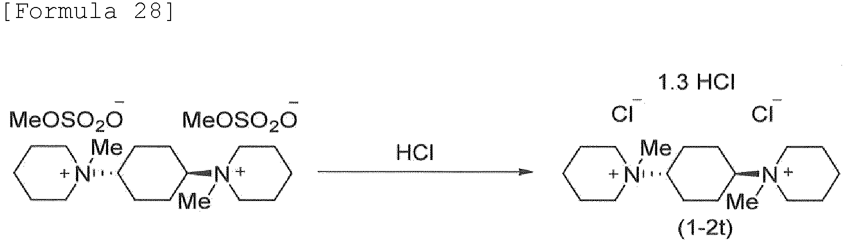

Into a round bottom flask, trans-1,1'-(1,4-cyclohexandiyl)dipiperidinium bis(methyl sulfate) (51.8 g, 103 mmol), ethyl acetate (210 mL), and hydrogen chloride (4.0 M, ethyl acetate solution, 260 mL, 1.04 mol) were added, and the mixture was stirred at room temperature for 17 hours. After filtration, the product was washed with ethyl acetate (100 mL × 2) to obtain a white solid of trans-1,1'-(1,4-cyclohexandiyl)bis(1-methylpiperidinium) dichloride hydrogen chloride adduct (formula (1-2t); yield quantity: 41.7 g, yield rate: quant.). ¹H-NMR (400 MHz, DMSO-d6) δ (ppm): 6.43 (br, 7.25H), 3.84-3.70 (m, 2H), 3.52-3.31 (m, 8H), 2.88 (s, 6H), 2.27-2.12 (m, 4H), 1.91-1.49 (m, 16H).

### Example 1-6

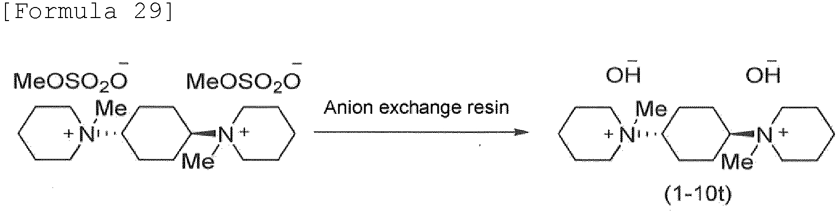

Into an aqueous solution (100 mL) of trans-1,1'-(1,4-cyclohexandiyl)dipiperidinium bis(methyl sulfate) (3.92 g, 7.80 mmol), an anion exchange resin (manufactured by Sigma-Aldrich Co. LLC, Ambersep(R) 900, OH-type, 100 mL) was added, and the mixture was left to stand for 12 hours. The anion exchange resin was separated by filtration, and then the aqueous solution was condensed with a rotary evaporator to obtain trans-1,1'-(1,4-cyclohexandiyl)bis(1-methylpiperidinium) dihydroxide (formula (1-10t); 13.8 weight% aqueous solution, yield quantity: 14.1 g, yield rate: 79%). ¹H-NMR (400 MHz, D2O) δ (ppm): 3.52-3.38 (m, 2H), 3.38-3.17 (m, 8H), 2.80 (s, 6H), 2.42-2.22 (m, 4H), 1.86-1.41 (m, 16H).

### Example 1-7

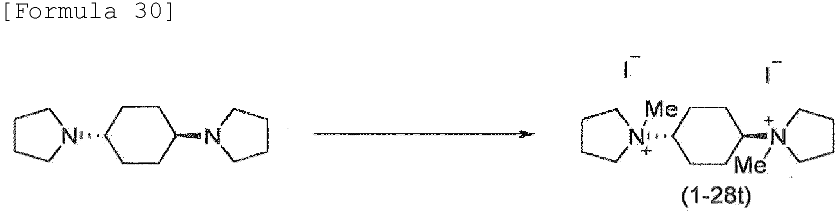

Into a round bottom flask, trans-1,1'-(1,4-cyclohexandiyl)dipyrrolidine (1.57 g, 7.08 mmol), chloroform (20.0 mL), and iodomethane (2.00 mL, 32.1 mmol) were added, and the mixture was stirred at 55°C for 16 hours. The mixture was left to be cooled to room temperature, then filtered, and washed with acetone to obtain a pale orange solid of trans-1,1'-(1,4-cyclohexandiyl)bis(1-methylpyrrolidinium) diiodide (formula (1-28t); yield quantity: 3.35 g, yield rate: 93%). ¹H-NMR (400 MHz, D2O) δ (ppm): 3.62-3.51 (m, 4H), 3.43-3.28 (m, 6H), 2.82 (s, 6H), 2.33-2.22 (m, 4H), 2.21-2.06 (m, 8H) 1.89-1.75 (m, 4H).

### Example 1-8

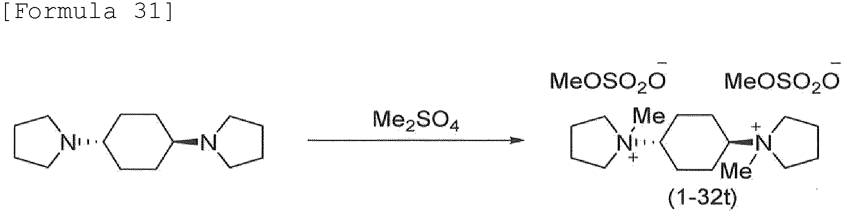

Into a round bottom flask, trans-1,1'-(1,4-cyclohexandiyl)dipyrrolidine (17.0 g, 76.5 mmol), acetonitrile (250 mL), and dimethyl sulfate (18.0 mL, 0.190 mol) were added, and the mixture was stirred at room temperature for 16 hours. After filtration, the product was washed with acetone to obtain a white solid of trans-1,1'-(1,4-cyclohexandiyl)dipyrrolidinium bis(methyl sulfate). The filtrate was condensed and washed with ethanol to obtain a white solid of trans-1,1'-(1,4-cyclohexandiyl)bis(1-methylpyrrolidinium) bis (methyl sulfate) (formula (1-32t)). These white solids were combined to obtain trans-1,1'-(1,4-cyclohexandiyl)dipyrrolidinium bis(methyl sulfate) (yield quantity: 26.6 g, yield rate: 73%). ¹H-NMR (400 MHz, D2O) δ (ppm): 3.67 (s, 6H), 3.60-3.53 (m, 4H), 3.42-3.25 (m, 6H), 2.83 (s, 6H), 2.33-2.23 (m, 4H), 2.22-2.09 (m, 8H), 1.88-1.72 (m, 4H).

### Example 1-9

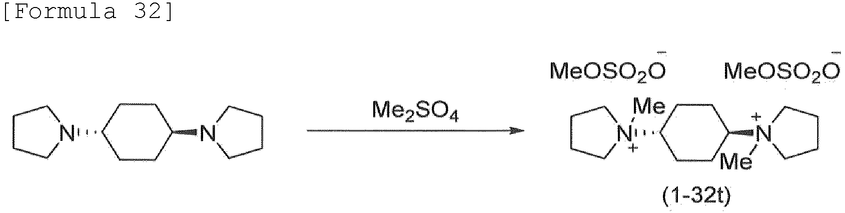

Into a round bottom flask, trans-1,1'-(1,4-cyclohexandiyl)dipyrrolidine (129 g, 581 mmol), acetonitrile (650 mL), and dimethyl sulfate (170 mL, 1.74 mol) were added, and the mixture was stirred at room temperature for 18 hours. The solid was collected by filtration, and then washed with acetone to obtain a white solid of trans-1,1'-(1,4-cyclohexandiyl)dipyrrolidinium bis(methyl sulfate). The filtrate was left to stand, and a generated white solid was washed with acetone to obtain a white solid of trans-1,1'-(1,4-cyclohexandiyl)bis(1-methylpyrrolidinium) bis(methyl sulfate). These white solids were combined to obtain trans-1,1'-(1,4-cyclohexandiyl)dipyrrolidinium bis(methyl sulfate) (formula (1-32t); yield quantity: 238 g, yield rate: 87%).

### Example 1-10

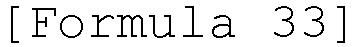

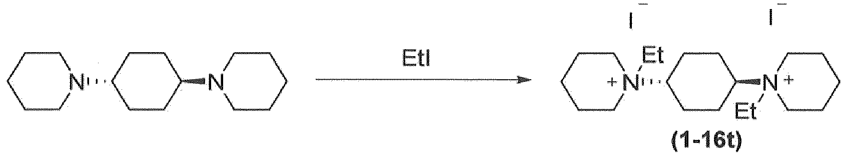

Into a round bottom flask, trans-1,1'-(1,4-cyclohexandiyl)dipiperidine (2.62 g, 10.5 mmol), chloroform (10 mL), and iodoethane (3.00 mL, 37.3 mmol) were added, and the mixture was stirred at 55°C for 88 hours. The generated solid was collected by filtration, and then washed with acetone to obtain a white solid of trans-1,1'-(1,4-cyclohexandiyl)bis(1-ethylpiperidinium) diiodide (formula (1-16t); yield quantity: 3.94 g, yield rate: 67%). ¹H-NMR (400 MHz, D2O) δ (ppm): 3.56-3.45 (m, 2H), 3.41 (q, J=7.5Hz, 4H), 3.36-3.18 (m, 8H), 2.37-2.23 (m, 4H), 1.91-1.44 (m, 16H), 1.20 (t, J=7.5Hz, 6H).

### Example 1-11

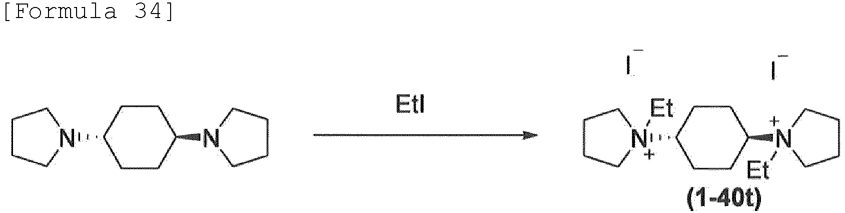

Into a 100-mL round bottom flask, trans-1,1'-(1,4-cyclohexandiyl)dipyrrolidine (733 mg, 3.30 mmol), acetonitrile (10.0 mL), and iodomethane (1.00 mL, 12.4 mmol) were added, and the mixture was stirred at 60°C for 18 hours. The mixture was left to be cooled to room temperature, then a solid was collected by filtration and washed with acetone to obtain a white solid of trans-1,1'-(1,4-cyclohexandiyl)bis(1-ethylpyrrolidinium) diiodide (formula (1-40t); yield quantity: 1.73 g, yield rate: 98%). ¹H-NMR (400 MHz, D2O) δ (ppm): 3.58-3.32 (m, 10H), 3.35 (q, J=7.1Hz, 4H), 2.32-2.23 (m, 4H), 2.08-1.97 (m, 8H), 1.79-1.64 (m, 4H), 1.24 (t, J=7.1Hz, 6H).

### Example 1-12

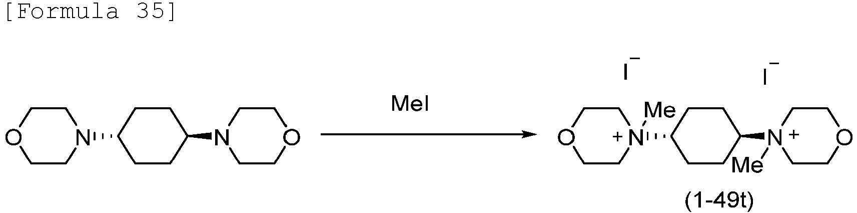

Into a 100-mL round bottom flask, trans-1,4-dimorpholinocyclohexane (1.54 g, 6.07 mmol), acetonitrile (10.0 mL), and iodomethane (1.51 mL, 24.2 mmol) were added, and the mixture was stirred at 45°C for 16 hours. The mixture was left to be cooled to room temperature, then a solid was collected by filtration and washed with acetone to obtain a white solid of trans-4,4'-(1,4-cyclohexandiyl)bis(4-methyl-4-morpholinium) diiodide (formula (1-49t); yield quantity: 3.10 g, yield rate: 95%). ¹H-NMR (400 MHz, D2O) δ (ppm): 4.04-3.94 (m, 8H), 3.62-3.52 (m, 2H), 3.51-3.45 (m, 8H), 3.03 (s, 6H), 2.48-2.36 (m, 4H), 1.84-1.71 (m, 4H).

### Example 1-13

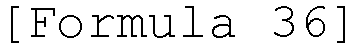

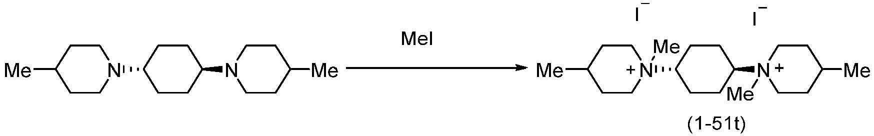

Into a 100-mL round bottom flask, 1,4-bis(4-methylpiperidin-1-yl)cyclohexane (2.00 g, 7.17 mmol), chloroform (15.0 mL), and iodomethane (2.00 mL, 32.1 mmol) were added, and the mixture was stirred at 55°C for 16 hours. The mixture was left to be cooled to room temperature, then a solid was collected by filtration and washed with chloroform to obtain a white solid of trans-1,1'-(1,4-cyclohexandiyl)bis(1,4-dimethylpiperidinium) diiodide (formula (1-51t); diastereomer mixture, yield quantity: 3.82 g, yield rate: 95%). ¹H-NMR (400 MHz, D2O) δ (ppm): 3.70-3.09 (m, 10H), 2.85-2.79 (s, 6H), 2.47-2.15 (m, 4H), 1.86-1.47 (m, 14H), 1.93-1.85 (d, 6H).

### Example 1-14

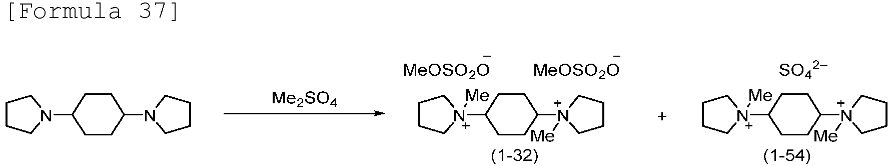

Into a 2-L round bottom flask, 1,1'-(1,4-cyclohexandiyl)dipyrrolidine (trans/cis = 80:20) (142 g, 639 mmol), acetonitrile (500 mL), and dimethyl sulfate (180 mL, 1.90 mmol) were added, and the mixture was stirred at 80°C for 3 hours. After filtration, the product was washed with acetone to obtain a white solid of a mixture of trans-1,1'-(1,4-cyclohexandiyl)dipyrrolidinium bis(methyl sulfate) (formula (1-32t)) and trans-1,1'-(1,4-cyclohexandiyl)bis(1-methylpyrrolidinium) sulfate (formula (1-54t)) (containing 22 mol% of the formula (1-54t)). The white solid precipitated from the filtrate was collected by filtration, and washed with acetone to obtain a white solid of a mixture of 1,1'-(1,4-cyclohexandiyl)bis(1-methylpyrrolidinium) bis(methyl sulfate) (formula (1-32)) and 1,1'-(1,4-cyclohexandiyl)bis(1-methylpyrrolidinium) sulfate (formula (1-54)) (trans/cis = 85:15, containing 28 mol% of the formula (1-54)). These white solids were combined to obtain 1,1'-(1,4-cyclohexandiyl)dipyrrolidinium bis(methyl sulfate) (formula (1-32); trans/cis = 98:2, containing 24 mol% of the formula (1-54), yield quantity: 204 g, yield rate of the formula (1-32): 52%). ¹H-NMR (400 MHz, D₂O) δ (ppm) of the formula (1-32): 3.67 (s, 6H), 3.60-3.53 (m, 4H), 3.42-3.25 (m, 6H), 2.88 (s, 0.1H, cis isomer), 2.83 (s, 5.9H, trans isomer), 2.33-2.23 (m, 4H), 2.22-2.09 (m, 8H), 1.88-1.72 (m, 4H).

### Example 1-15

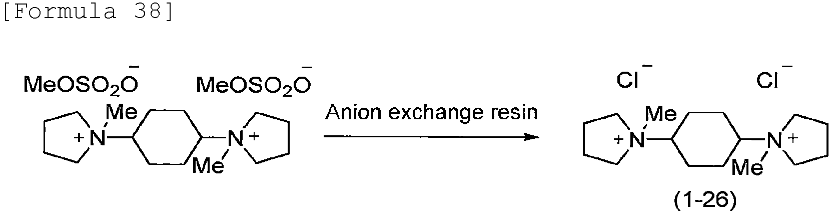

Into an aqueous solution (10 mL) of 1,1'-(1,4-cyclohexandiyl)bis(1-methylpyrrolidinium) bis(methyl sulfate) (trans/cis = 98:2) obtained in Example 1-14 (2.00 g, 4.21 mmol), an anion exchange resin (manufactured by ORGANO CORPORATION, Amberlite(R) IRA-400J CL, 40 mL) was added, and the mixture was left to stand for 5 hours. The anion exchange resin was separated by filtration, then the aqueous solution was condensed with a rotary evaporator, and acetone was added to recover the generated white solid by filtration. The solid was washed with acetone, and dried in vacuo at 60°C to obtain a white solid of 1,1'-(1,4-cyclohexandiyl)bis(1-methylpyrrolidinium) dichloride (formula (1-26); yield quantity: 1.49 g, yield rate: quant., trans/cis = 98:2). The obtained solid was analyzed by ion chromatography to detect a peak of the chloride ion, and the exchanging rate was 97%. ¹H-NMR (400 MHz, D₂O) δ (ppm): 3.59-3.51 (m, 4H), 3.41-3.25 (m, 6H), 2.87 (s, 0.16H, cis isomer), 2.81 (s, 5.84H, trans isomer), 2.32-2.21 (m, 4H), 2.20-2.02 (m, 8H) 1.87-1.72 (m, 4H).

### Example 1-16

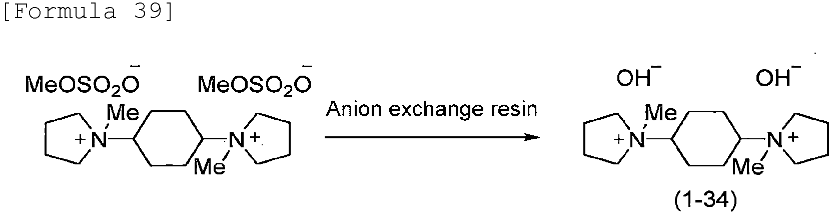

Into an aqueous solution (10 mL) of 1,1'-(1,4-cyclohexandiyl)bis(1-methylpyrrolidinium) bis(methyl sulfate) (trans/cis = 98:2) obtained in Example 1-14 (2.10 g, 4.42 mmol), an anion exchange resin (manufactured by Sigma-Aldrich Co. LLC, Amberlite(R) IRN78, OH type, 50 mL) was added, and the mixture was left to stand for 5 hours. The anion exchange resin was separated by filtration, and then the aqueous solution was condensed with a rotary evaporator to obtain 1,1'-(1,4-cyclohexandiyl)bis(1-methylpyrrolidinium) dihydroxide (formula (1-34); 12.6-weight% aqueous solution, yield quantity: 10.2 g, yield rate: quant., trans/cis = 98:2). Analysis by ¹H-NMR and ion chromatography found that the exchanging rate was >99%. ¹H-NMR (400 MHz, D₂O) δ (ppm): 3.66-3.58 (m, 4H), 3.48-3.30 (m, 6H), 2.95 (s, 0, 15H, cis isomer), 2.88 (s, 5.85H, trans isomer), 2.40-2.28 (m, 4H), 2.28-2.10 (m, 8H) 1.94-1.80 (m, 4H).

### (Synthesis of MSE-Type Zeolite)

### Example 2-1

Mixing 1,1'-(1,4-cyclohexandiyl)dipiperidinium diiodide obtained in Example 1-1, colloidal silica (LUDOX HS-40, SiO₂/Na₂O = 94 (mass ratio)), aluminum hydroxide gel (manufactured by Strem Chemicals Inc.), a 48-mass% potassium hydroxide aqueous solution, and pure water was performed to obtain a raw material composition having the following mole composition. SDA in the following composition was a 1,1'-(1,4-cyclohexandiyl)dipiperidinium cation (hereinafter, also referred to as "CHDPip").

| | |
|---|---|
| SiO₂/Al₂O₃ | = 20.0 |
| SDA/SiO₂ | = 0.06 |
| Na/SiO₂ | = 0.021 |
| K/SiO₂ | = 0.375 |
| OH/SiO₂ | = 0.396 |
| H₂O/SiO₂ | = 30 |

This raw material composition was fed into a 25-mL sealed container, and subjected to a reaction under a static condition of this container at 160°C for 84 hours to obtain a crystallized product. The obtained crystallized product was subjected to solid-liquid separation, washed with pure water, and then dried overnight in the atmosphere at 110°C to obtain an MSE-type zeolite. The MSE-type zeolite of this Example was a single-phase MSE-type zeolite having the following XRD peaks (unsubstituted crystalline aluminosilicate), and had a half width of the standard peak of 0.28°, SiO₂/Al₂O₃ of 23.4, Na/Al of 0.02 or less (under the detection limit), K/Al of 0.31, and the average crystal grain diameter of 0.094 µm.

**[Table 9]**

| d (Å) | Relative peak intensity (%) |
|---|---|
| 12.98 | 82 |
| 10.91 | 50 |
| 10.15 | 43 |
| 9.14 | 39 |
| 4.44 | 40 |
| 4.32 | 26 |
| 4.10 | 115 |
| 4.07 | 129 |
| 3.96 | 82 |
| 3.87 | 100 |
| 3.41 | 29 |
| 3.24 | 48 |

| | |
|---|---|
| **The relative peak intensity in Table is an intensity relative to a peak intensity at d = 3.87.** | |

### Example 2-2

A zeolite of this Example was obtained in the same manner as in Example 2-1 except that a raw material composition having the following mole composition was used, an 80-mL airtight container was used, and the reaction was performed for 240 hours. SDA in the following composition was CHDPip.

| | |
|---|---|
| SiO₂/Al₂O₃ | = 20.0 |
| SDA/SiO₂ | = 0.10 |
| Na/SiO₂ | = 0.021 |
| K/SiO₂ | = 0.375 |
| OH/SiO₂ | = 0.396 |
| H₂O/SiO₂ | = 30 |

The MSE-type zeolite of this Example was a single-phase MSE-type zeolite having the following XRD peaks (unsubstituted crystalline aluminosilicate), and had a half width of the standard peak of 0.27°, SiO₂/Al₂O₃ of 19.4, Na/Al of 0.02 or less (under the detection limit), K/Al of 0.28, and the average crystal grain diameter of 0.094 µm. The MSE-type zeolite of this Example comprised CHDPip, which was SDA, and a mole ratio of CHDPip to silica was 0.057.

**[Table 10]**

| d (Å) | Relative peak intensity (%) |
|---|---|
| 12.96 | 43 |
| 10.93 | 30 |
| 10.15 | 25 |
| 9.16 | 34 |
| 4.45 | 39 |
| 4.32 | 25 |
| 4.10 | 120 |
| 4.08 | 123 |
| 3.96 | 83 |
| 3.87 | 100 |
| 3.41 | 31 |
| 3.24 | 49 |

| | |
|---|---|
| **The relative peak intensity in Table is an intensity relative to a peak intensity at d = 3.87.** | |

### Example 2-3

A zeolite of this Example was obtained in the same manner as in Example 2-1 except that a raw material composition having the following mole composition was used. SDA in the composition of the following raw material composition was CHDPip.

| | |
|---|---|
| SiO₂/Al₂O₃ | = 30.0 |
| SDA/SiO₂ | = 0.06 |
| Na/SiO₂ | = 0.021 |
| K/SiO₂ | = 0.375 |
| OH/SiO₂ | = 0.396 |
| H₂O/SiO₂ | = 30 |

The MSE-type zeolite of this Example was a single-phase MSE-type zeolite having the following XRD peaks (unsubstituted crystalline aluminosilicate), and had a half width of the standard peak of 0.20°, SiO₂/Al₂O₃ of 27.3, Na/Al of 0.02 or less (under the detection limit), K/Al of 0.27, and the average grain diameter of 0.11 µm. The MSE-type zeolite of this Example comprised CHDPip, which was SDA, and a mole ratio of CHDPip to silica was 0.048.

**[Table 11]**

| d (Å) | Relative peak intensity (%) |
|---|---|
| 13.00 | 46 |
| 10.90 | 24 |
| 10.20 | 30 |
| 9.16 | 39 |
| 4.44 | 38 |
| 4.32 | 25 |
| 4.10 | 176 |
| 4.07 | 129 |
| 3.96 | 82 |
| 3.87 | 100 |
| 3.40 | 24 |
| 3.24 | 42 |

| | |
|---|---|
| **The relative peak intensity in Table is an intensity relative to a peak intensity at d = 3.87.** | |

### Example 2-4

A zeolite of this Example was obtained in the same manner as in Example 2-1 except that a raw material composition having the following mole composition was used, and trans-1,1'-(1,4-cyclohexandiyl)dipyrrolidinium diiodide obtained in Example 1-7 was used instead of 1,1'-(1,4-cyclohexandiyl)dipiperidinium diiodide. SDA in the composition of the following raw material composition was a trans-1,1'-(1,4-cyclohexandiyl)dipyrrolidinium cation (trans-CHDPyr).

| | |
|---|---|
| SiO₂/Al₂O₃ | = 20.0 |
| SDA/SiO₂ | = 0.06 |
| Na/SiO₂ | = 0.021 |
| K/SiO₂ | = 0.375 |
| OH/SiO₂ | = 0.396 |
| H₂O/SiO₂ | = 30 |

The MSE-type zeolite of this Example was a single-phase MSE-type zeolite having the following XRD peaks (unsubstituted crystalline aluminosilicate), and had a half width of the standard peak of 0.21°, SiO₂/Al₂O₃ of 19.7, Na/Al of 0.02 or less (under the detection limit), K/Al of 0.30, and the average grain diameter of 0.11 µm.

**[Table 12]**

| d (Å) | Relative peak intensity (%) |
|---|---|
| 12.95 | 92 |
| 10.94 | 63 |
| 10.17 | 52 |
| 9.18 | 53 |
| 4.44 | 42 |
| 4.32 | 27 |
| 4.09 | 228 |
| 4.06 | 84 |
| 3.96 | 86 |
| 3.87 | 100 |
| 3.40 | 30 |
| 3.24 | 40 |

| | |
|---|---|
| **The relative peak intensity in Table is an intensity relative to a peak intensity at d** = **3.87.** | |

### Example 2-5

A zeolite of this Example was obtained in the same manner as in Example 2-1 except that a raw material composition having the following mole composition was used, and trans-1,1'-(1,4-cyclohexandiyl)dipiperidinium bis(methyl sulfate) obtained in Example 1-3 was used instead of 1,1'-(1,4-cyclohexandiyl)dipiperidinium diiodide. SDA in the composition of the following raw material composition was CHDPip.

| | |
|---|---|
| SiO₂/Al₂O₃ | = 25.0 |
| SDA/SiO₂ | = 0.05 |
| Na/SiO₂ | = 0.021 |
| K/SiO₂ | = 0.375 |
| OH/SiO₂ | = 0.396 |
| H₂O/SiO₂ | = 30 |

The MSE-type zeolite of this Example was a single-phase MSE-type zeolite having the following XRD peaks (unsubstituted crystalline aluminosilicate), and had a half width of the standard peak of 0.21°, SiO₂/Al₂O₃ of 29.1, Na/Al of 0.02 or less (under the detection limit), K/Al of 0.27, and the average grain diameter of 0.22 µm. The MSE-type zeolite of this Example comprised CHDPip, which was SDA, and a mole ratio of CHDPip to silica was 0.044.

**[Table 13]**

| d (Å) | Relative peak intensity (%) |
|---|---|
| 12.98 | 62 |
| 10.90 | 27 |
| 10.15 | 31 |
| 9.19 | 44 |
| 4.45 | 42 |
| 4.32 | 22 |
| 4.10 | 196 |
| 4.06 | 96 |
| 3.96 | 85 |
| 3.87 | 100 |
| 3.40 | 26 |
| 3.24 | 40 |

| | |
|---|---|
| **The relative peak intensity in Table is an intensity relative to a peak intensity at d** = **3.87.** | |

### Example 2-6

A raw material composition having the following mole composition was obtained in the same manner as in Example 2-1 except that trans-1,1'-(1,4-cyclohexandiyl)dipiperidinium bis(methyl sulfate) obtained in Example 1-3 was used instead of 1,1'-(1,4-cyclohexandiyl)dipiperidinium diiodide. SDA in the composition of the following raw material composition was CHDPip.

| | |
|---|---|
| SiO₂/Al₂O₃ | = 20.0 |
| SDA/SiO₂ | = 0.05 |
| Na/SiO₂ | = 0.021 |
| K/SiO₂ | = 0.375 |
| OH/SiO₂ | = 0.396 |
| H₂O/SiO₂ | = 30 |

This raw material composition was fed into a sealed container, and the reaction was performed at 160°C for 66 hours with stirring while this container was rotated at 55 rpm. The obtained crystallized product was subjected to solid-liquid separation, washed with deionized water, and then dried overnight in the atmosphere at 110°C. The MSE-type zeolite of this Example was a single-phase MSE-type zeolite having the following XRD peaks (unsubstituted crystalline aluminosilicate), and had a half width of the standard peak of 0.26°, SiO₂/Al₂O₃ of 23.0, Na/Al of 0.02 or less (under the detection limit), K/Al of 0.31, and the average grain diameter of 0.20 µm.

**[Table 14]**

| d (Å) | Relative peak intensity (%) |
|---|---|
| 13.00 | 91 |
| 10.92 | 41 |
| 10.14 | 43 |
| 9.19 | 59 |
| 4.44 | 41 |
| 4.32 | 27 |
| 4.10 | 149 |
| 4.07 | 105 |
| 3.96 | 84 |
| 3.87 | 100 |
| 3.41 | 26 |
| 3.24 | 45 |

| | |
|---|---|
| **The relative peak intensity in Table is an intensity relative to a peak intensity at d** = **3.87.** | |

### Example 2-7

A zeolite of this Example was obtained in the same manner as in Example 2-6 except that a raw material composition having the following mole composition was used, and trans-1,1'-(1,4-cyclohexandiyl)dipyrrolidinium diiodide obtained in Example 1-7 was used instead of 1,1'-(1,4-cyclohexandiyl)dipiperidinium diiodide. SDA2 in the composition of the following raw material composition was trans-CHDPyr.

| | |
|---|---|
| SiO₂/Al₂O₃ | = 20.0 |
| SDA2/SiO₂ | = 0.05 |
| Na/SiO₂ | = 0.021 |
| K/SiO₂ | = 0.375 |
| OH/SiO₂ | = 0.396 |
| H₂O/SiO₂ | = 30 |

The MSE-type zeolite of this Example was a single-phase MSE-type zeolite having the following XRD peaks (unsubstituted crystalline aluminosilicate), and had a half width of the standard peak of 0.23°, SiO₂/Al₂O₃ of 20.3, Na/Al of 0.02 or less (under the detection limit), K/Al of 0.30, and the average grain diameter of 0.18 µm.

**[Table 15]**

| d (Å) | Relative peak intensity (%) |
|---|---|
| 12.98 | 99 |
| 10.91 | 55 |
| 10.15 | 44 |
| 9.19 | 53 |
| 4.44 | 43 |
| 4.32 | 27 |
| 4.10 | 208 |
| 4.06 | 110 |
| 3.96 | 85 |
| 3.87 | 100 |
| 3.41 | 36 |
| 3.24 | 38 |

| | |
|---|---|
| **The relative peak intensity in Table is an intensity relative to a peak intensity at d** = **3.87.** | |

### Example 2-8

A zeolite of this Example was obtained in the same manner as in Example 2-6 except that a raw material composition having the following mole composition was used, and trans-1,1'-(1,4-cyclohexandiyl)dipyrrolidinium bis(methyl sulfate) obtained in Example 1-9 was used instead of 1,1'-(1,4-cyclohexandiyl)dipyrrolidinium diiodide. SDA2 in the composition of the following raw material composition was trans-CHDPyr.

| | |
|---|---|
| SiO₂/Al₂O₃ | = 25.0 |
| SDA2/SiO₂ | = 0.05 |
| Na/SiO₂ | = 0.021 |
| K/SiO₂ | = 0.375 |
| OH/SiO₂ | = 0.396 |
| H₂O/SiO₂ | = 30 |

The MSE-type zeolite of this Example was a single-phase MSE-type zeolite having the following XRD peaks (unsubstituted crystalline aluminosilicate), and had a half width of the standard peak of 0.26°, SiO₂/Al₂O₃ of 23.7, Na/Al of 0.02 or less (under the detection limit), K/Al of 0.22, and the average grain diameter of 0.24 µm.

**[Table 16]**

| d (Å) | Relative peak intensity (%) |
|---|---|
| 12.97 | 47 |
| 10.92 | 36 |
| 10.12 | 37 |
| 9.16 | 37 |
| 4.45 | 37 |
| 4.32 | 23 |
| 4.10 | 160 |
| 4.07 | 110 |
| 3.96 | 97 |
| 3.87 | 100 |
| 3.40 | 30 |
| 3.24 | 41 |

| | |
|---|---|
| **The relative peak intensity in Table is an intensity relative to a peak intensity at d = 3.87.** | |

### Example 2-9

A zeolite of this Example was obtained in the same manner as in Example 2-1 except that a raw material composition having the following mole composition was used, and the reaction was performed for 144 hours. SDA in the following composition was CHDPip.

| | |
|---|---|
| SiO₂/Al₂O₃ | = 20.0 |
| SDA/SiO₂ | = 0.06 |
| Na/SiO₂ | = 0.209 |
| K/SiO₂ | = 0.188 |
| OH/SiO₂ | = 0.397 |
| H₂O/SiO₂ | = 30 |

The MSE-type zeolite of this Example was a single-phase MSE-type zeolite having the following XRD peaks (unsubstituted crystalline aluminosilicate), and had a half width of the standard peak of 0.22°, SiO₂/Al₂O₃ of 19.6, Na/Al of 0.04, K/Al of 0.23, and the average crystal grain diameter of 0.16 µm.

**[Table 17]**

| d (Å) | Relative peak intensity (%) |
|---|---|
| 13.00 | 65 |
| 10.93 | 30 |
| 10.15 | 29 |
| 9.20 | 39 |
| 4.45 | 39 |
| 4.33 | 25 |
| 4.10 | 179 |
| 4.07 | 97 |
| 3.97 | 82 |
| 3.88 | 100 |
| 3.41 | 28 |
| 3.24 | 45 |

| | |
|---|---|
| **The relative peak intensity in Table is an intensity relative to a peak intensity at d = 3.88.** | |

### Example 2-10

A zeolite of this Example was obtained in the same manner as in Example 2-6 except that a raw material composition having the following mole composition was used, and 1,1'-(1,4-cyclohexandiyl)dipyrrolidinium dichloride obtained in Example 1-15 was used instead of 1,1'-(1,4-cyclohexandiyl)dipiperidinium diiodide. SDA3 in the composition of the following raw material composition was CHDPyr (trans/cis = 98:2).

| | |
|---|---|
| SiO₂/Al₂O₃ | = 20.0 |
| SDA3/SiO₂ | = 0.05 |
| Na/SiO₂ | = 0.021 |
| K/SiO₂ | = 0.375 |
| OH/SiO₂ | = 0.396 |
| H₂O/SiO₂ | = 30 |

The MSE-type zeolite of this Example was a single-phase MSE-type zeolite having the following XRD peaks (unsubstituted crystalline aluminosilicate), and had a half width of the standard peak of 0.23°, SiO₂/Al₂O₃ of 18.5, Na/Al of 0.02 or less (under the detection limit), K/Al of 0.21, and the average grain diameter of 0.11 µm.

**[Table 18]**

| d (Å) | Relative peak intensity (%) |
|---|---|
| 12.98 | 67 |
| 10.92 | 37 |
| 10.16 | 31 |
| 9.17 | 31 |
| 4.44 | 41 |
| 4.32 | 25 |
| 4.10 | 198 |
| 4.06 | 101 |
| 3.96 | 86 |
| 3.87 | 100 |
| 3.40 | 33 |
| 3.24 | 43 |

| | |
|---|---|
| **The relative peak intensity in Table is an intensity relative to a peak intensity at d** = **3.87.** | |

### Example 2-11

A zeolite of this Example was obtained in the same manner as in Example 2-6 except that a raw material composition having the following mole composition was used, 1,1'-(1,4-cyclohexandiyl)dipyrrolidinium dihydroxide obtained in Example 1-16 was used instead of 1,1'-(1,4-cyclohexandiyl)dipiperidinium diiodide, and potassium iodide was used as an additional potassium source. SDA3 in the composition of the following raw material composition was CHDPyr (trans/cis = 98:2).

| | |
|---|---|
| SiO₂/Al₂O₃ | = 20.0 |
| SDA3/SiO₂ | = 0.06 |
| Na/SiO₂ | = 0.021 |
| K/SiO₂ | = 0.375 |
| OH/SiO₂ | = 0.396 |
| H₂O/SiO₂ | = 30 |

The MSE-type zeolite of this Example was a single-phase MSE-type zeolite having the following XRD peaks (unsubstituted crystalline aluminosilicate), and had a half width of the standard peak of 0.21°, SiO₂/Al₂O₃ of 18.8, Na/Al of 0.02 or less (under the detection limit), K/Al of 0.20, and the average grain diameter of 0.11 µm.

**[Table 19]**

| d (Å) | Relative peak intensity (%) |
|---|---|
| 12.87 | 46 |
| 10.88 | 36 |
| 10.12 | 32 |
| 9.11 | 34 |
| 4.44 | 41 |
| 4.31 | 24 |
| 4.09 | 208 |
| 4.05 | 96 |
| 3.95 | 82 |
| 3.86 | 100 |
| 3.40 | 33 |
| 3.24 | 43 |

| | |
|---|---|
| **The relative peak intensity in Table is an intensity relative to a peak intensity at d = 3.86.** | |

### Example 2-12

The MSE-type zeolite of Example 2-1 was calcined in dry air at 600°C for 2 hours to obtain an MSE-type zeolite that was a calcined product.

The MSE-type zeolite of this Example was a single-phase MSE-type zeolite having the following XRD peaks (unsubstituted crystalline aluminosilicate), and had a half width of the standard peak of 0.27°, SiO₂/Al₂O₃ of 23.4, Na/Al of 0.02 or less (under the detection limit), K/Al of 0.31, and the average crystal grain diameter of 0.094 µm. The MSE-type zeolite of this Example did not comprise SDA (the mole ratio of CHDPip to silica was 0.001 or less).

**[Table 20]**

| d (Å) | Relative peak intensity (%) |
|---|---|
| 13.01 | 48 |
| 10.92 | 47 |
| 10.11 | 45 |
| 9.15 | 119 |
| 4.44 | 32 |
| 4.31 | 15 |
| 4.09 | 156 |
| 4.05 | 87 |
| 3.94 | 72 |
| 3.85 | 100 |
| 3.41 | 30 |
| 3.24 | 47 |

| | |
|---|---|
| **The relative peak intensity in Table is an intensity relative to a peak intensity at d** = **3.85.** | |

### Example 2-13

The MSE-type zeolite of Example 2-6 was calcined in dry air at 600°C for 2 hours to obtain an MSE-type zeolite that was a calcined product.

The MSE-type zeolite of this Example was a single-phase MSE-type zeolite having the following XRD peaks (unsubstituted crystalline aluminosilicate), and had a half width of the standard peak of 0.29°, SiO₂/Al₂O₃ of 23.0, Na/Al of 0.02 or less (under the detection limit), K/Al of 0.31, and the average grain diameter of 0.20 µm. The MSE-type zeolite of this Example did not comprise SDA (the mole ratio of CHDPip to silica was 0.001 or less).

**[Table 21]**

| d (Å) | Relative peak intensity (%) |
|---|---|
| 12.97 | 45 |
| 10.89 | 45 |
| 10.08 | 42 |
| 9.15 | 102 |
| 4.44 | 26 |
| 4.32 | 13 |
| 4.10 | 136 |
| 4.05 | 86 |
| 3.94 | 65 |
| 3.85 | 100 |
| 3.41 | 26 |
| 3.24 | 50 |

| | |
|---|---|
| **The relative peak intensity in Table is an intensity relative to a peak intensity at d** = **3.85.** | |

### Example 2-14

The MSE-type zeolite of Example 2-7 was calcined in dry air at 600°C for 2 hours to obtain an MSE-type zeolite that was a calcined product.

The MSE-type zeolite of this Example was a single-phase MSE-type zeolite having the following XRD peaks (unsubstituted crystalline aluminosilicate), and had a half width of the standard peak of 0.29°, SiO₂/Al₂O₃ of 20.3, Na/Al of 0.02 or less (under the detection limit), K/Al of 0.30, and the average grain diameter of 0.18 µm. The MSE-type zeolite of this Example did not comprise SDA (the mole ratio of trans-CHDPyr to silica was 0.001 or less).

**[Table 22]**

| d (Å) | Relative peak intensity (%) |
|---|---|
| 13.01 | 48 |
| 10.92 | 47 |
| 10.11 | 45 |
| 9.17 | 103 |
| 4.45 | 27 |
| 4.32 | 14 |
| 4.09 | 160 |
| 4.06 | 92 |
| 3.94 | 65 |
| 3.85 | 100 |
| 3.41 | 29 |
| 3.24 | 56 |

| | |
|---|---|
| **The relative peak intensity in Table is an intensity relative to a peak intensity at d** = **3.85.** | |

### Example 2-15

The MSE-type zeolite of Example 2-8 was calcined in dry air at 600°C for 2 hours to obtain an MSE-type zeolite that was a calcined product.

The MSE-type zeolite of this Example was a single-phase MSE-type zeolite having the following XRD peaks (unsubstituted crystalline aluminosilicate), and had a half width of the standard peak of 0.29°, SiO₂/Al₂O₃ of 23.7, Na/Al of 0.02 or less (under the detection limit), K/Al of 0.22, and the average grain diameter of 0.24 µm. The MSE-type zeolite of this Example did not comprise SDA (the mole ratio of trans-CHDPyr to silica was 0.001 or less).

**[Table 23]**

| d (Å) | Relative peak intensity (%) |
|---|---|
| 12.96 | 61 |
| 10.94 | 51 |
| 10.10 | 47 |
| 9.17 | 115 |
| 4.44 | 34 |
| 4.32 | 17 |
| 4.10 | 158 |
| 4.06 | 92 |
| 3.94 | 83 |
| 3.85 | 100 |
| 3.41 | 29 |
| 3.24 | 51 |

| | |
|---|---|
| **The relative peak intensity in Table is an intensity relative to a peak intensity at d** = **3.85.** | |

### Example 2-16

The MSE-type zeolite of Example 2-10 was calcined in dry air at 600°C for 2 hours to obtain an MSE-type zeolite that was a calcined product.

The MSE-type zeolite of this Example was a single-phase MSE-type zeolite having the following XRD peaks (unsubstituted crystalline aluminosilicate), and had a half width of the standard peak of 0.27° and the average grain diameter of 0.11 µm. The MSE-type zeolite of this Example did not comprise SDA (the mole ratio of CHDPyr (trans/cis = 98:2) to silica was 0.001 or less).

**[Table 24]**

| d (Å) | Relative peak intensity (%) |
|---|---|
| 12.95 | 112 |
| 10.89 | 62 |
| 10.07 | 60 |
| 9.14 | 123 |
| 4.44 | 34 |
| 4.32 | 19 |
| 4.10 | 141 |
| 4.05 | 88 |
| 3.94 | 72 |
| 3.85 | 100 |
| 3.41 | 29 |
| 3.24 | 51 |

| | |
|---|---|
| **The relative peak intensity in Table is an intensity relative to a peak intensity at d** = **3.85.** | |

### Example 2-17

The MSE-type zeolite of Example 2-11 was calcined in dry air at 600°C for 2 hours to obtain an MSE-type zeolite that was a calcined product.

The MSE-type zeolite of this Example was a single-phase MSE-type zeolite having the following XRD peaks (unsubstituted crystalline aluminosilicate), and had a half width of the standard peak of 0.26° and the average grain diameter of 0.11 µm. The MSE-type zeolite of this Example did not comprise SDA (the mole ratio of CHDPyr (trans/cis = 98:2) to silica was 0.001 or less).

**[Table 25]**

| d (Å) | Relative peak intensity (%) |
|---|---|
| 12.96 | 103 |
| 10.91 | 59 |
| 10.11 | 55 |
| 9.19 | 118 |
| 4.45 | 34 |
| 4.32 | 16 |
| 4.09 | 115 |
| 4.06 | 96 |
| 3.94 | 75 |
| 3.85 | 100 |
| 3.41 | 32 |
| 3.24 | 60 |

| | |
|---|---|
| **The relative peak intensity in Table is an intensity relative to a peak intensity at d** = **3.85.** | |

### Comparative Example 1

Nipsil LP (manufactured by TOSOH SILICA CORPORATION), Y-type zeolite (trade name: HSZ-350HUA, manufactured by Tosoh Corporation), a sodium silicate aqueous solution (SiO₂: 28.9 mass%, Na₂O: 9.3 mass%), a potassium silicate aqueous solution (SiO₂: 29.5 wt%, K₂O: 17.6 mass%), a dimethyldipropylammonium (hereinafter, also referred to as "DMDPA") hydroxide aqueous solution, and pure water were mixed to obtain a raw material composition having the following mole composition. SDA in the following composition was dimethyldipropylammonium.
SiO₂/Al₂O₃ = 36
SDA/SiO₂ = 0.17
Na/SiO₂ = 0.15
K/SiO₂ = 0.15
OH/SiO₂ = 0.47
H₂O/SiO₂ = 5.0

A seed crystal was added into the raw material composition at 5 mass% relative to SiO₂ in the raw material composition. The obtained raw material composition was fed into an autoclave, and crystallized under a condition at 160°C under a self-generative pressure for three days (72 hours) at rotation of 55 rpm. The obtained crystallized product was subjected to solid-liquid separation, washed with deionized water, and then dried overnight in the atmosphere at 110°C. The MSE-type zeolite of this Comparative Example was a single-phase MSE-type zeolite having the following XRD peaks (unsubstituted crystalline aluminosilicate), and had a half width of the standard peak of 0.15°, SiO₂/Al₂O₃ of 18.6, Na/Al of 0.08, K/Al of 0.28, and the average crystal grain diameter of 1.3 µm. The MSE-type zeolite of this Comparative Example comprised the DMDPA cation as SDA, and the mole ratio of the DMDPA cation to silica was 0.101.

**[Table 26]**

| d (Å) | Relative peak intensity (%) |
|---|---|
| 13.03 | 47 |
| 10.96 | 26 |
| 10.16 | 40 |
| 9.21 | 63 |
| 4.46 | 24 |
| 4.33 | 18 |
| 4.11 | 139 |
| 4.07 | 82 |
| 3.96 | 70 |
| 3.87 | 100 |
| 3.42 | 28 |
| 3.25 | 37 |

| | |
|---|---|
| **The relative peak intensity in Table is an intensity relative to a peak intensity at d = 3.87.** | |

### Comparative Example 2

The MSE-type zeolite of Comparative Example 1 was calcined in dry air at 600°C for 2 hours to obtain a zeolite that was a calcined product.

The zeolite of this Comparative Example was a single-phase MSE-type zeolite having the following XRD peaks (unsubstituted crystalline aluminosilicate), and had a half width of the standard peak of 0.15°, SiO₂/Al₂O₃ of 18.6, Na/Al of 0.08, K/Al of 0.28, and the average crystal grain diameter of 1.3 µm. The MSE-type zeolite of this Comparative Example did not comprise SDA (the mole ratio of the DMDPA cation to silica was 0.001 or less).

**[Table 27]**

| d (Å) | Relative peak intensity (%) |
|---|---|
| 13.03 | 37 |
| 10.93 | 36 |
| 10.13 | 35 |
| 9.19 | 60 |
| 4.46 | 21 |
| 4.33 | 11 |
| 4.11 | 104 |
| 4.07 | 73 |
| 3.95 | 71 |
| 3.86 | 100 |
| 3.41 | 29 |
| 3.25 | 43 |

| | |
|---|---|
| **The relative peak intensity in Table is an intensity relative to a peak intensity at d** = **3.86.** | |

### Comparative Example 3

Colloidal silica (LUDOX HS-40, SiO₂/Na₂O = 94 (mass ratio)), aluminum hydroxide gel (manufactured by Strem Chemicals Inc.), a 48-mass% potassium hydroxide aqueous solution, and pure water were mixed, and then N,N,N',N'-tetraethylbicyclo[2.2.2]oct-7-ene-2,3:5,6-dipyrrolidinium (hereinafter, also referred to as "TEBOP") diiodide was added and mixed to obtain a raw material composition having the following mole composition. SDA in the following composition was TEBOP.

| | |
|---|---|
| SiO₂/Al₂O₃ | = 23.0 |
| SDA/SiO₂ | = 0.10 |
| Na/SiO₂ | = 0.021 |
| K/SiO₂ | = 0.375 |
| OH/SiO₂ | = 0.396 |
| H₂O/SiO₂ | = 30 |

This raw material composition was fed into a 80-mL sealed container, and the reaction was performed at 160°C for 288 hours while this container was under a static condition to obtain a crystallized product. The obtained crystallized product was subjected to solid-liquid separation, washed with pure water, and then dried overnight in the atmosphere at 110°C to obtain an MSE-type zeolite. The MSE-type zeolite of this Comparative Example was a single-phase MSE-type zeolite having the following XRD peaks (unsubstituted crystalline aluminosilicate), and had a half width of the standard peak of 0.40°, SiO₂/Al₂O₃ of 21.5, Na/Al of 0.02 or less (under the detection limit), K/Al of 0.22, and the average crystal grain diameter of 0.086 µm.

**[Table 28]**

| d (Å) | Relative peak intensity (%) |
|---|---|
| 12.99 | 32 |
| 10.96 | 25 |
| 10.23 | 20 |
| 9.15 | 27 |
| 4.45 | 40 |
| 4.32 | 22 |
| 4.10 | 227 |
| 4.07 | 126 |
| 3.98 | 66 |
| 3.89 | 100 |
| 3.41 | 25 |
| 3.25 | 38 |

| | |
|---|---|
| **The relative peak intensity in Table is an intensity relative to a peak intensity at d = 3.89.** | |

### Example 3 (production of Hydrocarbon Adsorbent)

Each of the MSE-type zeolite of Example 2-2 and the MSE-type zeolite of Comparative Example 1 was calcined in dry air at 600°C for 2 hours to obtain a calcined product. A 20-mass% ammonium chloride aqueous solution was flown through the calcined product, then the calcined product was contacted with a 2-mass% cesium chloride aqueous solution, and washed with a sufficient amount of warm water. Each of the washed zeolites was dried in the atmosphere at 110°C, then formed with pressurizing and crushed to obtain an irregularly shaped product having an aggregation diameter of 20 to 30 mesh as a hydrocarbon adsorbent. The composition of the obtained hydrocarbon adsorbent are shown below.

**[Table 29]**

| | SiO₂ / Al₂O₃ | Cs / Al | K / Al |
|---|---|---|---|
| Example 2-2 | 19.4 | 0.59 | 0.11 |
| Comparative Example 1 | 18.6 | 0.62 | 0.14 |

### (Evaluation of Hydrocarbon Adsorption Property)

In a normal-pressure fixed-bed tubular flow reactor, 0.1 g of each of the hydrocarbon adsorbents of Examples or Comparative Examples was filled, treated under nitrogen flow at 500°C for 30 minutes, and then cooled to 50°C as a pretreatment.

A hydrocarbon-containing gas was flown through each of the pretreated hydrocarbon adsorbents. The composition of the hydrocarbon-containing gas and the flowing condition were as follows.
Hydrocarbon-containing gas: Toluene 3000 vol-ppmC (concentration in terms of methane)
   Water 3 vol%
   Nitrogen Remainder
Gas flow rate: 200 mL/min
Measurement temperature: 50 to 600°C
Heating rate: 10 °C/min

Concentrations of the hydrocarbon (concentration in terms of methane) at an inlet and an outlet of the normal-pressure fixed-bed tubular flow reactor were measured by using an FID. An integrated value of the inlet concentration within a range of the measurement temperature from 50°C to 200°C was specified as "HC amount before flowing [µmolC]", and an integrated value of the outlet concentration was specified as "HC amount after flowing [µmolC]". A hydrocarbon purification rate was calculated from the HC amount before flowing and the HC amount after flowing by using the following formula. Hydrocarbon purification rate [%] = {(HC amount before flowing - HC amount after flowing) / HC amount before flowing} × 100

Change in a desorption amount of the hydrocarbon per mass of the hydrocarbon adsorbent with the rise in the measurement temperature was measured, and a lowest temperature at which the desorption amount reached 0 µmolC/g was specified as a desorption beginning temperature. The adsorption amount of the hydrocarbon per mass of the hydrocarbon adsorbent was determined from a difference between the HC amount before flowing and the HC amount after flowing (= HC amount before flowing - HC amount after flowing) [µmolC] relative to a mass [g] of the hydrocarbon adsorbent.

**[Table 30]**

| | desorption beginning temperature °C | Hydrocarbon purification rate % |
|---|---|---|
| Example 2-2 | 221 | 89 |
| Comparative Example 1 | 210 | 72 |

It has been confirmed from the Table that the hydrocarbon adsorbents using the MSE-type zeolite of Example have a high hydrocarbon purification rate, have a high desorption beginning temperature, and can hold the adsorbed hydrocarbon until high temperature compared with the MSE-type zeolite of Comparative Example. Accordingly, it is understood that the MSE-type zeolites of Examples are suitable as the hydrocarbon adsorbent used at high temperature.

The contents of the description, the claims, and the abstract in Japanese Patent Application No. 2022-54677, filed on March 29, 2022, and Japanese Patent Application No. 2022-190915, filed on November 30, 2022, are entirely incorporated herein by reference as the disclosure of the description of the present disclosure.

## Claims

1. An MSE-type zeolite having the following powder X-ray diffraction peaks.
**[Table 1]**
| d (Å) | Relative peak intensity (%) |
|---|---|
| 9.18±0.21 | 30~400 |
| 4.45±0.04 | 10~100 |
| 4.32±0.08 | 10~70 |
| 4.12±0.03 | 50~500 |
| 4.05±0.03 | 84~200 |
| 3.97±0.04 | 50~150 |
| 3.84±0.08 | 100 |

2. The MSE-type zeolite according to claim 1, wherein a mole ratio of silica to alumina is 10 or more and 40 or less.

3. The MSE-type zeolite according to claim 1 or 2, wherein an average crystal grain diameter is 0.005 µm or more and 1.0 µm or less.

4. An MSE-type zeolite, comprising a cyclohexylenediaminium cation represented by the following formula (1'), 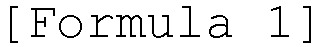 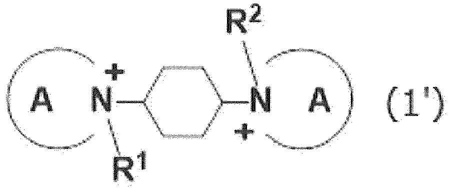
wherein R¹ and R² each independently represents an alkyl group having 1 to 4 carbon atoms, and the alkyl group is optionally substituted with one or more substituents selected from the group consisting of a halogen atom, a hydroxy group, an alkoxy group having 1 to 4 carbon atoms, and a dialkylamino group having 2 to 8 carbon atoms, and
wherein the ring A each independently represents a five to seven-membered nitrogen-containing heterocycle optionally having a crosslinking or a condensed ring, and the nitrogen-containing heterocycle is optionally substituted with one or more substituents selected from the group consisting of: a halogen atom; a hydroxy group; an oxo group; an alkyl group having 1 to 6 carbon atoms optionally substituted with a hydroxy group or an alkoxy group having 1 to 6 carbon atoms; a haloalkyl group having 1 to 6 carbon atoms; an alkoxy group having 1 to 6 carbon atoms; an aryl group having 6 to 12 carbon atoms; an aralkyl group having 7 to 13 carbon atoms; an aryloxy group having 6 to 12 carbon atoms; an alkyloxycarbonyl group having 1 to 6 carbon atoms; an acylamino group having 1 to 8 carbon atoms optionally substituted on a nitrogen atom with an alkyl group having 1 to 4 carbon atoms; an aminocarbonyl group having 1 to 9 carbon atoms optionally substituted on a nitrogen atom with one or more alkyl groups having 1 to 4 carbon atoms; an alkyloxycarbonyloxy group having 1 to 5 carbon atoms; an alkyloxycarbonylamino group having 1 to 5 carbon atoms optionally substituted on a nitrogen atom with an alkyl group having 1 to 4 carbon atoms; an ureide group optionally substituted on a nitrogen atom with one or more alkyl groups having 1 to 4 carbon atoms; a dialkylamino group having 2 to 12 carbon atoms; and a diarylamino group having 12 to 24 carbon atoms.

5. A cyclohexylenediaminium salt represented by the following formula (1), 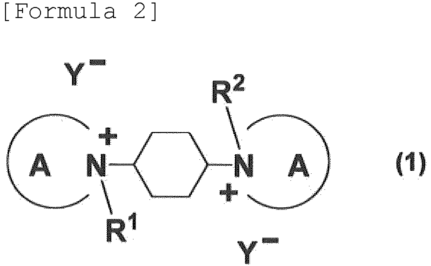
wherein R¹ and R² each independently represents an alkyl group having 1 to 4 carbon atoms, and the alkyl group is optionally substituted with one or more substituents selected from the group consisting of a halogen atom, a hydroxy group, an alkoxy group having 1 to 4 carbon atoms, and a dialkylamino group having 2 to 8 carbon atoms,
wherein the ring A each independently represents a five to seven-membered nitrogen-containing heterocycle optionally having a crosslinking or a condensed ring, and the nitrogen-containing heterocycle is optionally substituted with one or more substituents selected from the group consisting of: a halogen atom; a hydroxy group; an oxo group; an alkyl group having 1 to 6 carbon atoms optionally substituted with a hydroxy group or an alkoxy group having 1 to 6 carbon atoms; a haloalkyl group having 1 to 6 carbon atoms; an alkoxy group having 1 to 6 carbon atoms; an aryl group having 6 to 12 carbon atoms; an aralkyl group having 7 to 13 carbon atoms; an aryloxy group having 6 to 12 carbon atoms; an alkyloxycarbonyl group having 1 to 6 carbon atoms; an acylamino group having 1 to 8 carbon atoms optionally substituted on a nitrogen atom with an alkyl group having 1 to 4 carbon atoms; an aminocarbonyl group having 1 to 9 carbon atoms optionally substituted on a nitrogen atom with one or more alkyl groups having 1 to 4 carbon atoms; an alkyloxycarbonyloxy group having 1 to 5 carbon atoms; an alkyloxycarbonylamino group having 1 to 5 carbon atoms optionally substituted on a nitrogen atom with an alkyl group having 1 to 4 carbon atoms; an ureide group optionally substituted on a nitrogen atom with one or more alkyl groups having 1 to 4 carbon atoms; a dialkylamino group having 2 to 12 carbon atoms; and a diarylamino group having 12 to 24 carbon atoms, and
wherein Y⁻ represents an anion.

6. The cyclohexylenediaminium salt according to claim 5, wherein R¹ and R² in the formula (1) both represent the alkyl group having 1 to 4 carbon atoms.

7. The cyclohexylenediaminium salt according to claim 5 or 6, wherein A in the formula (1) all represent a five to six-membered nitrogen-containing heterocycle optionally having a crosslinking or a condensed ring, and the nitrogen-containing heterocycle is optionally substituted with one or more substituents selected from the group consisting of: a methyl group; an ethyl group; a methoxy group; an ethoxy group; a hydroxy group; and a halogen atom.

8. The cyclohexylenediaminium salt according to claim 5 or 6, wherein R¹ and R² in the formula (1) both represent a methyl group or an ethyl group.

9. The cyclohexylenediaminium salt according to claim 5 or 6, wherein Y⁻ in the formula (1) all represent Cl⁻ (a chloride ion), Br⁻ (a bromide ion), I⁻ (an iodide ion), CH₃OSO₂O⁻ (a methyl sulfate ion), CH₃CH₂OSO₂O⁻ (an ethyl sulfate ion), CH₃OCOO⁻ (a methyl carbonate ion), CH₃CH₂OCOO⁻ (an ethyl carbonate ion), C₆H₅SO₂O⁻ (a benzenesulfonate ion), p-CH₃C₆H₄SO₂O⁻ (a p-toluenesulfonate ion), CH₃SO₂O⁻ (a methanesulfonate ion), CF₃SO₂O⁻ (a trifluoromethanesulfonate ion), or OH⁻ (a hydroxide ion).

10. The cyclohexylenediaminium salt according to claim 5 or 6, wherein A in the formula (1) all represent a piperidine ring.

11. The cyclohexylenediaminium salt according to claim 5 or 6, wherein A in the formula (1) all represent a pyrrolidine ring.

12. A method for producing a zeolite, the method comprising a crystallizing step of crystallizing a composition comprising the cyclohexylenediaminium salt according to any one of claims 5 to 11, a silica source, an alumina source, an alkali source, and water.

13. The method for producing a zeolite according to claim 12, wherein the zeolite is an MSE-type zeolite.
